# EUROPEAN PATENT SPECIFICATION

(11) **EP 3 616 721 B1**
(45) Date of publication and mention of the grant of the patent: **04.12.2024**
(21) Application number: 18787098.5
(22) Date of filing: 13.03.2018
(51) Int. Cl.: A61K 31/194, A61K 45/00, A61P 43/00, A61P 7/00, A23L 33/00, A23L 33/135, A61P 19/06, A61P 13/12, A61P 9/10, A61P 9/00, A61K 9/20, A23L 33/10

(54) **BLOOD PURIFICATION BY ALKALINIZING AGENT**
BLUTREINIGUNG DURCH ALKALISIERUNGSMITTEL
PROCÉDÉ DE PURIFICATION DU SANG À L'AIDE D'UN AGENT D'ALCALINISATION

(30) Priority: 18.04.2017 JP 2017082423; 24.04.2017 JP 2017085741; 25.05.2017 JP 2017103935; 12.09.2017 WO PCT/JP2017/032931
(43) Date of publication of application: 04.03.2020
(73) Proprietor: Tohoku University, Sendai-shi, Miyagi 980-8577 (JP); Nippon Chemiphar Co., Ltd., Tokyo 101-0032 (JP)
(72) Inventor: ABE Michiaki, Sendai-shi Miyagi 980-8577 (JP); KOSHIBA Seizo, Sendai-shi Miyagi 980-8577 (JP); NISHIOKA Koichiro, Tokyo 101-0032 (JP); KAWAGUCHI Kazuhiko, Tokyo 101-0032 (JP); YAMASAKI Satomi, Tokyo 101-0032 (JP); TERANAKA Yasuyuki, Tokyo 101-0032 (JP)
(74) Representative: Kraus & Lederer PartGmbB
(86) International application number: PCT/JP2018/009679
(87) International publication number: WO 2018/193752

(56) References cited:
- WO-A1-2008/141353
- WO-A1-2016/153331
- JP-A- S60 105 619
- JP-A- S60 197 624
- SOROT PHISITKUL ET AL: "Amelioration of metabolic acidosis in patients with low GFR reduced kidney endothelin production and kidney injury, and better preserved GFR", KIDNEY INTERNATIONAL, vol. 77, no. 7, 1 April 2010 (2010-04-01), pages 617 - 623, XP055751794, ISSN: 0085-2538, DOI: 10.1038/ki.2009.519
- G A TANNER ET AL: "Potassium citrate/citric acid intake improves renal function in rats with polycystic kidney disease", JOURNAL OF THE AMERICAN SOCIETY OF NEPHROLOGY : JASN, 1 July 1998 (1998-07-01), United States, pages 1242 - 1248, XP055556949, Retrieved from the Internet <URL:https://jasn.asnjournals.org/content/jnephrol/9/7/1242.full.pdf>
- SOROT PHISITKUL ET AL: "Amelioration of metabolic acidosis in patients with low GFR reduced kidney endothelin production and kidney injury, and better preserved GFR", KIDNEY INTERNATIONAL, vol. 77, no. 7, 1 April 2010 (2010-04-01), GB, pages 617 - 623, XP055751794, ISSN: 0085-2538, DOI: 10.1038/ki.2009.519
- IONE DE BRITO-ASHURST ET AL: "Bicarbonate Supplementation Slows Progression of CKD and Improves Nutritional Status", JOURNAL OF THE AMERICAN SOCIETY OF NEPHROLOGY., vol. 20, no. 9, 1 September 2009 (2009-09-01), US, pages 2075 - 2084, XP055673749, ISSN: 1046-6673, DOI: 10.1681/ASN.2008111205
- NIMRIT GORAYA ET AL: "Dietary acid reduction with fruits and vegetables or bicarbonate attenuates kidney injury in patients with a moderately reduced glomerular filtration rate due to hypertensive nephropathy", KIDNEY INTERNATIONAL, vol. 81, no. 1, 1 January 2012 (2012-01-01), GB, pages 86 - 93, XP055751804, ISSN: 0085-2538, DOI: 10.1038/ki.2011.313
- ASHUTOSH MAHAJAN ET AL: "Daily oral sodium bicarbonate preserves glomerular filtration rate by slowing its decline in early hypertensive nephropathy", KIDNEY INTERNATIONAL, vol. 78, no. 3, 1 August 2010 (2010-08-01), GB, pages 303 - 309, XP055556945, ISSN: 0085-2538, DOI: 10.1038/ki.2010.129
- T. SOUMA ET AL: "Luminal Alkalinization Attenuates Proteinuria-Induced Oxidative Damage in Proximal Tubular Cells", JOURNAL OF THE AMERICAN SOCIETY OF NEPHROLOGY., vol. 22, no. 4, 1 April 2011 (2011-04-01), US, pages 635 - 648, XP055556938, ISSN: 1046-6673, DOI: 10.1681/ASN.2009111130
- F. DURANTON ET AL: "Normal and Pathologic Concentrations of Uremic Toxins", JOURNAL OF THE AMERICAN SOCIETY OF NEPHROLOGY, vol. 23, no. 7, 1 July 2012 (2012-07-01), pages 1258 - 1270, XP055176137, ISSN: 1046-6673, DOI: 10.1681/ASN.2011121175
- GEORGE A TANNER ET AL: "Potassium Citrate/Citric Acid Intake Improves Renal Function in Rats with Polycystic Kidney Disease Correspondence to", J AM SOC NEPHROL, vol. 9, 1 January 1998 (1998-01-01), pages 1242 - 1248, XP055752437
- GADOLA LILIANA ET AL: "Calcium citrate ameliorates the progression of chronic renal injury", KIDNEY INTERNATIONAL, NATURE PUBLISHING GROUP, LONDON, GB, vol. 65, no. 4, 1 April 2004 (2004-04-01), pages 1224 - 1230, XP009108222, ISSN: 0085-2538, DOI: 10.1111/J.1523-1755.2004.00496.X
- OLIVIER DELTOMBE ET AL: "Exploring Protein Binding of Uremic Toxins in Patients with Different Stages of Chronic Kidney Disease and during Hemodialysis", TOXINS, vol. 7, no. 10, 28 September 2015 (2015-09-28), pages 3933 - 3946, XP055751773, DOI: 10.3390/toxins7103933
- ROBERT MAIR ET AL: "Uremic Toxin Clearance and Cardiovascular Toxicities", TOXINS, vol. 10, no. 6, 2 June 2018 (2018-06-02), pages 226, XP055751782, DOI: 10.3390/toxins10060226
- SOUMA, T. ET AL.: "Luminal Alkalinization Attenuates Proteinuria-Induced Oxidative Damage in Proximal Tubular cells", J. AM. SOC. NEPHROL., vol. 22, no. 4, April 2011 (2011-04-01), pages 635 - 648, XP055556938, Retrieved from the Internet <URL:doi:10.1681/ASN.2009111130>
- ABE, MICHIAKI: "Luminal alkalinization attenuates proteinuria- induced oxidative damage in proximal tubular cells", GRANT-IN-AID FOR SCIENTIFIC RESEARCH, FINAL RESEARCH REPORT, 21 April 2016 (2016-04-21), pages 1 - 4, XP009516826, Retrieved from the Internet <URL:https://kaken.nii.ac.jp/grant/KAKENHI-PROJECT-22590199> [retrieved on 20180604]
- ABE, MICHIAKI: "Luminal alkalinization attenuates proteinuria-induced oxidative damage in proximal tubular cells", 24 July 2014 (2014-07-24), pages 1 - 2, XP009516837, Retrieved from the Internet <URL:https://kaken.nii.ac.jp/report/KAKENHI-PROJECT-22590199/225901992012jisseki/> [retrieved on 20180604]
- TORRES, V. E. ET AL.: "Renal Cystic Disease and Ammoniagenesis in Han:SPRD Rats", JOURNAL OF THE AMERICAN SOCIETY OF NEPHROLOGY, vol. 5, no. 5, 1994, pages 1193 - 1200, XP055556939
- NATH, K. A. ET AL.: "Pathophysiology of Chronic Tubulo-lnterstitial Disease in Rats, Interactions of Dietary Acid Load, Ammonia, and Complement Component C3", J. CLIN. INVEST., vol. 76, no. 2, 1985, pages 667 - 675, XP055556943
- MAHAJAN, A. ET AL.: "Daily oral sodium bicarbonate preserves glomerular filtration rate by slowing its decline in early hypertensive nephropathy", KIDNEY INTERNATIONAL, vol. 78, no. 3, 1 August 2010 (2010-08-01), pages 303 - 309, XP055556945, Retrieved from the Internet <URL:https://doi.org/10.1038/ki.2010.129>
- TANNER, G. A.: "Potassium Citrate/Citric Acid Intake Improves Renal Function in Rats with Polycystic Kidney Disease", J. AM. SOC. NEPHROL., vol. 9, no. 7, July 1998 (1998-07-01), pages 1242 - 1248, XP055556949
- ABE, MICHIAKI ET AL.: "Discussion of Uremic Substances that Undergo Pharmacological Blood Purification by Means of Oral Alkalinizers", PROCEEDINGS OF THE 60TH ANNUAL MEETING OF THE JAPANESE SOCIETY OF NEPHROLOGY, vol. 59, no. 3, 25 April 2017 (2017-04-25), pages 234, XP009516827, ISSN: 0385-2385
- ABE, MICHIAKI ET AL.: "Discussion of Uremic Substances that Undergo Pharmacological Blood Purification by Means of Oral Alkalinizers", THE JAPANESE JOURNAL OF NEPHROLOGY, vol. 59, no. 3, 25 April 2017 (2017-04-25), pages 234, XP009516827

## Description

### TECHNICAL FIELD

The present invention relates to blood purification by an alkalinizing agent. More specifically, the invention relates to a pharmaceutical composition comprising an alkalinizing agent for use in treating or preventing kidney tubular damage or in suppressing progression of chronic kidney disease, as further defined in the claims.

The invention is defined in the claims. Any subject-matter which is not encompassed by the claims is not part of the invention and provided for reference or comparison purposes only.

Any reference to a non-patentable method of treatment of the human or animal body by therapy involving the use of a compound or composition is to be understood as referring to said compound or composition for use in said treatment.

### BACKGROUND ART

The number of patients with end-stage kidney disease (ESKD) who require dialysis and transplantation is increasing worldwide. The number has tended to increase also in Japan, and the number of dialysis patients at the end of 2014 was 320,000.

Chronic kidney disease (CKD) is recognized to be likely to get ESKD. CKD is a concept encompassing kidney disease that chronically progresses regardless of the underlying disease, and is a concept encompassing all clinical conditions in which kidney function indicated by glomerular filtration rate (GFR) deteriorates, or findings that suggest damage in kidneys is maintained chronically (3 months or longer). Because CKD has not only a risk of progression to ESKD, but also a strong risk of developing into cardiovascular disease (CVD) etc., it is very important to detect CKD in early stages and perform appropriate treatment. Many CKD treatment methods have been established so far, but they are still insufficient, and further development of nephroprotective agents is required.

In CKD, various uremic substances accumulate in the body as renal clearance decreases. Among them, a concentration of indoxyl sulfate, which is a metabolic end-product of tryptophan, in blood increases with progression of CKD, and a high concentration (100 µM to 1 mM) of indoxyl sulfate accumulates in the blood. Indoxyl sulfate is known to be deeply involved in progression of kidney damage due to kidney fibrosis, and CKD complications such as CVD due to vascular calcification. It is reported that a concentration of indoxyl sulfate in serum correlates with a mortality rate and incidence of cardiovascular events in dialysis patients (Non-Patent Literature 1). In addition, it is considered that, by reducing a concentration of indoxyl sulfate in blood in CKD patients, progression to ESKD can be suppressed, and onset of CVD related to kidney failure can be suppressed. In fact, a spherical carbonaceous adsorbent (KREMEZIN (registered trademark)) which adsorbs indole, which is a precursor of indoxyl sulfate, in the intestinal tract to decrease a concentration of indoxyl sulfate in blood, delays introduction of dialysis in CKD patients, thereby ameliorating arteriosclerosis (Non-Patent Literature 2).

Meanwhile, because a concentration of bicarbonate ions (HCO₃⁻) in blood decreases and metabolic acidosis develops in patients with advanced CKD, an alkalinizing agent such as sodium bicarbonate or a citric acid preparation is administered. In addition, it is reported that progression of CKD is suppressed by administration of sodium bicarbonate that is an alkalinizing agent (Non-Patent Literature 3). Furthermore, it is reported that oral administration of sodium bicarbonate suppresses kidney tubular cell damage due to acidic urine in an animal model with nephrosis caused by protein overload (Non-Patent Literature 4).

However, there is no report regarding suppression of progression of kidney damage by administering an alkalinizing agent to early-stage CKD patients, and there is also no report regarding a decrease in concentration of uremic substance in blood.

Sorot Phisitkul et al. (Kidney International, vol. 77, no. 7, pages 617-623) report that amelioration of metabolic acidosis in patients with low GFR reduced kidney endothelin production and kidney injury, and better preserved GFR. Nimrit Goraya et al. (Kidney International, vol. 81, no. 1, pages 86-93) report that dietary acid reduction with fruits and vegetables or bicarbonate attenuates kidney injury in patients with a moderately reduced glomerular filtration rate due to hypertensive nephropathy. Ashutosh Mahajan et al. (Kidney International, vol. 78, no. 3, pages 303-309) report that daily oral sodium bicarbonate preserves glomerular filtration rate by slowing its decline in early hypertensive nephropathy. Duranton et al. (J. Am. Soc. Nephrol., vol. 23, no. 7, pages 1258-1270) report on normal and pathologic concentrations of uremic toxins. Tanner et al. (J. Am. Soc. Nephrol., vol. 9, 1998, pages 1242-1248) report that potassium citrate/citric acid intake improves renal function in rats with polycystic kidney disease. Gadola et al. (Kidney International, vol. 65, no. 4, 1 April 2004, pages 1224-1230) report that calcium citrate ameliorates the progression of chronic renal injury. Deltombe et al. (Toxins, vol. 7, no. 10, 28 September 2015, pages 3933-3946) describe a study exploring protein binding of uremic toxins in patients with different stages of chronic kidney disease and during hemodialysis. Mair et al. (Toxins, vol. 10, no. 6, 2 June 2018, page 226) report on uremic toxin clearance and cardiovascular toxicities. D11 WO 2016/153331 A1 describes a mixture of carboxylic acids for treating patients with kidney failure. Souma et al. (J. Am. Soc. Nephrol., vol. 22, no. 4, pages 635-648) report that luminal alkalinization attenuates proteinuria-induced oxidative damage in proximal tubular cells. Abe (Grant-in-Aid for Scientific Research, URL: https://kaken.nii.ac.jp/report/KAKENHI-PROJECT-22590199/225901992012jisseki/; and Grant-in-Aid for Scientific Research, Final Research Report, 21 April 2016, pages 1-4) reports that luminal alkalinization attenuates proteinuria-induced oxidative damage in proximal tubular cells. Torres et al. (J. Am. Soc. Nephrol., vol. 5, no. 5, 1994, pages 1193-1200) report on renal cystic disease and ammoniagenesis in Han:SPRD rats. Nath et al. (J. Clin. Invest., vol. 76, no. 2, 1985, pages 667-675) report on the pathophysiology of chronic tubulo-interstitial disease in rats, interactions of dietary acid load, ammonia, and complement component C3. Mahajan et al. (Kidney International, vol. 78, no. 3, pages 303-309) report that daily oral sodium bicarbonate preserves glomerular filtration rate by slowing its decline in early hypertensive nephropathy. Tanner (J. Am. Soc. Nephrol., vol. 9, no. 7, pages 1242-1248) report that potassium citrate/citric acid intake improves renal function in rats with polycystic kidney disease. Abe et al. (Proceedings of the 60th Annual Meeting of the Japanese Society of Nephrology, vol. 59, no. 3, page 234; and The Japanese Journal of Nephrology, vol. 59, no. 3, 25 April 2017, page 234) provide a discussion of uremic substances that undergo pharmacological blood purification by means of oral alkalinizers. JP S60 197624 A and JP S60 105619 A describe urine-alkalizing food compositions.

### CITATION LIST

### NON-PATENT LITERATURE

[Non-Patent Literature 1] Barreto, F. C., et al.: Serum indoxyl sulfate is associated with vascular disease and mortality in chronic kidney disease patients. Clin. J. Am. Soc. Nephrol., 4: 1551-1558, 2009.
[Non-Patent Literature 2] Nakamura T., et al.: Oral ADSORBENT AST-120 decreases carotid intima-media thickness and arterial stiffness in patients with chronic renal failure. Kidney Blood Press Res, 27: 121-6, 2004.
[Non-Patent Literature 3] Brito-Ashurst, I. D., et al.: Bicarbonate supplementation slows progression of CKD and improves nutritional status. J. Am. Soc. Nephrol., 20: 2075-2084, 2009.
[Non-Patent Literature 4] Souma T., et al.: Luminal alkalinization attenuates proteinuria-induced oxidative damage in proximal tubular cells. J. Am. Soc. Nephrol., 22: 635-648, 2011.

### SUMMARY OF THE INVENTION

### PROBLEMS TO BE SOLVED BY THE INVENTION

An object of the present invention is to provide a medicine useful for blood purification in a patient with kidney disease. More specifically, the object of the present invention is to provide a medicine useful for suppressing progression of chronic kidney disease (advancing in severity of chronic kidney disease) and a medicine useful for treating or preventing kidney tubular damage. Also described herein, but not claimed as such are a medicine useful for treating and preventing uremic symptoms, and delaying introduction of dialysis, a medicine useful for suppressing progression from acute kidney failure to chronic kidney disease, a food for promoting excretion of uremic substance outside the body, a food for maintenance of kidney function (for example, for suppression of kidney tubular damage, for protection of kidney tubular cells, or for maintenance of kidney tubular function), a method for determining suppression of progression of chronic kidney disease, or a method for determining a decrease in concentration of uremic substance in blood, and/or a promotion of excretion of uremic substance into urine.

### MEANS TO SOLVE THE PROBLEMS

The inventors of the present invention have performed extensive studies to achieve the above-mentioned objects. As a result, they have found that an agent that alkalinizes a body fluid is useful for promoting excretion of uremic substance from the body of a patient with kidney disease (for example, promoting excretion of uremic substance into urine), and therefore have completed the present invention.

Thus, the invention provides a pharmaceutical composition comprising an alkalinizing agent for use in treating or preventing kidney tubular damage or in suppressing progression of chronic kidney disease, wherein the alkalinizing agent is a mixture of sodium citrate or a hydrate thereof, and potassium citrate or a hydrate thereof, 0.1 to 5 g/day of each of sodium citrate or the hydrate thereof, and potassium citrate or the hydrate thereof are orally administered at a total of 0.2 to 10 g/day, 1 to 5 times a day, and the alkalinizing agent is administered to a patient with stage G2 to stage G3b chronic kidney disease.

Also described herein, but not claimed as such are a pharmaceutical composition for promoting excretion of uremic substance outside the body, comprising an alkalinizing agent, a pharmaceutical composition for decreasing a concentration of uremic substance in blood, comprising an alkalinizing agent, a pharmaceutical composition for promoting excretion into urine in chronic kidney disease, comprising an alkalinizing agent, a pharmaceutical composition for ameliorating uremic symptoms in chronic kidney disease, comprising an alkalinizing agent, a pharmaceutical composition for delaying introduction of dialysis in chronic kidney disease, comprising an alkalinizing agent, a pharmaceutical composition for treating or preventing cardiovascular disease associated with chronic kidney disease, comprising an alkalinizing agent, a pharmaceutical composition for suppressing progression from acute kidney failure to chronic kidney disease, comprising an alkalinizing agent, a food composition for promoting excretion of uremic substance outside the body, comprising an alkalinizing agent, a method for determining suppression of progression of chronic kidney disease, and a method for determining a decrease in concentration of uremic toxins in blood of a human, and/or a promotion of excretion of uremic toxins into urine.

That is, the present invention has the following aspects. (1) A pharmaceutical composition comprising an alkalinizing agent for use in treating or preventing kidney tubular damage, wherein the alkalinizing agent is a mixture of sodium citrate or a hydrate thereof, and potassium citrate or a hydrate thereof, 0.1 to 5 g/day of each of sodium citrate or the hydrate thereof, and potassium citrate or the hydrate thereof are orally administered at a total of 0.2 to 10 g/day, 1 to 5 times a day, and the alkalinizing agent is administered to a patient with stage G2 to stage G3b chronic kidney disease.

(2) A pharmaceutical composition comprising an alkalinizing agent for use in suppressing progression of chronic kidney disease, wherein the alkalinizing agent is a mixture of sodium citrate or a hydrate thereof, and potassium citrate or a hydrate thereof, 0.1 to 5 g/day of each of sodium citrate or the hydrate thereof, and potassium citrate or the hydrate thereof are orally administered at a total of 0.2 to 10 g/day, 1 to 5 times a day, and the alkalinizing agent is administered to a patient with stage G2 to stage G3b chronic kidney disease.

(3) The pharmaceutical composition for use according to any one of (1) or (2), wherein 0.5 to 1.5 g/day of each of sodium citrate dihydrate (C₆H₅Na₃O₇·2H₂O), and potassium citrate hydrate (C₆H₅K₃O₇·H₂O) are orally administered at a total of 1 to 3 g/day, 1 to 5 times a day.

(4) The pharmaceutical composition for use according to any one of (1) to (3), wherein a decrease in concentration of the uremic substance in blood compared to before the start of administration of the alkalinizing agent is detected 12 weeks after the administration of the alkalinizing agent.

(5) The pharmaceutical composition for use according to any one of (1) to (4), wherein an increase in concentration of the uremic substance in urine compared to before the start of administration of the alkalinizing agent is detected 12 weeks after the administration of the alkalinizing agent.

(6) The pharmaceutical composition for use according to (4) or (5), wherein the uremic substance is at least one selected from the group consisting of indoxyl sulfate, p-cresyl sulfate, phenylacetyl-L-glutamine, hippuric acid and argininosuccinic acid.

(7) The pharmaceutical composition for use according to (4) or (5), wherein the uremic substance is indoxyl sulfate.

(8) The pharmaceutical composition for use according to any one of (1) to (7), wherein the pharmaceutical composition is a tablet.

(9) The pharmaceutical composition according to any one of (1) or (2), which is administered to a patient with stage G2 and stage G3a chronic kidney disease.

(10) The pharmaceutical composition according to any one of (1) or (2), which is administered to a patient with stage G2 chronic kidney disease.

(11) The pharmaceutical composition according to any one of (1) or (2), which is administered to a patient in which a urinary β2-microglobulin concentration is 290 µg/L or less.

(12) The pharmaceutical composition according to any one of (1) or (2), which is administered to a patient in which a urinary β2-microglobulin concentration is 50 to 150 µg/L.

(13) The pharmaceutical composition according to any one of (1) or (2), which is administered to a patient in which a blood cystatin C concentration is 0.5 to 2.2 mg/L.

(14) The pharmaceutical composition according to any one of (1) or (2), which is administered to a patient in which a blood cystatin C concentration is 1.0 to 1.3 mg/L.

(15) The pharmaceutical composition according to any one of (1) or (2), wherein an increase in amount of the uremic substance excreted outside the body compared to before the start of administration of the alkalinizing agent is detected 12 weeks after the administration of the alkalinizing agent.

(16) The pharmaceutical composition according to any one of (1) or (2), wherein administration of the alkalinizing agent suppresses an increase of a urinary β2-microglobulin concentration.

(17) The pharmaceutical composition according to any one of (1) or (2), wherein, by administration of the alkalinizing agent, after 12 weeks from the administration, an increase of a urinary β2-microglobulin concentration is suppressed.

(18) The pharmaceutical composition according to any one of (1) or (2), wherein, by administration of the alkalinizing agent, a urinary β2-microglobulin concentration is not substantially decreased compared to before the start of the administration.

(19) The pharmaceutical composition according to any one of (1) or (2), wherein, by administration of the alkalinizing agent, after 12 weeks from the administration, a urinary β2-microglobulin concentration is not substantially decreased compared to before the start of the administration.

(20) The pharmaceutical composition according to any one of (1) or (2), wherein administration of the alkalinizing agent does not substantially increase blood cystatin C compared to before the start of the administration.

(21) The pharmaceutical composition according to any one of (1) or (2), wherein, by administration of the alkalinizing agent, after 12 weeks from the administration, blood cystatin C is not substantially increased compared to before the start of the administration.

(22) The pharmaceutical composition according to any one of (1) or (2), wherein, by administration of the alkalinizing agent, amelioration of kidney proximal tubular damage and/or amelioration of glomerular damage is not recognized compared to before the start of the administration; and a decrease in a concentration of the uremic substance in blood, a promotion of excretion of the uremic substance into urine, and/or a promotion of excretion of the uremic substance outside the body is recognized compared to before the start of the administration.

(23) The pharmaceutical composition according to any one of (1) or (2), wherein, by administration of the alkalinizing agent, after 12 weeks from the administration of the alkalinizing agent, amelioration of kidney proximal tubular damage and/or amelioration of glomerular damage is not recognized compared to before the start of the administration; and a decrease in a concentration of the uremic substance in blood, a promotion of excretion of the uremic substance into urine, and/or a promotion of excretion of the uremic substance outside the body is recognized, compared to before the start of the administration.

(24) The pharmaceutical composition according to any one of (1) or (2), wherein the alkalinizing agent is administered such that a pH of early morning urine becomes pH 5.2 to pH 6.8.

(25) The pharmaceutical composition according to any one of (1) or (2), wherein the alkalinizing agent is administered such that a pH of early morning urine becomes pH 6.0 or more and less than pH 6.2.

(26) The pharmaceutical composition according to any one of (1) or (2), wherein the alkalinizing agent is administered for 12 weeks or longer.

(27) The pharmaceutical composition according to any one of (1) or (2), wherein the alkalinizing agent is administered for 12 weeks.

(28) The pharmaceutical composition according to any one of (1) or (2), wherein administration of the alkalinizing agent exerts an effect of decreasing a concentration of uremic substance in blood, an effect of promoting excretion of uremic substance into urine, and/or an effect of promoting excretion of uremic substance outside the body, and the effects are recognized with respect to placebo administration.

(29) The pharmaceutical composition according to any one of (1) or (2), wherein administration of the alkalinizing agent exerts an effect of decreasing a concentration of uremic substance in blood, an effect of promoting excretion of uremic substance into urine, and/or an effect of promoting excretion of uremic substance outside the body, and the effects are recognized with respect to before administration of the alkalinizing agent.

(30) The pharmaceutical composition according to any one of (1) or (2), wherein the urine is early morning urine.

### EFFECTS OF THE INVENTION

By the pharmaceutical composition provided by the present invention, uremic substance is excreted outside the body in mammals. By the method described herein, it is possible to preliminarily determine as to whether or not uremic substance is excreted outside the body and/or whether or not a progression of chronic kidney disease can be suppressed.

### BRIEF DESCRIPTION OF DRAWINGS

FIG. 1 is a graph showing a correlation between a concentration of indoxyl sulfate in urine and a concentration of indoxyl sulfate in plasma of patients of a control group after 6, 12, and 24 weeks from the start of the test.
FIG. 2 is a graph showing a correlation between a concentration of indoxyl sulfate in urine and a concentration of indoxyl sulfate in plasma of patients of a group to which a combination preparation of hydrates of potassium citrate and sodium citrate has been administered, after 6, 12, and 24 weeks from the start of the test.
FIG. 3 is a graph showing a correlation between a concentration of indoxyl sulfate in urine and a concentration of indoxyl sulfate in plasma of patients of a group to which a sodium bicarbonate preparation has been administered, after 6, 12, and 24 weeks from the start of the test.
FIG. 4 is a graph showing a correlation between a concentration of indoxyl sulfate in urine and a concentration of indoxyl sulfate in plasma of all the patients after 6, 12, and 24 weeks from the start of the test.
FIG. 5 is a graph showing a correlation between a concentration of p-cresyl sulfate in urine and a concentration of p-cresyl sulfate in plasma of patients of a control group after 6, 12, and 24 weeks from the start of the test.
FIG. 6 is a graph showing a correlation between a concentration of p-cresyl sulfate in urine and a concentration of p-cresyl sulfate in plasma of patients of a group to which a combination preparation of hydrates of potassium citrate and sodium citrate has been administered, after 6, 12, and 24 weeks from the start of the test.
FIG. 7 is a graph showing a correlation between a concentration of p-cresyl sulfate in urine and a concentration of p-cresyl sulfate in plasma of patients of a group to which a sodium bicarbonate preparation has been administered, after 6, 12, and 24 weeks from the start of the test.
FIG. 8 is a graph showing a correlation between a concentration of p-cresyl sulfate in urine and a concentration of p-cresyl sulfate in plasma of all the patients after 6, 12, and 24 weeks from the start of the test.
FIG. 9 is a graph showing a correlation between a concentration of hippuric acid in urine and a concentration of hippuric acid in plasma of patients of a control group after 6, 12, and 24 weeks from the start of the test.
FIG. 10 is a graph showing a correlation between a concentration of hippuric acid in urine and a concentration of hippuric acid in plasma of patients of a group to which a combination preparation of hydrates of potassium citrate and sodium citrate has been administered, after 6, 12, and 24 weeks from the start of the test.
FIG. 11 is a graph showing a correlation between a concentration of hippuric acid in urine and a concentration of hippuric acid in plasma of patients of a group to which a sodium bicarbonate preparation has been administered, after 6, 12, and 24 weeks from the start of the test.
FIG. 12 is a graph showing a correlation between a concentration of hippuric acid in urine and a concentration of hippuric acid in plasma of all the patients after 6, 12, and 24 weeks from the start of the test.
FIG. 13 is a graph showing a correlation between a concentration of argininosuccinic acid in urine and a concentration of argininosuccinic acid in plasma of patients of a control group after 6, 12, and 24 weeks from the start of the test.
FIG. 14 is a graph showing a correlation between a concentration of argininosuccinic acid in urine and a concentration of argininosuccinic acid in plasma of patients of a group to which a combination preparation of hydrates of potassium citrate and sodium citrate has been administered, after 6, 12, and 24 weeks from the start of the test.
FIG. 15 is a graph showing a correlation between a concentration of argininosuccinic acid in urine and a concentration of argininosuccinic acid in plasma of patients of a group to which a sodium bicarbonate preparation has been administered, after 6, 12, and 24 weeks from the start of the test.
FIG. 16 is a graph showing a correlation between a concentration of argininosuccinic acid in urine and a concentration of argininosuccinic acid in plasma of all the patients after 6, 12, and 24 weeks from the start of the test.
FIG. 17 is a graph showing a correlation between a concentration of phenylacetyl-L-L-glutamine in urine and a concentration of phenylacetyl-L--L-glutamine in plasma of patients of a control group after 6, 12, and 24 weeks from the start of the test.
FIG. 18 is a graph showing a correlation between a concentration of phenylacetyl-L-L-glutamine in urine and a concentration of phenylacetyl-L--L-glutamine in plasma of patients of a group to which a combination preparation of hydrates of potassium citrate and sodium citrate has been administered, after 6, 12, and 24 weeks from the start of the test.
FIG. 19 is a graph showing a correlation between a concentration of phenylacetyl-L-L-glutamine in urine and a concentration of phenylacetyl-L-glutamine in plasma of patients of a group to which a sodium bicarbonate preparation has been administered, after 6, 12, and 24 weeks from the start of the test.
FIG. 20 is a graph showing a correlation between a concentration of phenylacetyl-L-glutamine in urine and a concentration of phenylacetyl-L-glutamine in plasma of all the patients after 6, 12, and 24 weeks from the start of the test.

### EMBODIMENTS FOR CARRYING OUT THE INVENTION

### 1. Pharmaceutical composition

A pharmaceutical composition provided by the present invention comprises an alkalinizing agent as an active ingredient, wherein the alkalinizing agent is a mixture of sodium citrate or a hydrate thereof, and potassium citrate or a hydrate thereof, as further defined in the claims.

In general, an alkalinizing agent is an agent having the ability to increase the HCO₃⁻ concentration and pH of body fluids of mammals (particularly a human), such as blood or urine. Examples of alkalinizing agents include a pharmaceutically acceptable salt of citric acid or a hydrate thereof, or a mixture thereof, and sodium bicarbonate (baking soda). Examples of a pharmaceutically acceptable salt of citric acid include an alkali metal salt of citric acid. Examples of an alkali metal salt of citric acid include potassium citrate and sodium citrate. Potassium citrate may be a hydrate such as a stable potassium citrate monohydrate (C₆H₅K₃O₇·H₂O), and sodium citrate may be a hydrate such as sodium citrate dihydrate (C₆H₅Na₃O₇·2H₂O). In any case, the alkalinizing agent contained in the the pharmaceutical composition provided by the present invention is a mixture of sodium citrate or a hydrate thereof, and potassium citrate or a hydrate thereof.

For example, the alkalinizing agent may be a mixture of a potassium citrate monohydrate (C₆H₅K₃O₇·H₂O) and sodium citrate dihydrate (C₆H₅Na₃O₇·2H₂O). A mixing ratio of a potassium citrate monohydrate (C₆H₅K₃O₇·H₂O) and a sodium citrate dihydrate (C₆H₅Na₃O₇·2H₂O) can be appropriately set by those skilled in the art. For example, a molar ratio of a potassium citrate monohydrate to a sodium citrate dihydrate can be set to 1 (for a potassium citrate monohydrate) to 0.01 to 100 (for a sodium citrate dihydrate). The mixing ratio may be about 1: 1 as a molar ratio.

The alkalinizing agent contained in the pharmaceutical composition of the present invention may be composed of only a mixture of sodium citrate or a hydrate thereof and potassium citrate or a hydrate thereof.

In the present specification, when referring to the weight of an alkalinizing agent (for example, a potassium citrate monohydrate (C₆H₅K₃O₇·H₂O) and a sodium citrate dihydrate (C₆H₅Na₃O₇·2H₂O)), this weight may be a dry weight.

In the present specification, a uremic substance is a substance (waste products, toxins, and the like) excreted by normal kidneys, and it means a substance that increases (accumulates) in the blood and causes uremic symptoms or diseases when the excretory function deteriorates due to some cause such as deteriorated kidney function. Examples of uremic substance include indoxyl sulfate, p-cresyl sulfate, phenylacetyl-L-glutamine, hippuric acid, and argininosuccinic acid.

Among them, indoxyl sulfate is produced by oxidation and sulfate conjugation of indole produced by enteric bacteria from tryptophan derived from dietary protein. Most of the indoxyl sulfate is present by being bound to albumin in the blood and is not metabolized. In healthy subjects, it is excreted from the kidneys into the urine, but in a case of a patient with kidney disease, it remains accumulated at high concentrations in the blood.

Indoxyl sulfate, which is a uremic substance, not only causes uremia in a patient with kidney disease, but also causes a patient with chronic kidney disease to be subjected to introduction of dialysis.

Accordingly, by reducing a concentration of indoxyl sulfate in blood, the uremic symptoms of a patient with kidney disease are ameliorated, and thereby treatment and/or prevention of uremia becomes possible. In addition, it is possible to delay the introduction of dialysis in a patient with chronic kidney disease by decreasing a concentration of indoxyl sulfate in blood. In one embodiment, a patient with chronic kidney disease has progressive chronic kidney disease.

In the present specification, the expression [A, B, and/or C] represents "at least one selected from the group consisting of A, B, and C." Accordingly, for example, "indoxyl sulfate, p-cresyl sulfate, phenylacetyl-L-glutamine, hippuric acid, and/or argininosuccinic acid" means at least one selected from the group consisting of "indoxyl sulfate, p-cresyl sulfate, phenylacetyl-L-glutamine, hippuric acid, and argininosuccinic acid."

In addition, indoxyl sulfate, which is a uremic substance, causes myocardial fibrosis, arteriosclerosis, vascular smooth muscle cell proliferation, vascular endothelial cell injury, thickening of the arterial wall, calcification of the aorta, and the like, and allows cardiovascular disease (for example, heart failure, myocardial infarction), which is one of the complications in a patient with chronic kidney disease, and/or a stroke, which is a cerebrovascular disease, to develop.

Accordingly, decreasing a concentration of indoxyl sulfate in blood suppresses myocardial fibrosis, arteriosclerosis, vascular smooth muscle cell proliferation, vascular endothelial cell injury, thickening of the arterial wall, calcification of the aorta, and the like, and thereby it becomes possible to treat and/or prevent cardiovascular disease, which is one of the complications in a patient with chronic kidney disease, and/or cerebrovascular disease.

In one aspect, in the pharmaceutical composition provided by the present invention, it is possible to decrease a concentration of uremic substance (for example, indoxyl sulfate, p-cresyl sulfate, phenylacetyl-L-glutamine, hippuric acid, argininosuccinic acid, and a combination thereof) in the blood. Examples of indoxyl sulfate, p-cresyl sulfate, phenylacetyl-L-glutamine, hippuric acid, argininosuccinic acid, and a combination thereof include indoxyl sulfate, p-cresyl sulfate, hippuric acid, and phenylacetyl-L-glutamine; indoxyl sulfate, p-cresyl sulfate, and phenylacetyl-L-glutamine; indoxyl sulfate, hippuric acid, and phenylacetyl-L-glutamine; indoxyl sulfate and p-cresyl sulfate; indoxyl sulfate and hippuric acid; indoxyl sulfate and phenylacetyl-L-glutamine; p-cresyl sulfate and phenylacetyl-L-glutamine; hippuric acid and phenylacetyl-L-glutamine; indoxyl sulfate; p-cresyl sulfate; hippuric acid; and phenylacetyl-L-glutamine.

In the present specification, "decreasing a concentration of uremic substance in the blood" means that the concentration of uremic substance in the blood after administration of the pharmaceutical composition provided by the present invention decreases compared to the concentration of uremic substance in the blood before the administration, or means that the concentration of uremic substance in the blood is decreased by administration of the pharmaceutical composition provided by the present invention, compared to placebo administration.

In one embodiment, administration of the pharmaceutical composition provided by the present invention decreases a concentration of uremic substance (for example, indoxyl sulfate, p-cresyl sulfate, phenylacetyl-L-glutamine, hippuric acid, argininosuccinic acid, and a combination thereof (for example, indoxyl sulfate, p-cresyl sulfate, hippuric acid, and phenylacetyl-L-glutamine; indoxyl sulfate, p-cresyl sulfate, and phenylacetyl-L-glutamine; indoxyl sulfate, hippuric acid, and phenylacetyl-L-glutamine; indoxyl sulfate and p-cresyl sulfate; indoxyl sulfate and hippuric acid; indoxyl sulfate and phenylacetyl-L-glutamine; p-cresyl sulfate and phenylacetyl-L-glutamine; hippuric acid and phenylacetyl-L-glutamine; indoxyl sulfate; p-cresyl sulfate; hippuric acid; or phenylacetyl-L-glutamine)) in the blood compared to before administration, but an amount of decrease thereof is 1% to 5%, 3% to 5%, 1% to 10%, 3% to 10%, 5% to 10%, 1% to 15%, 3% to 15%, 5% to 15%, 1% to 30%, 1% to 40%, 3% to 40%, 5% to 40%, 1% to 50%, 3% to 50%, 5% to 50%, 30% to 50%, 1% to 60%, 5% or more, 10% or more, or 30% or more of a concentration of uremic substance in the blood before administration.

In one embodiment, an amount of decrease in concentration of uremic substance in the blood is calculated by Calculation Equation (1). Amount of decrease in concentration of uremic substance in blood (%) = [(concentration (ng/mL) of uremic substance in blood before administration of pharmaceutical composition - concentration (ng/mL) of uremic substance in blood after administration of pharmaceutical composition)/concentration (ng/mL) of uremic substance in blood before administration of pharamceutical composition] × 100

In one embodiment, continuous administration of the pharmaceutical composition provided by the present invention for 6, 12, or 24 weeks decreases a concentration of uremic substance (for example, indoxyl sulfate, p-cresyl sulfate, phenylacetyl-L-glutamine, hippuric acid, argininosuccinic acid, and a combination thereof (for example, indoxyl sulfate, p-cresyl sulfate, hippuric acid, and phenylacetyl-L-glutamine; indoxyl sulfate, p-cresyl sulfate, and phenylacetyl-L-glutamine; indoxyl sulfate, hippuric acid, and phenylacetyl-L-glutamine; indoxyl sulfate and p-cresyl sulfate; indoxyl sulfate and hippuric acid; indoxyl sulfate and phenylacetyl-L-glutamine; p-cresyl sulfate and phenylacetyl-L-glutamine; hippuric acid and phenylacetyl-L-glutamine; indoxyl sulfate; p-cresyl sulfate; hippuric acid; or phenylacetyl-L-glutamine)) in the blood compared to before administration, but an amount of decrease thereof is 1% to 5%, 3% to 5%, 1% to 10%, 3% to 10%, 5% to 10%, 1% to 15%, 3% to 15%, 5% to 15%, 1% to 30%, 1% to 40%, 3% to 40%, 5% to 40%, 1% to 50%, 3% to 50%, 5% to 50%, 30% to 50%, 1% to 60%, 5% or more, 10% or more, or 30% or more of a concentration of uremic substance in the blood before administration.

In one aspect, in the pharmaceutical composition provided by the present invention, it is possible to promote excretion of uremic substance (for example, indoxyl sulfate, p-cresyl sulfate, phenylacetyl-L-glutamine, hippuric acid, argininosuccinic acid, and a combination thereof) into urine. Examples of indoxyl sulfate, p-cresyl sulfate, phenylacetyl-L-glutamine, hippuric acid, argininosuccinic acid, and a combination thereof include indoxyl sulfate, p-cresyl sulfate, phenylacetyl-L-glutamine, hippuric acid, and argininosuccinic acid; indoxyl sulfate, p-cresyl sulfate, phenylacetyl-L-glutamine, and argininosuccinic acid; indoxyl sulfate, p-cresyl sulfate, hippuric acid, and phenylacetyl-L-glutamine; indoxyl sulfate, p-cresyl sulfate, and phenylacetyl-L-glutamine; indoxyl sulfate, phenylacetyl-L-glutamine, and argininosuccinic acid; indoxyl sulfate, hippuric acid, and phenylacetyl-L-glutamine; indoxyl sulfate and hippuric acid; indoxyl sulfate and phenylacetyl-L-glutamine; hippuric acid and phenylacetyl-L-glutamine; indoxyl sulfate and p-cresyl sulfate; p-cresyl sulfate and phenylacetyl-L-glutamine; p-cresyl sulfate and hippuric acid; indoxyl sulfate and argininosuccinic acid; p-cresyl sulfate and argininosuccinic acid; hippuric acid and argininosuccinic acid; phenylacetyl-L-glutamine and argininosuccinic acid; indoxyl sulfate; p-cresyl sulfate; hippuric acid; argininosuccinic acid; and phenylacetyl-L-glutamine.

In the present specification, "promoting excretion of uremic substance into the urine" means that a concentration of uremic substance in the urine after administration increases compared to the concentration of uremic substance in the urine before administration of the pharmaceutical composition provided by the present invention; means that a concentration of uremic substance in the urine increases by administration of the pharmaceutical composition provided by the present invention compared to placebo administration; means that an amount of uremic substance in the urine after administration increases compared to the amount of uremic substance in urine before administration of the pharmaceutical composition provided by the present invention; or means that the amount of uremic substance in the urine increases by administration of the pharmaceutical composition provided by the present invention compared to placebo administration.

In the present specification, "in urine" means, for example, "in early morning urine."

In one embodiment, by administration of the pharmaceutical composition provided by the present invention, a concentration of uremic substance (for example, indoxyl sulfate, p-cresyl sulfate, phenylacetyl-L-glutamine, hippuric acid, argininosuccinic acid, and a combination thereof (for example, indoxyl sulfate, p-cresyl sulfate, phenylacetyl-L-glutamine, hippuric acid, and argininosuccinic acid; indoxyl sulfate, p-cresyl sulfate, phenylacetyl-L-glutamine, and argininosuccinic acid; indoxyl sulfate, p-cresyl sulfate, hippuric acid, and phenylacetyl-L-glutamine; indoxyl sulfate, p-cresyl sulfate, and phenylacetyl-L-glutamine; indoxyl sulfate, phenylacetyl-L-glutamine, and argininosuccinic acid; indoxyl sulfate, hippuric acid, and phenylacetyl-L-glutamine; indoxyl sulfate and hippuric acid; indoxyl sulfate and phenylacetyl-L-glutamine; hippuric acid and phenylacetyl-L-glutamine; indoxyl sulfate and p-cresyl sulfate; p-cresyl sulfate and phenylacetyl-L-glutamine; p-cresyl sulfate and hippuric acid; indoxyl sulfate and argininosuccinic acid; p-cresyl sulfate and argininosuccinic acid; hippuric acid and argininosuccinic acid; phenylacetyl-L-glutamine and argininosuccinic acid; indoxyl sulfate; p-cresyl sulfate; hippuric acid; argininosuccinic acid; or phenylacetyl-L-glutamine)) in urine increases by 1% to 100%, 1% to 50%, 3% to 50%, 5% to 50%, 10% to 50%, 15% to 50%, 1% to 40%, 5% to 40%, 10% to 40%, 1% to 30%, 5% to 30%, 10% to 30%, 15% to 30%, 10% or more, 20% or more, 30% or more, or 40% or more, compared to before administration.

In one embodiment, the amount of increase in concentration of uremic substance in urine is calculated by Calculation Equation (2). Amount of increase in concentration of uremic substance in urine (%) = [(concentration (ng/mL) of uremic substance in urine after administration of pharmaceutical composition - concentration (ng/mL) of uremic substance in urine before administration of pharmaceutical composition)/concentration (ng/mL) of uremic substance in urine before administration of pharmaceutical composition] × 100

In one embodiment, by continuous administration of the pharmaceutical composition provided by the present invention for 6, 12, or 24 weeks, a concentration of uremic substance (for example, indoxyl sulfate, p-cresyl sulfate, phenylacetyl-L-glutamine, hippuric acid, argininosuccinic acid, and a combination thereof (for example, indoxyl sulfate, p-cresyl sulfate, phenylacetyl-L-glutamine, hippuric acid, and argininosuccinic acid; indoxyl sulfate, p-cresyl sulfate, phenylacetyl-L-glutamine, and argininosuccinic acid; indoxyl sulfate, p-cresyl sulfate, hippuric acid, and phenylacetyl-L-glutamine; indoxyl sulfate, p-cresyl sulfate, and phenylacetyl-L-glutamine; indoxyl sulfate, phenylacetyl-L-glutamine, and argininosuccinic acid; indoxyl sulfate, hippuric acid, and phenylacetyl-L-glutamine; indoxyl sulfate and hippuric acid; indoxyl sulfate and phenylacetyl-L-glutamine; hippuric acid and phenylacetyl-L-glutamine; indoxyl sulfate and p-cresyl sulfate; p-cresyl sulfate and phenylacetyl-L-glutamine; p-cresyl sulfate and hippuric acid; indoxyl sulfate and argininosuccinic acid; p-cresyl sulfate and argininosuccinic acid; hippuric acid and argininosuccinic acid; phenylacetyl-L-glutamine and argininosuccinic acid; indoxyl sulfate; p-cresyl sulfate; hippuric acid; argininosuccinic acid; or phenylacetyl-L-glutamine)) in urine increases by 1% to 100%, 1% to 50%, 3% to 50%, 5% to 50%, 10% to 50%, 15% to 50%, 1% to 40%, 5% to 40%, 10% to 40%, 1% to 30%, 5% to 30%, 10% to 30%, 15% to 30%, 10% or more, 20% or more, 30% or more, or 40% or more, compared to before administration.

In one aspect, in the pharmaceutical composition provided by the present invention, it is possible to promote excretion of uremic substance (for example, indoxyl sulfate, p-cresyl sulfate, phenylacetyl-L-glutamine, hippuric acid, argininosuccinic acid, and a combination thereof) from blood into urine, thereby promoting excretion of the uremic substance outside the body. Examples of indoxyl sulfate, p-cresyl sulfate, phenylacetyl-L-glutamine, hippuric acid, argininosuccinic acid, and a combination thereof include indoxyl sulfate, p-cresyl sulfate, phenylacetyl-L-glutamine, hippuric acid, and argininosuccinic acid; indoxyl sulfate, p-cresyl sulfate, phenylacetyl-L-glutamine, and argininosuccinic acid; indoxyl sulfate, p-cresyl sulfate, hippuric acid, and phenylacetyl-L-glutamine; indoxyl sulfate, p-cresyl sulfate, and phenylacetyl-L-glutamine; indoxyl sulfate, phenylacetyl-L-glutamine, and argininosuccinic acid; indoxyl sulfate, hippuric acid, and phenylacetyl-L-glutamine; indoxyl sulfate and hippuric acid; indoxyl sulfate and phenylacetyl-L-glutamine; hippuric acid and phenylacetyl-L-glutamine; indoxyl sulfate and p-cresyl sulfate; p-cresyl sulfate and phenylacetyl-L-glutamine; p-cresyl sulfate and hippuric acid; indoxyl sulfate and argininosuccinic acid; p-cresyl sulfate and argininosuccinic acid; hippuric acid and argininosuccinic acid; phenylacetyl-L-glutamine and argininosuccinic acid; indoxyl sulfate; p-cresyl sulfate; hippuric acid; argininosuccinic acid; and phenylacetyl-L-glutamine.

In one embodiment, by administration of the pharmaceutical composition provided by the present invention, a ratio of a concentration of uremic substance in urine to a concentration of uremic substance in blood after administration increases compared to a ratio of a concentration of uremic substance in urine to a concentration of uremic substance in blood before administration of the pharmaceutical composition provided by the present invention. In one embodiment, administration of the pharmaceutical composition provided by the present invention increases the ratio of a concentration of uremic substance in urine to a concentration of uremic substance in blood, compared to placebo administration.

In one embodiment, by administration of the pharmaceutical composition provided by the present invention, a ratio of a concentration of uremic substance (for example, indoxyl sulfate, p-cresyl sulfate, phenylacetyl-L-glutamine, hippuric acid, argininosuccinic acid, and a combination thereof (for example, indoxyl sulfate, p-cresyl sulfate, phenylacetyl-L-glutamine, hippuric acid, and argininosuccinic acid; indoxyl sulfate, p-cresyl sulfate, phenylacetyl-L-glutamine, and argininosuccinic acid; indoxyl sulfate, p-cresyl sulfate, hippuric acid, and phenylacetyl-L-glutamine; indoxyl sulfate, p-cresyl sulfate, and phenylacetyl-L-glutamine; indoxyl sulfate, phenylacetyl-L-glutamine, and argininosuccinic acid; indoxyl sulfate, hippuric acid, and phenylacetyl-L-glutamine; indoxyl sulfate and hippuric acid; indoxyl sulfate and phenylacetyl-L-glutamine; hippuric acid and phenylacetyl-L-glutamine; indoxyl sulfate and p-cresyl sulfate; p-cresyl sulfate and phenylacetyl-L-glutamine; p-cresyl sulfate and hippuric acid; indoxyl sulfate and argininosuccinic acid; p-cresyl sulfate and argininosuccinic acid; hippuric acid and argininosuccinic acid; phenylacetyl-L-glutamine and argininosuccinic acid; indoxyl sulfate; p-cresyl sulfate; hippuric acid; argininosuccinic acid; or phenylacetyl-L-glutamine)) in urine with respect to a concentration of uremic substance in blood (a concentration (ng/mL) in urine/a concentration (ng/mL) in blood) increases by 1% to 100%, 1% to 50%, 3% to 50%, 5% to 50%, 10% to 50%, 15% to 50%, 1% to 40%, 5% to 40%, 10% to 40%, 1% to 30%, 5% to 30%, 10% to 30%, 15% to 30%, 10% or more, 20% or more, 30% or more, or 40% or more, compared to before administration.

In one embodiment, an increase in ratio of a concentration of uremic substance in urine to a concentration of uremic substance in blood (a concentration (ng/mL) in urine/a concentration (ng/mL) in blood) is calculated by Calculation Equation (3). Amount of increase in ratio of a concentration of uremic substance in urine to a concentration of uremic substance in blood (%) = [(ratio of concentration of uremic substance in urine to concentration of uremic substance in blood after administration of pharmaceutical composition - ratio of concentration of uremic substance in urine to concentration of uremic substance in blood before administration of pharmaceutical composition)/ratio of concentration of uremic substance in urine to concentration of uremic substance in blood before administration of pharmaceutical composition] × 100

In one embodiment, by continuous administration of the pharmaceutical composition provided by the present invention for 6, 12, or 24 weeks, a ratio of a concentration of uremic substance (for example, indoxyl sulfate, p-cresyl sulfate, phenylacetyl-L-glutamine, hippuric acid, argininosuccinic acid, and a combination thereof (for example, indoxyl sulfate, p-cresyl sulfate, phenylacetyl-L-glutamine, hippuric acid, and argininosuccinic acid; indoxyl sulfate, p-cresyl sulfate, phenylacetyl-L-glutamine, and argininosuccinic acid; indoxyl sulfate, p-cresyl sulfate, hippuric acid, and phenylacetyl-L-glutamine; indoxyl sulfate, p-cresyl sulfate, and phenylacetyl-L-glutamine; indoxyl sulfate, phenylacetyl-L-glutamine, and argininosuccinic acid; indoxyl sulfate, hippuric acid, and phenylacetyl-L-glutamine; indoxyl sulfate and hippuric acid; indoxyl sulfate and phenylacetyl-L-glutamine; hippuric acid and phenylacetyl-L-glutamine; indoxyl sulfate and p-cresyl sulfate; p-cresyl sulfate and phenylacetyl-L-glutamine; p-cresyl sulfate and hippuric acid; indoxyl sulfate and argininosuccinic acid; p-cresyl sulfate and argininosuccinic acid; hippuric acid and argininosuccinic acid; phenylacetyl-L-glutamine and argininosuccinic acid; indoxyl sulfate; p-cresyl sulfate; hippuric acid; argininosuccinic acid; or phenylacetyl-L-glutamine)) in urine with respect to a concentration of uremic substance in blood (a concentration (ng/mL) in urine/a concentration (ng/mL) in blood) increases by 1% to 100%, 1% to 50%, 3% to 50%, 5% to 50%, 10% to 50%, 15% to 50%, 1% to 40%, 5% to 40%, 10% to 40%, 1% to 30%, 5% to 30%, 10% to 30%, 15% to 30%, 10% or more, 20% or more, 30% or more, or 40% or more, compared to before administration.

In one embodiment, the administration of the pharmaceutical composition provided by the present invention promotes excretion of the uremic substance outside the body depending on a concentration of uremic substance in blood.

In one embodiment, the administration of the pharmaceutical composition provided by the present invention promotes excretion of uremic substance (for example, indoxyl sulfate, p-cresyl sulfate, phenylacetyl-L-glutamine, hippuric acid, argininosuccinic acid, and a combination thereof) into urine depending on a concentration of uremic substance in blood. For example, in a case where a concentration of uremic substance in blood is high, the amount of uremic substance excreted into the urine becomes a high value in accordance with the high concentration of uremic substance in blood. In a case where the concentration of uremic substance in blood is low, the amount of uremic substance excreted into the urine becomes a low value. Such excretion of uremic substance into urine depending on a concentration of uremic substance in blood suggests that the pharmaceutical composition provided by the present invention has a low risk of side effects and excellent safety. Examples of indoxyl sulfate, p-cresyl sulfate, phenylacetyl-L-glutamine, hippuric acid, argininosuccinic acid, and a combination thereof include indoxyl sulfate, p-cresyl sulfate, phenylacetyl-L-glutamine, hippuric acid, and argininosuccinic acid; indoxyl sulfate, p-cresyl sulfate, and phenylacetyl-L-glutamine; indoxyl sulfate and phenylacetyl-L-glutamine; indoxyl sulfate and p-cresyl sulfate; p-cresyl sulfate and phenylacetyl-L-glutamine; indoxyl sulfate; p-cresyl sulfate; and phenylacetyl-L-glutamine.

In one embodiment, the administration of the pharmaceutical composition provided by the present invention promotes excretion of uremic substance (for example, indoxyl sulfate, p-cresyl sulfate, phenylacetyl-L-glutamine, hippuric acid, argininosuccinic acid, and a combination thereof) into urine depending on a concentration of uremic substance in blood, and thereby a concentration of uremic substance in blood is decreased. Examples of indoxyl sulfate, p-cresyl sulfate, phenylacetyl-L-glutamine, hippuric acid, argininosuccinic acid, and a combination thereof include indoxyl sulfate, p-cresyl sulfate, phenylacetyl-L-glutamine, hippuric acid, and argininosuccinic acid; indoxyl sulfate, p-cresyl sulfate, and phenylacetyl-L-glutamine; indoxyl sulfate and phenylacetyl-L-glutamine; indoxyl sulfate and p-cresyl sulfate; p-cresyl sulfate and phenylacetyl-L-glutamine; indoxyl sulfate; p-cresyl sulfate; and phenylacetyl-L-glutamine.

In one embodiment, administration of the pharmaceutical composition provided by the present invention excretes indoxyl sulfate into the urine depending on a concentration of indoxyl sulfate in blood, and as a result, a ratio of a concentration of indoxyl sulfate in urine to a concentration of indoxyl sulfate in blood (a concentration in urine/a concentration in blood) can become 1 to 1000, preferably 1 to 200, more preferably 1 to 100, and even more preferably 10 to 100. In this embodiment, the pharmaceutical composition provided by the present invention may be administered to a human (for example, a patient with chronic kidney disease) in which a concentration of indoxyl sulfate in blood is 0.01 to 100 µg/mL (for example, 0.1 to 30 µg/mL). Furthermore, as a result of the administration, the concentration of indoxyl sulfate in blood may become 0.01 to 10 µg/mL (for example, 0.03 to 10 µg/mL).

In one embodiment, administration of the pharmaceutical composition provided by the present invention excretes p-cresyl sulfate into the urine depending on a concentration of p-cresyl sulfate in blood, and as a result, a ratio of a concentration of p-cresyl sulfate in urine to a concentration of p-cresyl sulfate in blood (a concentration in urine/a concentration in blood) can become 0.1 to 1000, preferably 1 to 300, more preferably 1 to 150, and even more preferably 1 to 100. In this embodiment, the pharmaceutical composition provided by the present invention may be administered to a human (for example, a patient with chronic kidney disease) in which a concentration of p-cresyl sulfate in blood is 0.003 to 300 µg/mL (for example, 0.01 to 30 µg/mL). Furthermore, as a result of the administration, the concentration of p-cresyl sulfate in blood may become 0.001 to 100 µg/mL (for example, 0.001 to 30 µg/mL).

In one embodiment, administration of the pharmaceutical composition provided by the present invention excretes phenylacetyl-L-glutamine into the urine depending on a concentration of phenylacetyl-L-glutamine in blood, and as a result, a ratio of a concentration of phenylacetyl-L-glutamine in urine to a concentration of phenylacetyl-L-glutamine in blood (a concentration in urine/a concentration in blood) can become 1 to 1500, preferably 1 to 1000, more preferably 1 to 800, and even more preferably 10 to 600. In this embodiment, the pharmaceutical composition provided by the present invention may be administered to a human (for example, a patient with chronic kidney disease) in which a concentration of phenylacetyl-L-glutamine in blood is 0.03 to 30 µg/mL (for example, 0.1 to 10 µg/mL). Furthermore, as a result of the administration, the concentration of phenylacetyl-L-glutamine in blood may become 0.01 to 10 µg/mL (for example, 0.03 to 10 µg/mL).

In one embodiment, the pharmaceutical composition provided by the present invention is administered to a plurality of humans (for example, patients with chronic kidney disease), and a ratio of a concentration of indoxyl sulfate in urine to a concentration of indoxyl sulfate in blood in each individual (a concentration in urine/a concentration in blood) shows high correlation. In this embodiment, high correlation may be indicated by a Pearson test in which an r value is 0.4 to 1, 0.5 to 1, 0.6 to 1, or 0.7 to 1 (preferably 0.7 to 1). In addition, the pharmaceutical composition provided by the present invention may be administered to a human (for example, a patient with chronic kidney disease) in which a concentration of indoxyl sulfate in blood is 0.01 to 10 µg/mL (for example, 0.1 to 10 µg/mL), and as a result of the administration, a concentration of indoxyl sulfate in blood may become 0.01 to 10 µg/mL (for example, 0.1 to 10 µg/mL).

In one embodiment, the pharmaceutical composition provided by the present invention is administered to a plurality of humans (for example, patients with chronic kidney disease), and a ratio of a concentration of p-cresyl sulfate in urine to a concentration of p-cresyl sulfate in blood in each individual (a concentration in urine/a concentration in blood) shows high correlation. In this embodiment, high correlation may be indicated by a Pearson test in which an r value is 0.4 to 1, 0.5 to 1, 0.6 to 1, or 0.7 to 1 (preferably 0.7 to 1). In addition, the pharmaceutical composition provided by the present invention may be administered to a human (for example, a patient with chronic kidney disease) in which a concentration of p-cresyl sulfate in blood is 0.001 to 100 µg/mL (for example, 0.01 to 50 µg/mL), and as a result of the administration, a concentration of p-cresyl sulfate in blood may become 0.001 to 100 µg/mL (for example, 0.01 to 50 µg/mL).

In one embodiment, the pharmaceutical composition provided by the present invention is administered to a plurality of humans (for example, patients with chronic kidney disease), and a ratio of a concentration of phenylacetyl-L-glutamine in urine to a concentration of phenylacetyl-L-glutamine in blood in each individual (a concentration in urine/a concentration in blood) shows high correlation. In this embodiment, high correlation may be indicated by a Pearson test in which an r value is 0.4 to 1, 0.5 to 1, 0.6 to 1, or 0.7 to 1 (preferably 0.7 to 1). In addition, the pharmaceutical composition provided by the present invention may be administered to a human (for example, a patient with chronic kidney disease) in which a concentration of phenylacetyl-L-glutamine in blood is 0.01 to 10 µg/mL (for example, 0.05 to 10 µg/mL), and as a result of the administration, a concentration of phenylacetyl-L-glutamine in blood may become 0.01 to 10 µg/mL (for example, 0.05 to 10 µg/mL).

The pharmaceutical composition for the use according to the present invention is a pharmaceutical composition comprising an alkalinizing agent for use in treating or preventing kidney tubular damage as further defined in claim 1 or a pharmaceutical composition comprising an alkalinizing agent for use in suppressing progression of chronic kidney disease as further defined in claim 2. Because of the characteristics of the pharmaceutical composition, the pharmaceutical composition not only can be used as a pharmaceutical composition for decreasing a concentration of uremic substance in blood and/or a pharmaceutical composition for promoting excretion of uremic substance into urine, but also can be used as any of a pharmaceutical composition for ameliorating uremic symptoms in a patient with kidney disease, a pharmaceutical composition for treating and/or preventing uremic symptoms in a patient with kidney disease, a pharmaceutical composition for suppressing progression of chronic kidney disease, and a pharmaceutical composition for delaying introduction of dialysis in a patient with chronic kidney disease.

In the present specification, the term "amelioration" is a concept encompassing bringing "pathological" or "abnormal" symptoms, conditions, or diseases closer to "healthy" or "normal" conditions, or action therefor; and making "pathological" or "abnormal" symptoms, conditions, or diseases "healthy" or "normal" conditions, or action therefor. Accordingly, in one embodiment, the term "amelioration" encompasses a concept in which a numerical value indicative of "pathological" or "abnormal" symptoms or conditions decreases or increases in accordance with the "amelioration," and becomes closer to a normal value or becomes a normal value. In another embodiment, the term "amelioration" encompasses a concept in which, in accordance with the "amelioration," a concentration of uremic substance in blood is decreased, a concentration of uremic substance in urine increases, and a concentration of uremic substance in urine may turn to a decrease when a concentration of uremic substance in blood becomes sufficiently small.

In the present specification, the term "healthy" represents a state in which there is no acute or chronic disease or disorder, and the term "normal" represents in a state that a healthy subject usually expresses.

In the present specification, the term "treatment" encompasses a concept in which "pathological" or "abnormal" symptoms, conditions, or diseases are eliminated, completely cured, healed, or in remission, and action therefor; a deterioration in "pathological" or "abnormal" symptoms, conditions, or diseases is suppressed, and action therefor; and the "amelioration." The term suppression has the meaning to be described later. In one embodiment, the term "treatment" means that "pathological" or "abnormal" symptoms, conditions, or diseases are eliminated, completely cured, healed, or in remission, and means action therefor. In another embodiment, the term "treatment" means that "pathological" or "abnormal" symptoms, conditions, or diseases are eliminated, completely cured, healed, or in remission.

In the present specification, the term "prevention" encompasses a concept in which onset of "pathological" or "abnormal" symptoms, conditions, or diseases is prevented before they occur, and action therefor.

In the present specification, the term "delay" encompasses a concept in which a time for an object phenomenon to occur is extended, and action therefor; and a time is extended so that the object phenomenon does not occur.

In the present specification, the term "suppression" encompasses a concept in which a deterioration or progression of symptoms, conditions, or diseases is stopped or slowed down, or action therefor; and the symptoms, conditions, or the diseases are ameliorated, or action therefor. The term amelioration has the meaning as described above. The above-mentioned "deterioration or progression of symptoms, conditions, or diseases" includes a deterioration or progression of "pathological" or "abnormal" symptoms, conditions, or diseases; and a deterioration or progression of "pathological" or "abnormal" symptoms, conditions, or diseases from "healthy" or "normal" conditions. In one embodiment, the term "suppression" means that a deterioration or progression of symptoms, conditions, or diseases is stopped or slowed down, or means action therefor. In another embodiment, the term "suppression" means that a deterioration or progression of symptoms, conditions, or diseases is stopped or slowed down.

The symptoms, conditions, or diseases are compared before and after administration of the pharmaceutical composition provided by the present invention.

In addition, because of the characteristics of the pharmaceutical composition as described above, the pharmaceutical composition can be used as any of a pharmaceutical composition for suppressing myocardial fibrosis in a patient with kidney disease, a pharmaceutical composition for suppressing arteriosclerosis in a patient with kidney disease, a pharmaceutical composition for suppressing vascular smooth muscle cell proliferation in a patient with kidney disease, a pharmaceutical composition for suppressing vascular endothelial cell injury in a patient with kidney disease, a pharmaceutical composition for suppressing thickening of the arterial wall of a patient with kidney disease, a pharmaceutical composition for suppressing calcification of the aorta of a patient with kidney disease, and a pharmaceutical composition for treating and/or preventing cardiovascular disease associated with chronic kidney disease.

In addition, it has been reported that, when a drug that decreases a concentration of indoxyl sulfate in blood was administered to a patient with non-diabetic chronic kidney disease, the pulse wave velocity and carotid intima-media complex thickness, which are indicators of arteriosclerosis, were significantly improved compared to those before administration (Nakamura T., et al.: Oral ADSORBENT AST-120 decreases carotid intima-media thickness and arterial stiffness in patients with chronic renal failure. Kidney Blood Press Res, 27:121-6, 2004.). Accordingly, a pharmaceutical composition which decreases the concentration of indoxyl sulfate in blood can be used as a pharmaceutical composition for ameliorating arteriosclerosis or a pharmaceutical composition for ameliorating thickening of the arterial wall (for example, carotid artery) of a patient with kidney disease (preferably a patient with chronic kidney disease, more preferably a patient with non-diabetic chronic kidney disease).

In addition, drugs that decrease the concentration of indoxyl sulfate in blood have been reported to suppress cisplatin-induced acute kidney injury (Morisaki T., et. Al.,: Regulation of renal organic ion transporters in cisplatin-induced acute kidney injury and uremia in rats. Pharm. Res., 25 (11): 2526-33, 2008). Accordingly, a pharmaceutical composition which decreases the concentration of indoxyl sulfate in blood can be used as a pharmaceutical composition for treating acute kidney failure, or a pharmaceutical composition for suppressing progression from acute kidney failure to chronic kidney disease.

In addition, p-cresyl sulfate, which is a uremic substance, has been reported to be a causative substance of vascular endothelial damage (Meijers B.K., et. Al.,: The uremic retention solute p-cresyl sulfate and markers of endothelial damage., Am. J. Kidney Dis., 54: 891-901, 2009).

Accordingly, a pharmaceutical composition which promotes excretion of p-cresyl sulfate into urine can be used as a pharmaceutical composition for suppressing vascular endothelial damage in a patient with kidney disease (preferably a patient with chronic kidney disease).

In addition, phenylacetyl-L-glutamine, which is a uremic substance, has been reported to increase the risk of developing cardiovascular disease in a patient with chronic kidney disease.

Accordingly, a pharmaceutical composition provided which promotes excretion of phenylacetyl-L-glutamine into urine can be used as a pharmaceutical composition for treating and/or preventing cardiovascular disease in a patient with chronic kidney disease.

Since the pharmaceutical composition promotes excretion of uremic substance into urine, such as indoxyl sulfate, p-cresyl sulfate, hippuric acid, argininosuccinic acid, and phenylacetyl-L-glutamine, the pharmaceutical composition can be used as a pharmaceutical composition for promoting excretion of indoxyl sulfate, p-cresyl sulfate, hippuric acid, argininosuccinic acid, and/or phenylacetyl-L-glutamine into urine of a patient with kidney disease (preferably a patient with chronic kidney disease).

In one aspect, the pharmaceutical composition provided by the present invention can be used as a pharmaceutical composition for treating kidney tubular damage, a pharmaceutical composition for preventing kidney tubular damage, or a pharmaceutical composition for suppressing kidney tubular damage. A kidney tubule may be, for example, a kidney proximal tubule.

In another aspect, the pharmaceutical composition provided by the present invention can be used as a pharmaceutical composition for maintaining kidney function.

In one embodiment, the pharmaceutical composition provided by the present invention is a pharmaceutical composition for suppressing kidney tubular cell damage, a pharmaceutical composition for protecting kidney tubular cells, or a pharmaceutical composition for maintaining kidney tubular cell function (for example, reabsorption of water, sodium ion, potassium ion, calcium ion, phosphate ion, bicarbonate ion, chloride ion, glucose, amino acid, vitamin, and the like).

In one embodiment, the pharmaceutical composition provided by the present invention is a pharmaceutical composition for suppressing kidney proximal tubular cell damage, a pharmaceutical composition for protecting kidney proximal tubular cells, or a pharmaceutical composition for maintaining kidney proximal tubular cell function (for example, reabsorption of glucose, amino acid, vitamin, and the like).

In one embodiment, the pharmaceutical composition provided by the present invention suppresses an increase in the amount (concentration) of β2-microglobulin in urine (for example, early morning urine) associated with progression of a stage of chronic kidney disease.

In one embodiment, the pharmaceutical composition provided by the present invention does not affect the glomerular function of a patient with chronic kidney disease, whereas it suppresses kidney proximal tubular cell damage associated with progression of a stage of chronic kidney disease to protect kidney proximal tubular cells.

In the present specification, "maintenance of kidney function" means, for example, suppression of kidney tubular damage, protection of kidney tubular cells, or maintenance of kidney tubular function. A kidney tubule may be, for example, a kidney proximal tubule, and one aspect of maintenance of kidney tubular function or kidney proximal tubular function is to maintain kidney tubular cell function or to maintain kidney proximal tubular cell function.

In the present specification, "protection of cells" means maintenance or preservation of cell states or suppression of cell damage. The term suppression has the above-mentioned meaning.

In the present specification, "maintenance of cell function" means preservation of cell function or suppression of deterioration of cell function. The term suppression has the above-mentioned meaning.

The state or function of cells is compared before and after administration of the pharmaceutical composition provided by the present invention.

In the present specification, "early morning urine" represents the first urine after getting up.

In one embodiment, the pharmaceutical composition provided by the present invention suppresses an increase in the amount (concentration) of β2-microglobulin in urine (for example, early morning urine) 6 weeks, 12 weeks, and/or 24 weeks after administration, as compared with that before the start of administration.

In one embodiment, the pharmaceutical composition provided by the present invention suppresses an increase in the amount (concentration) of β2-microglobulin in urine (for example, early morning urine) associated with progression of a stage of chronic kidney disease, but an amount (concentration) of β2-microglobulin in urine (for example, early morning urine) is not substantially decreased compared to that before the start of administration of the pharmaceutical composition provided by the present invention. In this embodiment, for example, in a case where the amount (concentration) of β2-microglobulin in urine (for example, early morning urine) before the start of administration of the pharmaceutical composition provided by the present invention is 1, the amount (concentration) of β2-microglobulin in urine (for example, early morning urine) after administration may be 0.7 to 1.0 or 1.0 or more, 0.8 to 1.0 or 1.0 or more, 0.85 to 1.0 or 1.0 or more, 0.9 to 1.0 or 1.0 or more 0.7 to 2.0, 0.8 to 2.0, 0.85 to 2.0, 0.9 to 2.0, 0.7 to 1.6, 0.8 to 1.6, 0.85 to 1.6, or 0.9 to 1.6.

In one embodiment, the pharmaceutical composition provided by the present invention suppresses an increase in the amount (concentration) of β2-microglobulin in urine (for example, early morning urine) associated with progression of a stage of chronic kidney disease, but an amount (concentration) of β2-microglobulin in urine (for example, early morning urine) is not substantially decreased 6 weeks after administration, 12 weeks after administration, and/or 24 weeks after administration, compared to that before the start of administration of the pharmaceutical composition provided by the present invention. In this embodiment, for example, in a case where the amount (concentration) of β2-microglobulin in urine (for example, early morning urine) before the start of administration of the pharmaceutical composition provided by the present invention is 1, the amount (concentration) of β2-microglobulin in urine (for example, early morning urine) 6 weeks after administration, 12 weeks after administration, or 24 weeks after administration may be 0.7 to 1.0 or 1.0 or more, 0.8 to 1.0 or 1.0 or more, 0.85 to 1.0 or 1.0 or more, 0.9 to 1.0 or 1.0 or more 0.7 to 2.0, 0.8 to 2.0, 0.85 to 2.0, 0.9 to 2.0, 0.7 to 1.6, 0.8 to 1.6, 0.85 to 1.6, or 0.9 to 1.6.

In one embodiment, the pharmaceutical composition provided by the present invention does not substantially increase the amount (concentration) of cystatin C in the blood (for example, plasma) compared to before the start of administration. In this embodiment, for example, in a case where the amount (concentration) of cystatin C in blood (for example, in plasma) before the start of administration of the pharmaceutical composition provided by the present invention is 1, the amount (concentration) of cystatin C in blood (for example, in plasma) after administration may be 1.0 or less or 1.0 to 1.2, 1.0 or less or 1.0 to 1.15, 1.0 or less or 1.0 to 1.1, 1.0 or less or 1.0 to 1.05, 0.9 to 1.2, 0.9 to 1.15, 0.9 to 1.1, 0.9 to 1.05, 0.95 to 1.2, 0.95 to 1.15, 0.95 to 1.1, or 0.95 to 1.05.

In one embodiment, the pharmaceutical composition provided by the present invention does not substantially increase the amount (concentration) of cystatin C in the blood (for example, plasma) 6 weeks after administration, 12 weeks after administration, and/or 24 weeks after administration, compared to before the start of administration. In this embodiment, for example, in a case where the amount (concentration) of cystatin C in blood (for example, in plasma) before the start of administration of the pharmaceutical composition provided by the present invention is 1, the amount (concentration) of cystatin C in blood (for example, in plasma) 6 weeks after administration, 12 weeks after administration, or 24 weeks after administration may be 1.0 or less or 1.0 to 1.2, 1.0 or less or 1.0 to 1.15, 1.0 or less or 1.0 to 1.1, 1.0 or less or 1.0 to 1.05, 0.9 to 1.2, 0.9 to 1.15, 0.9 to 1.1, 0.9 to 1.05, 0.95 to 1.2, 0.95 to 1.15, 0.95 to 1.1, or 0.95 to 1.05.

In one embodiment, by administration of the pharmaceutical composition provided by the present invention, despite no amelioration in kidney proximal tubular damage and/or glomerular damage is recognized 6 weeks after administration, 12 weeks after administration, and/or 24 weeks after administration as compared to before the start of administration, a decrease in the blood concentration of the uremic substance, promotion of urinary excretion of the uremic substance, and/or promotion of excretion of the uremic substance outside of the body is observed 6 weeks after administration, 12 weeks after administration, and/or 24 weeks after administration, as compared to before the start of administration.

In one embodiment, by administration of the pharmaceutical composition provided by the present invention, despite no amelioration in kidney proximal tubular damage and/or glomerular damage is recognized 6 weeks after administration, 12 weeks after administration, and/or 24 weeks after administration as compared to before the start of administration, a decrease in the blood concentration of the uremic substance, promotion of urinary excretion of the uremic substance, and/or promotion of excretion of the uremic substance outside the body is observed 6 weeks after administration, 12 weeks after administration, and/or 24 weeks after administration, as compared to before the start of administration.

The pharmaceutical composition provided by the present invention is orally administered to a human or other mammals.

The pharmaceutical composition provided by the present invention may be prepared as an alkalinizing agent as it is or may be prepared by mixing an alkalinizing agent with a pharmaceutically acceptable carrier such as excipients (for example, lactose, D-mannitol, crystalline cellulose, and glucose), binders (for example, hydroxypropylcellulose (HPC), gelatin, and polyvinylpyrrolidone (PVP)), lubricants (for example, magnesium stearate, and talc), disintegrants (for example, starch and carboxymethylcellulose calcium (CMC-Ca)), diluents (for example, water for injection and saline), and other additives if necessary (for example, pH adjusters, surfactants, solubilizers, preservatives, emulsifiers, tonicity agents, and stabilizers), or may be preparation such as a tablet, capsule, suspension, injection, or suppository. For example, in the case of a tablet, the alkalinizing agent may be mixed with excipients (for example, lactose, D-mannitol, crystalline cellulose, and glucose), disintegrants (for example, starch and carboxymethylcellulose calcium (CMC-Ca)), binders (for example, hydroxypropylcellulose (HPC), gelatin, and polyvinylpyrrolidone (PVP)), lubricants (for example, magnesium stearate and talc), and the like to be formulated.

The tablet according to the present invention will be described in more detail below.

In one embodiment, the pharmaceutical composition provided by the present invention is a tablet. The tablet provided by the present invention may comprise pharmaceutically acceptable additives customary in the pharmaceutical field, in addition to the alkalinizing agent. Examples of such additives include excipients, binders, disintegrants, fluidizers, flavoring agents, lubricants, pH adjusters, surfactants, stabilizers, and fragrances.

The content of the alkalinizing agent in the tablet provided by the present invention may be 10% to 95% by weight, preferably 30% to 90% by weight, and more preferably 60% to 85% by weight with respect of the tablet.

Examples of excipients that can be used in the tablets provided by the present invention include lactose (for example, lactose hydrate, and anhydrous lactose), sugars such as glucose, sucrose, fructose, and maltose, sugar alcohols such as erythritol, sorbitol, maltitol, xylitol, and D-mannitol, starch (for example, corn starch, potato starch, rice starch, and wheat starch), crystalline cellulose, magnesium aluminate metasilicate, anhydrous calcium phosphate, precipitated calcium carbonate, calcium silicate, calcium lactate, and ethylcellulose, among which crystalline cellulose is particularly preferable.

The content of the excipient in the tablet provided by the present invention may be 1% to 95% by weight, preferably 1% to 80% by weight, more preferably 3% to 80% by weight, and even more preferably 3% to 20% by weight with respect to the tablet.

Examples of binders that can be used in the tablets provided by the present invention include hydroxypropylcellulose, hydroxypropyl methylcellulose, polyvinylpyrrolidone, dextrin, methylcellulose, polyvinyl alcohol, sodium alginate, aminoalkyl methacrylate copolymer, polyethylene glycol, pregelatinized starch (for example, partially pregelatinized starch), agar, and gelatin, among which hydroxypropylcellulose is particularly preferable.

The content of the binder in the tablet provided by the present invention may be 0.1% to 30% by weight, preferably 0.1% to 10% by weight, and more preferably 0.3% to 3% by weight with respect to the tablet.

Examples of disintegrants that may be used in tablets provided by the present invention include croscarmellose sodium, carmellose calcium, carboxymethyl starch sodium, low substituted hydroxypropylcellulose, crospovidone, starch (for example, wheat starch, corn starch, and partially pregelatinized starch), and carmellose, among which partially pregelatinized starch is particularly preferable.

The content of the disintegrant in the tablet provided by the present invention may be 0.3% to 20% by weight, preferably 1% to 10% by weight, and more preferably 3% to 10% by weight with respect to the tablet.

Examples of fluidizers that can be used in the tablets provided by the present invention include light anhydrous silicic acid, talc, and magnesium aluminate metasilicate.

The content of the fluidizers in the tablet provided by the present invention may be 0.03% to 3% by weight, preferably 0.1% to 3% by weight, and more preferably 0.3% to 3% by weight with respect to the tablet.

Examples of flavoring agents that can be used in the tablets provided by the present invention include citric acid (for example, anhydrous citric acid), acidulants such as malic acid, acetic acid, tartaric acid, fumaric acid, and ascorbic acid (where, the said flavoring agent does not comprise the alkalinizing agent according to the present invention), saccharin sodium, dipotassium glycyrrhizinate, aspartame (registered trademark), and sweeteners such as stevia, thaumatin, and sucralose.

The content of the flavoring agent in the tablet provided by the present invention may be 0.03% to 3% by weight, preferably 0.1% to 3% by weight, and more preferably 0.3% to 3% by weight with respect to the tablet.

Examples of lubricants that can be used in the tablets provided by the present invention include magnesium stearate, calcium stearate, talc, light anhydrous silicic acid, sucrose fatty acid esters, carnauba wax, macrogol, and sodium stearyl fumarate, among which magnesium stearate is particularly preferable.

The lubricant content in the tablet provided by the present invention may be 0.1% to 30% by weight, preferably 0.3% to 10% by weight, and more preferably 1% to 3% by weight with respect to the tablet.

Examples of pH adjusters that can be used in the tablets provided by the present invention include citric acid, phosphates (for example, sodium dihydrogen phosphate and potassium dihydrogen phosphate), carbonates (for example, magnesium carbonate and sodium carbonate), tartrate, fumarate, acetate, and amino acid salts (where, the pH adjuster does not include the alkalinizing agent according to the present invention).

The content of the pH adjuster in the tablet provided by the present invention may be 0.1% to 30% by weight, preferably 0.3% to 10% by weight, and more preferably 1% to 5% by weight with respect to the tablet.

Examples of surfactants that can be used in the tablets provided by the present invention include sodium lauryl sulfate, polysorbate, sucrose fatty acid ester, polyoxyethylene hydrogenated castor oil, polyoxyl stearate, macrogol, and poloxamer. The content of the surfactant in the tablet provided by the present invention may be 0.01% to 3% by weight, preferably 0.03% to 1% by weight, and more preferably 0.03% to 0.5% by weight with respect to the tablet.

Examples of stabilizers that can be used in tablets provided by the present invention include citric acid (for example, anhydrous citric acid), malic acid, acetic acid, tartaric acid, maleic acid, ascorbic acid, edetate sodium, and tocopherol (where, the stabilizer does not include the alkalinizing agent according to the present invention), among which anhydrous citric acid is particularly preferable.

The content of the stabilizer in the tablet provided by the present invention may be 0.01% to 30% by weight, preferably 0.1% to 30% by weight, and more preferably 1% to 20% by weight with respect to the tablet.

Examples of fragrances that can be used in the tablets provided by the present invention include citrus flavors such as lemon, orange, and grapefruit, peppermint, spearmint, and menthol. An appropriate amount thereof can be contained in the tablet (for example, 0.01% to 1% by weight, and more preferably 0.01% to 0.1% by weight with respect to the tablet).

The total content of the alkalinizing agent and the pharmaceutically acceptable additives in the tablet provided by the present invention does not exceed 100% by weight with respect to the tablet.

The tablet provided by the present invention can be an uncoated tablet comprising the above components and not having a coating layer, or a film-coated tablet having a coating layer. The content of the coating layer can be appropriately set by those skilled in the art. For example, it may be 0.1% to 10% by weight with respect to the uncoated tablet. In the coating layer, in addition to the coating base, a plasticizer, a coloring agent, a brightening agent, and the like can be appropriately incorporated.

Examples of coating bases that can be used for tablets provided by the present invention include hydroxypropylcellulose, hydroxypropyl methylcellulose, ethylcellulose, cellulose acetate phthalate, methacrylic acid copolymer, and polyvinylpyrrolidone, among which hydroxypropyl methylcellulose is particularly preferable. The content of the coating base in the tablet provided by the present invention may be 0.01% to 10% by weight, and preferably 0.3% to 3% by weight with respect to the tablet.

Examples of coating plasticizers that can be used in tablets provided by the present invention include triethyl citrate, medium chain fatty acid triglyceride, triacetin, glycerin, and propylene glycol and polyethylene glycol (for example, Macrogol 6000), among which Macrogol 6000 is particularly preferable. The content of the coating plasticizer in the tablet provided by the present invention may be 0.01% to 1% by weight, and preferably 0.03% to 3% by weight with respect to the tablet.

Examples of coating colorants that can be used in the tablets provided by the present invention include titanium oxide, yellow iron sesquioxide, iron sesquioxide, black iron oxide, food blue No. 2, and food blue No. 2 aluminum lake. The content of the coating colorant in the tablet provided by the present invention may be 0.01% to 1% by weight, and preferably 0.03% to 3% by weight with respect to the tablet.

Examples of coating brighteners that can be used in tablets provided by the present invention include carnauba wax. The content of the coating brightener in the tablet provided by the present invention may be 0.0001% to 0.1% by weight, and preferably 0.001% to 0.01% by weight with respect to the tablet.

The pharmaceutical composition provided by the present invention can be produced by a method known in the pharmaceutical field. For example, in the case of tablets, the production method may comprise a mixing step of mixing an additive with the alkalinizing agent, a granulation step, a tableting step, and/or a coating step.

The mixing step may comprise a step of mixing an alkalinizing agent and an additive such as an excipient, a stabilizer, a disintegrant and/or a binder. In addition, before the tableting step, a step in which a mixture comprising the alkalinizing agent and an additive is mixed with a lubricant, flavoring agent, and/or fragrance may further be provided. Mixing can be performed using a V-type mixer, a W-type mixer, a container mixer, a tumbler mixer, a stirring mixer, or the like.

The granulation step can be performed by a known granulation method in the pharmaceutical field. Examples of granulation methods include a dry granulation method, a wet granulation method, and a fluidized bed granulation method.

As one embodiment, the mixture obtained in the mixing step and the granulated product obtained in the granulation step are appropriately pulverized and/or sieved to obtain a mixture or granulated product having a desired particle size. The pulverization can be performed by a pulverizer known in the pharmaceutical field, such as a ball mill, a jet mill, or a hammer mill. The sieving can be performed using a 16 mesh sieve (aperture of 1000 µm) to 32 mesh sieve (aperture of 500 µm), and the like.

The tableting step can be performed by a tableting method known in the pharmaceutical field. Examples of tableting methods include a direct tableting method, a dry tableting method, a wet tableting method, and an external lubricant tableting method. For example, the mixture or granulated product obtained in the above steps can be tableted using a tableting machine known in the pharmaceutical field, such as a single-shot tableting machine or a rotary tableting machine. When using a single tableting machine, a rotary tableting machine, and the like, a tableting pressure of 1 kN to 30 kN can be employed.

The coating step can be performed by a method known in the pharmaceutical field. For example, the coating can be performed by spray coating the outside of the uncoated tablet with a coating liquid appropriately comprising a coating base, and a plasticizer, colorant, brightener, or the like.

In one embodiment, the tablet provided by the present invention can be produced by mixing an alkalinizing agent with excipients (for example, lactose, D-mannitol, crystalline cellulose, and/or glucose), binders (for example, hydroxypropylcellulose (HPC), gelatin, and/or polyvinylpyrrolidone (PVP)), stabilizers (for example, anhydrous citric acid), disintegrants (for example, starch (for example, partially pregelatinized starch) and/or carboxymethylcellulose calcium (CMC-Ca)), and lubricants (for example, magnesium stearate) to obtain uncoated tablets by tableting; and forming, on the outside of the uncoated tablet, a coating layer comprising a coating base (for example, hydroxypropylcellulose, hydroxypropyl methylcellulose, and/or PVP), plasticizers (for example, triethyl citrate and/or Macrogol 6000), colorants (for example, iron sesquioxide and/or titanium oxide), and brighteners (for example, carnauba wax).

In one embodiment, the tablets obtained can have a hardness of 10 to 200 N, preferably 30 to 150 N.

The amount of the alkalinizing agent in the pharmaceutical composition provided by the present invention can be appropriately set within the limits defined by independent claims 1 and 2.

In one embodiment, the amount of the alkalinizing agent in the pharmaceutical composition provided by the present invention may be set to an amount that enables amelioration in acid urine in gout or hyperuricemia by administering the alkalinizing agent to a human, or an amount smaller that the above amount. For example, an amount may be set to be 1% to 50% or 10% to 20% of the daily dose approved in Japan for amelioration in acid urine in gout or hyperuricemia (for example, in a case where the alkalinizing agent is a citric acid preparation: a tablet in which one tablet comprises 231.5 mg of potassium citrate (C₆H₅K₃O₇·H₂O) and 195.0 mg of sodium citrate hydrate (C₆H₅Na₃O₇·2H₂O) is orally administered 3 times a day, 2 tablets at one time).

In one embodiment, the pharmaceutical composition provided by the present invention is a tablet, and it may comprise, in one tablet, 10 mg to 300 mg for each, a total of 20 mg to 600 mg, preferably, 150 to 250 mg for each, a total of 400 to 500 mg, and more preferably 190 to 240 mg for each, a total of 400 to 450 mg of potassium citrate monohydrate and sodium citrate dihydrate.

In one embodiment, the pharmaceutical composition provided by the present invention is a tablet, and it comprises, as an alkalinizing agent, potassium citrate monohydrate 231.5 mg and sodium citrate dihydrate 195.0 mg; and may comprise, as an additive, anhydrous citric acid, crystalline cellulose, partially pregelatinized starch, hydroxypropylcellulose, magnesium stearate, hypromellose, macrogol 6000, titanium oxide, and carnauba wax.

As one embodiment, a tablet comprising potassium citrate monohydrate 231.5 mg and sodium citrate dihydrate 195.0 mg may be a single dosage unit.

In the present specification, "dosage unit" represents a unit of the preparation, and "one dosage unit" represents the minimum unit of the preparation. Accordingly, for example, in the case of tablets, the dosage unit is each tablet, and one dosage unit represents one tablet. In the case of an injection, the dosage unit is an injection contained in a sealed container such as an ampoule or vial, and one dosage unit represents an injection contained in a sealed container such as one ampoule or vial.

In a case where the pharmaceutical composition provided by the present invention is administered to a human or other mammals, one or more of the above dosage units may be administered at one time, or the one dosage unit may be divided and administered.

The dose of the alkalinizing agent is appropriately determined according to the type of the alkalinizing agent, the method of administration, the age, weight, sex, symptom, sensitivity to the drug, and the like of a subject of administration, but it may be adjusted depending on the condition of symptom amelioration, provided that 0.1 to 5 g/day of each of sodium citrate or the hydrate thereof, and potassium citrate or the hydrate thereof are orally administered at a total of 0.2 to 10 g/day, 1 to 5 times a day.

In one embodiment, in a case of an oral administration of a mixture of potassium citrate monohydrate and sodium citrate dihydrate as an alkalinizing agent to a human, half of the daily dose approved in Japan for amelioration in acid urine in gout or hyperuricemia (for example, in a case where the alkalinizing agent is a citric acid preparation: a tablet in which one tablet comprises 231.5 mg of potassium citrate (C₆H₅K₃O₇·H₂O) and 195.0 mg of sodium citrate hydrate (C₆H₅Na₃O₇·2H₂O) is orally administered 3 times a day, 2 tablets at one time) may be set to a daily dose.

In one embodiment, in a case of an oral administration of a mixture of potassium citrate monohydrate and sodium citrate dihydrate as an alkalinizing agent to a human, the daily dose approved in Japan for amelioration in acid urine in gout or hyperuricemia (for example, in a case where the alkalinizing agent is a citric acid preparation: a tablet in which one tablet comprises 231.5 mg of potassium citrate (C₆H₅K₃O₇·H₂O) and 195.0 mg of sodium citrate hydrate (C₆H₅Na₃O₇·2H₂O) is orally administered 3 times a day, 2 tablets at one time) may be set to a daily dose.

In one embodiment, in a case of an oral administration of a mixture of potassium citrate monohydrate and sodium citrate dihydrate as an alkalinizing agent to a human, half of the daily dose approved in Japan for amelioration in acid urine in gout or hyperuricemia (for example, in a case where the alkalinizing agent is a citric acid preparation: a tablet in which one tablet comprises 231.5 mg of potassium citrate (C₆H₅K₃O₇·H₂O) and 195.0 mg of sodium citrate hydrate (C₆H₅Na₃O₇·2H₂O) is orally administered 3 times a day, 2 tablets at one time) may be set to a daily dose to start administration, and thereafter, the dosage may be increased to a daily dosage approved in Japan for the amelioration of acidic urine in gout and hyperuricemia.

In one embodiment, the dosage amount of the alkalinizing agent may be a dosage amount such that, when the alkalinizing agent is orally administered, the pH of human urine (for example, early morning urine) becomes pH 5.2 to pH 6.8, pH 5.5 to pH 6.8, pH 5.8 to pH 6.8, pH 5.8 to pH 6.5, pH 5.8 to pH 6.2, pH 5.8 or more and less than pH 6.2, pH 6.0 to pH 6.5, pH 6.0 to pH 6.4, pH 6.0 to pH 6.3, pH 6.0 to pH 6.2, pH 6.0 or more and less than pH 6.2, pH 6.1 to pH 6.3, pH 6.2 to 6.8, pH 6.2 to pH 6.5, or pH 6.5 to 6.8.

In one embodiment, the dosage amount of the alkalinizing agent may be a dosage amount such that, when the alkalinizing agent is orally administered, the pH of human urine (for example, early morning urine) becomes pH 5.2 to pH 6.8, pH 5.5 to pH 6.8, pH 5.8 to pH 6.8, pH 5.8 to pH 6.5, pH 5.8 to pH 6.2, pH 5.8 or more and less than pH 6.2, pH 6.0 to pH 6.5, pH 6.0 to pH 6.4, pH 6.0 to pH 6.3, pH 6.0 to pH 6.2, pH 6.0 or more and less than pH 6.2, pH 6.1 to pH 6.3, pH 6.2 to 6.8, pH 6.2 to pH 6.5, or pH 6.5 to 6.8, 6 weeks, 12 weeks, or 24 weeks after the oral administration of the alkalinizing agent.

In one embodiment, when a mixture of potassium citrate monohydrate and sodium citrate dihydrate as an alkalinizing agent is orally administered to a human, potassium citrate monohydrate and sodium citrate dihydrate may be administered at 0.1 to 3g/day for each, a total of 0.2 to 6g/day, 0.5 to 3g/day for each, a total of 1 to 6g/day, preferably, 0.5 to 1.5 g/day for each, a total of 1 to 3 g/day, 1 to 1.5g/day for each, a total of 2 to 3g/day, or 0.5 to 1 g/day for each, a total of 1 to 2 g/day; or may be divided and administered 1 to 5 times a day, preferably 3 times a day.

In one embodiment, the alkalinizing agent may be administered for a long period of time, for example, for 1 week, 2 weeks, 3 weeks, 6 weeks, 8 weeks, 10 weeks, 12 weeks, 24 weeks, 40 weeks, 60 weeks, 80 weeks, 100 weeks, 120 weeks, 1 week or more, 2 weeks or more, 3 weeks or more, 6 weeks or more, 8 weeks or more, 10 weeks or more, 12 weeks or more, 24 weeks or more, 40 weeks or more, 60 weeks or more, 80 weeks or more, 100 weeks or more, 120 weeks or more, 6 to 24 weeks, 12 to 24 weeks, 6 to 30 weeks, 12 to 30 weeks, 6 to 40 weeks, 12 to 40 weeks, 6 to 60 weeks, 12 to 60 weeks, 6 to 80 weeks, 12 to 80 weeks, 6 to 100 weeks, 12 to 100 weeks, 6 to 120 weeks, or 12 to 120 weeks.

In one embodiment, by continuous administration for 6 weeks, continuous administration for 12 weeks, and/or continuous administration for 24 weeks of the pharmaceutical composition provided by the present invention, beneficial effects for a patient with kidney disease (for example, chronic kidney disease) (for example, an effect of decreasing a concentration of uremic substance (for example, indoxyl sulfate, p-cresyl sulfate, phenylacetyl-L-glutamine, hippuric acid, and/or argininosuccinic acid) in blood, an effect of increasing a concentration of uremic substance (for example, indoxyl sulfate, p-cresyl sulfate, phenylacetyl-L-glutamine, hippuric acid, and/or argininosuccinic acid) in urine (an effect of promoting excretion into urine), and/or an effect of increasing a urinary β2-microglobulin concentration) can be detected.

The pharmaceutical composition provided by the present invention is administered to a patient with stage G2 to stage G3b chronic kidney disease. Kidney disease includes acute kidney failure and chronic kidney disease unless otherwise specified.

Examples of acute kidney failure include acute kidney failure due to drugs (for example, platinum preparations such as non-steroidal anti-inflammatory drugs, angiotensin converting enzyme inhibitors, angiotensin II receptor antagonists, aminoglycoside antibiotics, new quinolone antibacterials, iodinated contrast agents, platinum preparations such as cisplatin, and the like), and acute kidney failure caused by kidney ischemia.

Chronic kidney disease (CKD) is a concept encompassing kidney disease that chronically progresses regardless of the underlying disease, and is a concept encompassing all clinical conditions in which kidney function expressed by glomerular filtration rate (GFR) deteriorates, or findings that suggest damage in kidneys is maintained chronically (3 months or longer).

According to the CKD Medical Guide 2012 (Nichijinkaishi 2012), the severity of chronic kidney disease is evaluated by classification according to cause (Cause: C), kidney function (GFR: G), and proteinuria (albuminuria: A).

The classification of GFR is as follows.
G1: GFR is normal or high (≥ 90 mL/min/1.73 m²)
G2: Normal or slight decrease in GFR (60 to 89 mL/min/1.73 m²)
G3a: Mild to moderate decrease in GFR (45 to 59 mL/min/1.73 m²)
G3b: Moderate to high decrease in GFR (30 to 44 mL/min/1.73 m²)
G4: GFR is highly reduced (15 to 29 mL/min/1.73 m²)
G5: End-stage kidney disease (ESKD) (< 15 mL/min/1.73 m²)

The classification by proteinuria (albuminuria: A) is classified as follows using the urine albumin/creatinine (Cr) ratio when the primary disease is diabetes.
A1: Normal (less than 30 mg/gCr)
A2: Microalbuminuria (30 to 299 mg/gCr)
A3: Overt albuminuria (300 mg/gCr or more)

In addition, in a case where the primary disease is hypertension other than diabetes, nephritis, polycystic kidneys, transplanted kidneys, and the like, proteinuria (albuminuria: A) is classified using the urine protein/creatinine (Cr) ratio as follows.
A1: Normal (less than 0.15 g/gCr)
A2: Mild proteinuria (0.15 to 0.49 g/gCr)
A3: High proteinuria (0.50 g/gCr or more)

According to CKD medical care guide 2012 (Nichijinkaishi 2012), the severity classification of chronic kidney disease (CKD) is expressed as, for example, diabetes G2A3, chronic nephritis G3bA1, and the like using the above C, G, and A.

However, it is considered that the severity of chronic kidney disease has conventionally been described only in the stages classified by GFR, and as in the related art, the severity of chronic kidney disease can be expressed in stages of G1, G2, G3a, G3b, G4, and G5.

The pharmaceutical composition provided by the present invention is administered to a patient with chronic kidney disease in stage G2 or more and stage G3b or less (for example, stage G2 and stage G3a; or stage G2, stage G3a and stage G3b).

In one embodiment, the pharmaceutical composition provided by the present invention is administered to a patient with chronic kidney disease in stage G3b or less and microalbuminuria, preferably administered to a patient with kidney disease in stage G2 and chronic albuminuria.

In one embodiment, the pharmaceutical composition provided by the present invention is administered to a patient with chronic kidney disease in stage G2 or more and stage G3b or less (for example, stage G2 and stage G3a; or stage G2, stage G3a and stage G3b) and which is microalbuminuria.

In one embodiment, the pharmaceutical composition provided by the present invention is administered to a patient with chronic kidney disease in stage G2 to stage G3b or lower and whose urinary protein excretion is less than 3.5 g/gCr, and is preferably administered to a patient with chronic kidney disease in stage G2 and whose urinary protein excretion is less than 3.5 g/gCr.

In one embodiment, the pharmaceutical composition provided by the present invention is administered to a patient with chronic kidney disease in stage G2 or more and stage G3b or less (for example, stage G2 and stage G3a; or stage G2, stage G3a and stage G3b) and whose urinary protein excretion is less than 3.5 g/gCr.

In one embodiment, the pharmaceutical composition provided by the present invention is administered to a patient with stage G2 to stage G3b progressive chronic kidney disease.

In one embodiment, the pharmaceutical composition provided by the present invention is administered to a patient with stage G2 to stage G3b chronic kidney disease whose concentration of β2-microglobulin in urine (for example, early morning urine) is 2000 µg/L or less, 1000 µg/L or less, 800 µg/L or less, 290 µg/L or less, 200 µg/L or less, 1 to 2000µg/L, 1 to 1000µg/L, 1 to 800µg/L, 1 to 290µg/L, 1 to 200µg/L, 10 to 2000 µg/L, 10 to 1000 µg/L, 10 to 800 µg/L, 10 to 290 µg/L, 10 to 200 µg/L, or 80 to 200 µg/L.

In one embodiment, the pharmaceutical composition provided by the present invention is administered to a patient with stage G2 to stage G3b chronic kidney disease whose cystatin C in blood (for example, in plasma or in serum) is 0.1 to 3.0 mg/L, 0.1 to 2.0 mg/L, 0.1 to 1.6 mg/L, 0.1 to 1.3 mg/L, 0.5 to 3.0 mg/L, 0.5 to 2.0 mg/L, 0.5 to 1.6 mg/L, 0.5 to 1.3 mg/L, or 0.9 to 1.3 mg/mL.

In one embodiment, the pharmaceutical composition provided by the present invention is administered to a patient with stage G2 to stage G3b chronic kidney disease whose indoxyl sulfate concentration in blood is 0.001 to 100 µg/mL (for example, 0.1 to 30 µg/mL).

In one embodiment, the pharmaceutical composition provided by the present invention is administered to a patient with stage G2 to stage G3b chronic kidney disease whose concentration of p-cresyl sulfate in blood is 0.003 to 300 µg/mL (for example, 0.01 to 30 µg/mL).

In one embodiment, the pharmaceutical composition provided by the present invention is administered to a patient with stage G2 to stage G3b chronic kidney disease whose hippuric acid concentration in blood is 0.01 to 100 µg/mL (for example, 0.01 to 10 µg/mL).

In one embodiment, the pharmaceutical composition provided by the present invention is administered to a patient with stage G2 to stage G3b chronic kidney disease whose argininosuccinic acid concentration in blood is 0.01 to 100 µg/mL (for example, 0.1 to 10 µg/mL).

In one embodiment, the pharmaceutical composition provided by the present invention is administered to a patient with stage G2 to stage G3b chronic kidney disease whose concentration of phenylacetyl-L-glutamine in blood is 0.03 to 30 µg/mL (for example, 0.1 to 10 µg/mL).

In one embodiment, the pharmaceutical composition provided by the present invention is administered to a patient with stage G2 to stage G3b chronic kidney disease receiving treatment according to a CKD medical guide. For example, it is administered to a patient subjected to, according to CKD medical guide, blood pressure management (administration of RA inhibitors such as ARB and ACE inhibitors, diuretics, Ca antagonists), anti-proteinuria measures (administration of RA inhibitors, and the like), blood glucose level control (administration of α-glucosidase inhibitor, and the like), lipid management (administration of statins, fibrates, and the like), anemia management (for example, erythropoietin administration), and/or bone/mineral measures (bisphosphonate administration and the like).

In one embodiment, the pharmaceutical composition provided by the present invention is used in combination with an antihypertensive agent (for example, ARB, ACE inhibitor, diuretic, Ca antagonist).

In one embodiment, the pharmaceutical composition provided by the present invention is used in combination with a spherical carbonaceous adsorbent (sold in Japan as KREMEZIN (registered trademark)) obtained by oxidation and reduction treatment of spherical fine porous carbon derived from petroleum hydrocarbons at high temperature.

In one embodiment, the pharmaceutical composition provided by the present invention is administered to a patient with low-severity, stage G2 to stage G3b chronic kidney disease (for example, a patient with chronic kidney disease in stage G2 and stage G3a, and even more preferably stage G2), and thereby a concentration of uremic substance (for example, indoxyl sulfate, hippuric acid, and/or phenylacetyl-L-glutamine) in blood of a patient is decreased, and excretion of uremic substance (for example, indoxyl sulfate, p-cresyl sulfate, hippuric acid, argininosuccinic acid, and/or phenylacetyl-L-glutamine) outside the body (for example, into urine) is promoted. In this embodiment, the pharmaceutical composition provided by the present invention may be a pharmaceutical composition for ameliorating uremic symptoms, a pharmaceutical composition for treating or preventing uremia, a pharmaceutical composition for suppressing progression of chronic kidney disease, a pharmaceutical composition for delaying introduction of dialysis, a pharmaceutical composition for suppressing myocardial fibrosis, a pharmaceutical composition for suppressing arteriosclerosis, a pharmaceutical composition for ameliorating arteriosclerosis, a pharmaceutical composition for suppressing vascular smooth muscle cell proliferation, a pharmaceutical composition for suppressing vascular endothelial cell injury, a pharmaceutical composition for suppressing arterial wall thickening, a pharmaceutical composition for ameliorating arterial wall thickening, a pharmaceutical composition for suppressing calcification of the aorta, or a pharmaceutical composition for treating or preventing cardiovascular disease, which is a complication (for example, heart failure, myocardial infarction, stroke, and the like).

In one embodiment, the pharmaceutical composition provided by the present invention is administered to a patient with low-severity, stage G2 to stage G3b chronic kidney disease (for example, a patient with chronic kidney disease in stage G2 and stage G3a, and even more preferably stage G2), and thereby an increase in urinary β2-microglobulin in the patient is suppressed. In this embodiment, the pharmaceutical composition provided by the present invention may be a composition for suppressing damage of kidney tubule (for example, kidney proximal tubule), a composition for suppressing damage of kidney tubular cells (for example, kidney proximal tubular cells), a composition for protecting kidney tubular cells (for example, kidney proximal tubular cells), or a pharmaceutical composition for maintaining kidney tubular cell function (for example, reabsorption of glucose, amino acids, and the like) (for example, kidney proximal tubular cell function).

In one embodiment, the pharmaceutical composition provided by the present invention is administered to a patient with chronic kidney disease in stage G3b, and thereby a concentration of uremic substance (for example, indoxyl sulfate, hippuric acid, and/or phenylacetyl-L-glutamine) in blood of the patient is decreased, and excretion of uremic substance (for example, indoxyl sulfate, p-cresyl sulfate, hippuric acid, argininosuccinic acid, and/or phenylacetyl-L-glutamine) outside the body (for example, into urine) is promoted. In this embodiment, the pharmaceutical composition provided by the present invention may be a pharmaceutical composition for ameliorating uremic symptoms, a pharmaceutical composition for treating or preventing uremia, a pharmaceutical composition for suppressing progression of chronic kidney disease, a pharmaceutical composition for delaying introduction of dialysis, a pharmaceutical composition for suppressing myocardial fibrosis, a pharmaceutical composition for suppressing arteriosclerosis, a pharmaceutical composition for ameliorating arteriosclerosis, a pharmaceutical composition for suppressing vascular smooth muscle cell proliferation, a pharmaceutical composition for suppressing vascular endothelial cell injury, a pharmaceutical composition for suppressing arterial wall thickening, a pharmaceutical composition for ameliorating arterial wall thickening, a pharmaceutical composition for suppressing calcification of the aorta, a pharmaceutical composition for suppressing a decrease in energy production in muscle cells, a pharmaceutical composition for suppressing a decrease in muscle mass and/or muscle strength, or a pharmaceutical composition for treating or preventing cardiovascular disease, which is a complication (for example, heart failure, myocardial infarction, stroke, and the like).

In one embodiment, the pharmaceutical composition provided by the present invention is administered to a patient with chronic kidney disease in stage G3b, and thereby an increase in urinary β2-microglobulin in the patient is suppressed. In this embodiment, the pharmaceutical composition provided by the present invention may be a composition for suppressing damage of kidney tubule (for example, kidney proximal tubule), a composition for suppressing damage of kidney tubular cells (for example, kidney proximal tubular cells), a composition for protecting kidney tubular cells (for example, kidney proximal tubular cells), or a pharmaceutical composition for maintaining kidney tubular cell function (for example, reabsorption of glucose, amino acids, and the like) (for example, kidney proximal tubular cell function).

### 2. Food composition

A food composition described herein, but not claimed as such, comprises an alkalinizing agent and exhibits an effect of promoting excretion, outside the body, of uremic substance (for example, indoxyl sulfate, p-cresyl sulfate, phenylacetyl-L-glutamine, hippuric acid, and/or argininosuccinic acid, preferably indoxyl sulfate, p-cresyl sulfate, and phenylacetyl-L-glutamine, more preferably indoxyl sulfate and phenylacetyl-L-glutamine, and even more preferably indoxyl sulfate).

In one embodiment, the food composition comprises an alkalinizing agent and exhibits an effect of decreasing the concentration of uremic substance in blood.

In one embodiment, the food composition comprises an alkalinizing agent and exhibits an effect of promoting excretion of uremic substance into urine.

In one embodiment, the food composition comprises an alkalinizing agent and exhibits a kidney function maintenance effect.

In one embodiment, the food composition comprises an alkalinizing agent and exhibits an effect of suppressing kidney tubular damage. Examples of kidney tubules include a kidney proximal tubule.

In one embodiment, the food composition comprises an alkalinizing agent and exhibits an effect of suppressing kidney proximal tubular cell damage.

In one embodiment, the food composition comprises an alkalinizing agent and exhibits an effect of protecting kidney proximal tubular cells.

In one embodiment, the food composition comprises an alkalinizing agent, and exhibits an effect of maintaining kidney tubular function (for example, reabsorption of water, sodium ion, potassium ion, calcium ion, phosphate ion, bicarbonate ion, chloride ion, glucose, amino acid, vitamin, and the like). Examples of kidney tubules include a kidney proximal tubule, and examples of kidney proximal tubular function include reabsorption of glucose, amino acids, vitamins, and the like.

In the above-described embodiment, the food composition exhibits an effect of suppressing an increase in the amount (concentration) of β2-microglobulin in urine (for example, early morning urine) associated with progression of a stage of chronic kidney disease.

In the above-described embodiment, the food composition does not affect the glomerular function of a patient with chronic kidney disease, whereas it exhibits an effect of suppressing kidney proximal tubular cell damage associated with progression of a stage of chronic kidney disease to protect kidney proximal tubular cells.

For the alkalinizing agent, the alkalinizing agent described in "1. Pharmaceutical composition" above can be applied. Examples of alkalinizing agents include a pharmaceutically acceptable salt of citric acid as acceptable salts of citric acid as food (for example, an alkali metal salt of citric acid or a hydrate thereof, or a mixture thereof), and sodium bicarbonate. A mixture of a potassium citrate monohydrate (C₆H₅K₃O₇·H₂O) and a sodium citrate dihydrate (C₆H₅Na₃O₇·2H₂O), or sodium citrate dihydrate is preferable.

Uremic substance is also as described in "1. Pharmaceutical composition" above. Examples of uremic substances include indoxyl sulfate, p-cresyl sulfate, phenylacetyl-L-glutamine, hippuric acid, and argininosuccinic acid.

The content of the alkalinizing agent in the food composition can be appropriately determined depending on the type of food. Examples of food compositions include foods for specified health use, dietary supplements, functional foods, hospital patient foods, and supplements. The form of these food compositions is not particularly limited as long as it comprises an effective amount of an alkalinizing agent for exerting the above-mentioned effects and can be taken orally. The form may be a form of a normal food or drink, or the food composition may be provided as a preparation suitable for oral administration, for example, a preparation such as a tablet, a capsule, or a suspension, among preparations that can be applied to the pharmaceutical composition. Regarding the constitution and production method of these preparations, in the present specification, the constitution and production method of the pharmaceutical preparation described in the above "1. Pharmaceutical composition" can be applied as it is, and known formulation techniques in the field of pharmaceutical preparation technology itself can be also be applied.

For example, in the case of foods for specified health use, dietary supplements, functional foods, or foods for hospital patients, per serving of food, an amount of 1/3 of a total of 1 to 3 g of a potassium citrate monohydrate and a sodium citrate dihydrate may be contained as an alkalinizing agent, or an amount of 1/3 of 1 to 6 g of a sodium hydrogen carbonate may be contained as an alkalinizing agent. When specified health foods, dietary supplements, functional foods, hospital patient foods, or supplements are provided as tablets, for example, per tablet, 70% to 80% by weight of the alkalinizing agent may be contained in 300 mg to 600 mg of a tablet.

When the food composition is not formulated and is provided in the form of a normal food or drink, it can be appropriately produced by those skilled in the art depending on the type of the food. For example, it can be produced by blending an alkalinizing agent (for example, potassium citrate and/or sodium citrate) with the food material.

The form of the food and drink is a liquid or milky or pasty food such as a beverage, soy sauce, milk, yogurt, or miso; a semi-solid food such as jelly or gummy; solid foods such as rice cakes, gums, tofu, and supplements; powdered foods; and the like.

Examples of beverages include fruit juice, fruit drinks, coffee drinks, oolong tea drinks, green tea drinks, tea drinks, barley tea drinks, vegetable drinks, carbonated soft drinks, fruit extract drinks, vegetable extract juices, near water, sports drinks, diet drinks, and the like.

In beverages, additives such as antioxidants, fragrances, various esters, organic acids, organic acid salts, inorganic acids, inorganic acid salts, inorganic salts, pigments, emulsifiers, preservatives, seasonings, sweeteners, acidulants, fruit juice extracts, vegetable extracts, nectar extract, pH adjusters, and quality stabilizers can be blended in individually or in combination.

The food composition can be used in the same manner as the method of using the pharmaceutical composition described in the above "1. Pharmaceutical composition," and can also be used within a range not intended to treat or prevent diseases. That is, when the alkalinizing agent contained in the food composition is used as a reference, it can be applied to an application target of the pharmaceutical composition such that the amount of the alkalinizing agent used in the food composition becomes the same as the alkalinizing agent contained in the pharmaceutical composition. In addition, in one embodiment, the "food composition" can be applied to a subject (for example, a human or other mammals) who does not have "morbid" or "abnormal" symptoms, conditions, or diseases, that is, a subject (for example, a human or other mammals) in a "healthy" or "normal" state in order to maintain or enhance a "healthy" or "normal" state. Furthermore, it can be applied to a "healthy subject concerned about kidney health" or "healthy subject concerned about kidney tubule health" in order to maintain or improve a "healthy" or "normal" state. In this case, even if the alkalinizing agent is a component of the pharmaceutical composition or a component of the food composition, the pharmacological effect of the alkalinizing agent itself is basically the same. Accordingly, the application amount and application method of the food composition can be appropriately adjusted based on the alkalinizing agent according to an expected effect.

A food composition applied to a subject (for example, a human or other mammals) who does not have "morbid" or "abnormal" symptoms, conditions, or diseases, that is, a subject (for example, a human or other mammals) in a "healthy" or "normal" state in order to maintain or enhance a "healthy" or "normal" state, may be particularly referred to as "functional food" in some cases.

The term "administration" described in the above "1. Pharmaceutical composition" can also be applied to the "food composition". Furthermore, regarding the "food composition", the term "administration" can be read as "ingestion." Accordingly, for example, the terms "administer," "administered," and the like can be read as "is ingested," "ingest," "ingesting," and the like, with different word forms depending on the context.

On packaging, a container, or an instruction leaflet for the food composition, effects of decreasing a concentration of uremic substance in blood; effects of promotion of excretion of uremic substance into urine; effects of maintenance of kidney function; effects of suppression of kidney tubular damage; effects of suppression of kidney tubular cell damage; effects of suppression of kidney proximal tubular cell damage; effects of protection of kidney tubular cells; effects of protection of kidney proximal tubular cells; effects of maintenance of kidney tubular function (for example, reabsorption of water, sodium ion, potassium ion, calcium ion, phosphate ion, bicarbonate ion, chloride ion, glucose, amino acid, vitamin, and the like), preferably maintenance of kidney proximal tubular function (for example, reabsorption of glucose, amino acids, vitamins, and the like); and the like; are preferably indicated.

In one embodiment, the "food composition" is ingested by a subject (for example, a human or other mammals) in which a urinary β2-microglobulin concentration is 290 µg/L or less, preferably 50 to 150 µg/L.

In one embodiment, the "food composition" is ingested by a subject (for example, a human or other mammals) in which a blood cystatin C concentration is 0.5 to 2.2 mg/L, preferably 1.0 to 1.3 mg/L.

In one embodiment, the ingestion of the "food composition" suppresses an increase in urinary β2-microglobulin concentration.

In one embodiment, the ingestion of the "food composition" suppresses an increase in urinary β2-microglobulin concentration 12 weeks after the administration.

In one embodiment, the ingestion of the "food composition" does not substantially decrease a urinary β2-microglobulin concentration compared to before the start of administration.

In one embodiment, the ingestion of the "food composition" does not substantially decrease a urinary β2-microglobulin concentration 12 weeks after administration, compared to before the start of administration.

In one embodiment, the ingestion of the "food composition" does not substantially increase blood cystatin C compared to before the start of administration.

In one embodiment, the ingestion of the "food composition" does not substantially increase blood cystatin C compared to before the start of administration.

In one embodiment, the ingestion of the "food composition" suppresses, in early morning urine, an increase of a β2-microglobulin amount which is associated with progression of a stage of chronic kidney disease.

In one embodiment, the ingestion of the "food composition" does not affect the glomerular function of a patient with chronic kidney disease, whereas it suppresses kidney proximal tubular cell damage associated with progression of a stage of chronic kidney disease progression to protect kidney proximal tubular cells.

### 3. Method for determining decrease in concentration of uremic substance in blood

A method for determining a decrease in concentration of uremic substance (for example, indoxyl sulfate, p-cresyl sulfate, phenylacetyl-L-glutamine, hippuric acid, and/or argininosuccinic acid, preferably indoxyl sulfate, hippuric acid, and phenylacetyl-L-glutamine, more preferably indoxyl sulfate and phenylacetyl-L-glutamine, and even more preferably indoxyl sulfate) in blood of a patient with chronic kidney disease, which is described herein but not claimed as such, comprises measuring a pH of urine.

A method for determining a promotion of excretion of uremic substance (indoxyl sulfate, p-cresyl sulfate, phenylacetyl-L-glutamine, hippuric acid, and/or argininosuccinic acid, preferably indoxyl sulfate, p-cresyl sulfate, hippuric acid, and phenylacetyl-L-glutamine, more preferably indoxyl sulfate and phenylacetyl-L-glutamine, and even more preferably indoxyl sulfate) into urine of a patient with chronic kidney disease, which is described herein but not claimed as such, comprises measuring a pH of urine.

Measurement of the content of uremic substance (for example, indoxyl sulfate) in body fluid can be performed by a HPLC method or enzyme method. However, these measurement methods require specialized and expensive reagents.

As described in the present specification, a concentration of uremic substance in blood (for example, indoxyl sulfate, p-cresyl sulfate, phenylacetyl-L-glutamine, hippuric acid, and/or argininosuccinic acid) is decreased by administering the alkalinizing agent, and thereby excretion of these uremic substance into the urine is promoted. Accordingly, by measuring a pH of urine of a patient with chronic kidney disease, very simply and inexpensively, it is possible to determine a decrease in concentration of uremic substance in blood (for example, indoxyl sulfate, p-cresyl sulfate, phenylacetyl-L-glutamine, hippuric acid, and/or argininosuccinic acid), and/or a promotion of excretion of uremic substance into urine (for example, indoxyl sulfate, p-cresyl sulfate, phenylacetyl-L-glutamine, hippuric acid, and/or argininosuccinic acid). For the measurement of pH, a well-known technique may be used. For example, a pH test paper, a pH test solution, or a simple pH measuring device can be used.

For examples, a patient with chronic kidney disease measures a pH of early morning urine (wake up first urine) over time from the start of taking an alkalinizing agent (for example, a mixture of a potassium citrate monohydrate and a sodium citrate dihydrate, or sodium citrate dihydrate), and if the urine pH is high, it can be easily determined that a decrease in concentration of uremic substance in blood (for example, indoxyl sulfate, p-cresyl sulfate, phenylacetyl-L-glutamine, hippuric acid, and/or argininosuccinic acid, preferably indoxyl sulfate, hippuric acid, and/or phenylacetyl-L-glutamine, more preferably indoxyl sulfate and phenylacetyl-L-glutamine, and even more preferably indoxyl sulfate), and/or a promotion of excretion of uremic substance into urine (for example, indoxyl sulfate, p-cresyl sulfate, phenylacetyl-L-glutamine, hippuric acid, and/or argininosuccinic acid, preferably indoxyl sulfate, p-cresyl sulfate, hippuric acid, and/or phenylacetyl-L-glutamine, more preferably indoxyl sulfate and phenylacetyl-L-glutamine, and even more preferably indoxyl sulfate) is achieved.

For example, a patient with chronic kidney disease measures a pH of early morning urine (wake up first urine) over time after taking an alkalinizing agent (for example, a mixture of a potassium citrate monohydrate and a sodium citrate dihydrate, or sodium citrate dihydrate). If the urine pH is within a range of 5.2 to 6.8 (for example, the pH of urine is within a range of pH 5.5 to pH 6.8, pH 5.8 to pH 6.8, pH 5.8 to pH 6.5, pH 5.8 to pH 6.2, pH 5.8 or more and less than pH 6.2, pH 6. 0 to pH 6.5, pH 6.0 to pH 6.4, pH 6.0 to pH 6.3, pH 6.0 to pH 6.2, pH 6.0 or more and less than pH 6.2, pH 6.1 to pH 6.3, pH 6.2 to 6.8, pH 6.2 to pH 6.5, or pH 6.5 to 6.8), it can be easily determined that a decrease in concentration of uremic substance in blood (for example, indoxyl sulfate, p-cresyl sulfate, phenylacetyl-L-glutamine, hippuric acid, and/or argininosuccinic acid, preferably indoxyl sulfate, hippuric acid, and/or phenylacetyl-L-glutamine, more preferably indoxyl sulfate and phenylacetyl-L-glutamine, and even more preferably indoxyl sulfate), and/or a promotion of excretion of uremic substance into urine (for example, indoxyl sulfate, p-cresyl sulfate, phenylacetyl-L-glutamine, hippuric acid, and/or argininosuccinic acid, preferably indoxyl sulfate, p-cresyl sulfate, hippuric acid, and/or phenylacetyl-L-glutamine, more preferably indoxyl sulfate and phenylacetyl-L-glutamine, and even more preferably indoxyl sulfate) is achieved.

The determination, which is thus obtained as above, as to whether or not a decrease in concentration of uremic substance in blood (for example, indoxyl sulfate, p-cresyl sulfate, phenylacetyl-L-glutamine, hippuric acid, and/or argininosuccinic acid), and/or a promotion of excretion of uremic substance into urine (for example, indoxyl sulfate, p-cresyl sulfate, phenylacetyl-L-glutamine, hippuric acid, and/or argininosuccinic acid) is achieved can aid the diagnosis of whether or not progression of chronic kidney disease is suppressed.

Accordingly, a method for determining suppression of progression of chronic kidney disease, comprises measuring the pH of urine (for example, early morning urine) of a patient to which an alkalinizing agent (for example, a mixture of a potassium citrate monohydrate and a sodium citrate dihydrate, or sodium citrate dihydrate) has been administered. If it is recognized that the urine pH increases over time or the urine pH is within the range of 5.8 to 6.8 (for example, the urine pH is within the range of 6.0 to 6.2), this can then aid the diagnosis that progression of a stage of chronic kidney disease progression are suppressed.

In one embodiment, the pharmaceutical composition provided by the present invention is a pharmaceutical composition comprising an alkalinizing agent for use in suppressing progression of chronic kidney disease, in which 0.5 to 1.5 g/day of each of a potassium citrate monohydrate and a sodium citrate dihydrate as the alkalinizing agent are orally administered at a total of 1 to 3 g/day, 1 to 5 times a day, preferably 3 times a day.

In one embodiment, the pharmaceutical composition provided by the present invention is a pharmaceutical composition comprising an alkalinizing agent for use in suppressing progression of chronic kidney disease, in which one dosage unit (preferably one tablet) contains 231.5 mg potassium citrate monohydrate and 195.0 mg sodium citrate dihydrate, and 3 to 6 dosage units per day are orally administered in three divided doses per day.

In one embodiment, the pharmaceutical composition provided by the present invention is a pharmaceutical composition comprising an alkalinizing agent for use in treating or preventing kidney tubular damage, in which 0.5 to 1.5 g/day of each of a potassium citrate monohydrate and a sodium citrate dihydrate as the alkalinizing agent are orally administered at a total of 1 to 3 g/day, 1 to 5 times a day, preferably 3 times a day.

In one embodiment, the pharmaceutical composition provided by the present invention is a pharmaceutical composition comprising an alkalinizing agent for use in treating or preventing kidney tubular damage, in which one dosage unit (preferably one tablet) contains 231.5 mg potassium citrate monohydrate and 195.0 mg sodium citrate dihydrate, and 3 to 6 dosage units per day are orally administered in three divided doses per day.

Hereinafter, the present invention will be further described by examples, but the present invention is not limited thereto.

### EXAMPLES

A human clinical trial was conducted to examine whether oral administration of a combination preparation of hydrates of potassium citrate and sodium citrate, and a sodium bicarbonate preparation, which are oral alkalinizing agents, promotes excretion of uremic substance into urine.

### 1. Method

47 patients with chronic kidney diseases in stage G2 to G3b (eGFR: 30 to 89 ml/min/1.73m²) were randomly divided into a group to which a combination preparation of hydrates of potassium citrate and sodium citrate had been administered (a group A: 16 patients), a group to which a sodium bicarbonate preparation (sodium hydrogen carbonate) had been administered (a group B: 16 patients), and a control group (a group C: 15 patients). Patients were assigned to each group so that age, sex, presence of diabetes, and eGFR were not biased. Each group was treated according to the "CKD medical care guide-summary of treatment" (hereinafter referred to as standard treatment).

Only the treatment of patients in group A is according to the invention. The treatment of patients in groups B and C is for comparison and/or reference only.

An alkalinizing agent was not administered to the control group. To the group A, 3 tablets a day in which each tablet contained potassium citrate (C₆H₅K₃O₇·H₂O) 231.5 mg and a sodium citrate hydrate (C₆H₅Na₃O₇·2H₂O) 195.0 mg were orally administered 3 times a day (morning, noon, evening) for 24 weeks. A pH of early morning urine was controlled over time so that the dosage could be increased up to 6 tablets, 3 times a day (morning, noon, evening) as needed according to the discretion of the physician in cases where a pH of early morning urine was less than pH 6.5. To the group B, 3 tablets a day in which each tablet contained 500 mg of sodium bicarbonate were orally administered 3 times a day (morning, noon, evening) for 24 weeks. A pH of early morning urine was controlled over time so that the dosage could be increased up to 6 tablets, 3 times a day (morning, noon, evening) as needed according to the discretion of the physician in cases where a pH of early morning urine was less than pH 6.5.

Early morning urine and blood were collected before the start of administration, and 6 weeks, 12 weeks, and 24 weeks after the start of administration, and each specimen was stored at -80°C. Methods used in the present field for indoxyl sulfate, p-cresyl sulfate, phenylacetyl-L-glutamine, hippuric acid, and argininosuccinic acid in urine and blood (Sato, E., et. al., Metabolic alteration by indoxyl sulfate in skeletal muscle induce uremic sarcopenia in chronic kidney disease., Sci Rep. 2016 Nov 10;6:36618. doi: 10.1038/srep36618. and the like) were referred to, and quantitative analysis was performed using the following liquid chromatography triple quadrupole mass spectrometer (LC-MS/MS).

For LC, NANOSPACE SI-2 (manufactured by Shiseido) was used, and CAPCELLPAK MGIII was selected as the analytical column.

For MS, TSQ Quantiva (manufactured by Thermo Fisher Scientific Co., Ltd.) was used, and five compounds were ionized in the negative mode and detected using the Selected Reaction Monitoring method. The quantitative value was calculated using a calibration curve prepared with a standard solution of each compound.

In addition, the amount of urinary β2-microglobulin was measured by latex agglutination method using the LZ test "Eiken" β2-M and LZ-β2-M standard U "Eiken" (Eiken Chemical Co., Tokyo, Japan). Furthermore, the amount of cystatin C in the serum was measured by a colloidal gold agglutination method using Nescoat GC Cystatin C (Nm) (Alfresa Pharma, Osaka, Japan).

For statistical analysis, the Mann-Whitney test was used for comparison between groups, and the Wilcoxon test was used for comparison of changes over time. The Pearson test was used for correlation.

### 2. Result

Based on the measurement results using LC-MS/MS, for respective patients in the group A (the group to which the combination preparation of hydrates of potassium citrate and sodium citrate was administered), the group B (the group to which the sodium bicarbonate preparation was administered), and the group C (the control group), the following was calculated:
(i) Concentration of each uremic substance in plasma before administration
(ii) Concentration of each uremic substance in early morning urine before administration
(iii) Ratio of uremia substance concentration in early morning urine and plasma uremic substance concentration before the start of administration (amount of uremic substance in urine/amount of uremic substance in plasma)
(iv) Concentration of each uremic substance in plasma 6 weeks, 12 weeks, and 24 weeks after the start of administration
(v) Concentration of each uremic substance in early morning urine 6 weeks, 12 weeks, and 24 weeks after the start of administration
(vi) Ratio of uremic substance concentration in early morning urine and uremic substance concentration in plasma at 6 weeks, 12 weeks, and 24 weeks after the start of administration (uremic substance amount in urine/uremic substance amount in plasma)
(vii) Amount of change in the concentration of each uremic substance in the plasma 6 weeks, 12 weeks, and 24 weeks after the start of administration from before the start of administration
(viii) Amount of change in the concentration of each uremic substance in the early morning urine 6 weeks, 12 weeks, and 24 weeks, from before the start of administration
(ix) Amount of change in ratio of uremic substance concentration in early morning urine and uremic substance concentration in plasma 6 weeks, 12 weeks, and 24 weeks after the start of administration (amount of uremic substance in urine/amount of uremic substance in plasma), from before the start of administration

Then, the average value and SD of each group were calculated for (i) to (ix) above. For each of (iv) to (ix) above, the average value and SD of each group were calculated for all data of 6 weeks, 12 weeks, and 24 weeks after the start of administration in each group.

The results are shown in tables below. In the tables and drawings, the group A: the group to which the combination preparation of hydrates of potassium citrate and sodium citrate had been administered was described as "Citrate," and the group B: the group to which the sodium bicarbonate preparation had been administered was described as "Bicarbonate." In addition, the numerical value in the parenthesis in the tables indicates the number of cases.
Table 1-1-1: Amount (ng/mL) of indoxyl sulfate in plasma
Table 1-1-2: Change in amount (ng/mL) of indoxyl sulfate in plasma from before the start of administration
Table 1-2-1: Amount (ng/mL) of indoxyl sulfate in early morning urine
Table 1-2-2: Change in amount (ng/mL) of indoxyl sulfate in early morning urine from before the start of administration
Table 1-3-1: Ratio of amount of indoxyl sulfate in urine to an amount of indoxyl sulfate in plasma
Table 1-3-2: Amount of change in ratio of amount of indoxyl sulfate in urine to amount of indoxyl sulfate in plasma from before the start of administration
Table 2-1-1: Amount (ng/mL) of p-cresyl sulfate in plasma
Table 2-1-2: Change in amount (ng/mL) of p-cresyl sulfate in plasma from before the start of administration
Table 2-2-1: Amount (ng/mL) of p-cresyl sulfate in early morning urine
Table 2-2-2: Change in amount (ng/mL) of p-cresyl sulfate in early morning urine from before the start of administration
Table 2-3-1: Ratio of amount of p-cresyl sulfate in urine to an amount of p-cresyl sulfate in plasma
Table 2-3-2: Amount of change in ratio of amount of p-cresyl sulfate in urine to amount of p-cresyl sulfate in plasma from before the start of administration
Table 3-1-1: Amount (ng/mL) of hippuric acid in plasma
Table 3-1-2: Change in amount (ng/mL) of hippuric acid in plasma from before the start of administration
Table 3-2-1: Amount (ng/mL) of hippuric acid in early morning urine
Table 3-2-2: Change in amount (ng/mL) of hippuric acid in early morning urine from before the start of administration
Table 3-3-1: Ratio of amount of hippuric acid in urine to amount of hippuric acid in plasma
Table 3-3-2: Amount of change in ratio of amount of hippuric acid in urine to amount of hippuric acid in plasma from before the start of administration
Table 4-1-1: Amount (ng/mL) of argininosuccinic acid in plasma
Table 4-1-2: Change in amount (ng/mL) of argininosuccinic acid in plasma from before the start of administration
Table 4-2-1: Amount (ng/mL) of argininosuccinic acid in early morning urine
Table 4-2-2: Change in amount (ng/mL) of argininosuccinic acid in early morning urine from before the start of administration
Table 4-3-1: Ratio of amount of argininosuccinic acid in urine to amount of argininosuccinic acid in plasma
Table 4-3-2: Amount of change in ratio of amount of argininosuccinic acid in urine to amount of argininosuccinic acid in plasma from before the start of administration
Table 5-1-1: Amount (ng/mL) of phenylacetyl-L-glutamine (PAG) in plasma
Table 5-1-2: Change in amount (ng/mL) of phenylacetyl-L-glutamine (PAG) in plasma from before the start of administration
Table 5-2-1: Amount (ng/mL) of phenylacetyl-L-glutamine (PAG) in early morning urine
Table 5-2-2: Change in amount (ng/mL) of phenylacetyl-L-glutamine (PAG) in early morning urine from before the start of administration
Table 5-3-1: Ratio of amount of phenylacetyl-L-glutamine (PAG) in urine to amount of phenylacetyl-L-glutamine (PAG) in plasma
Table 5-3-2: Amount of change in ratio of amount of phenylacetyl-L-glutamine (PAG) in urine to amount of phenylacetyl-L-glutamine (PAG) in plasma from before the start of administration

In the group A (Citrate: the group to which the combination preparation of hydrates of potassium citrate and sodium citrate had been administered), an indoxyl sulfate concentration in plasma 6, 12, and 24 weeks after the administration was a lower value, compared to those of the group B (Bicarbonate: the group to which the sodium bicarbonate preparation had been administered) and the group C (Control: the control group) (refer to Table 1-1-1). In addition, in the group A, an indoxyl sulfate (IS) concentration in early morning urine 12 and 24 weeks after administration was a higher value than that in the group C (refer to Table 1-2-1). The indoxyl sulfate concentration in plasma at 6 to 24 weeks was a significantly lower value in the group A than those in the groups B and C (refer to Table 1-1-1), and the indoxyl sulfate concentration in early morning urine at 6 to 24 weeks was significantly greater in the group A than those in the groups B and C (refer to Table 1-2-2).

In addition, by administering the combination preparation of hydrates of potassium citrate and sodium citrate to patients with chronic kidney disease, the concentration of indoxyl sulfate, which is a uremic substance, in urine increased compared to before administration, and the concentration of indoxyl sulfate in blood decreased compared to before administration. Even with the same alkalinizing agent, such an effect was not recognized in the case of the sodium bicarbonate preparation. Compared with the sodium bicarbonate preparation, the combination preparation of hydrates of potassium citrate and sodium citrate exhibited an excellent effect of decreasing an indoxyl sulfate concentration in blood, and an excellent effect of increasing an indoxyl sulfate concentration in urine. The effect of decreasing the indoxyl sulfate concentration in blood and the effect of increasing the indoxyl sulfate concentration in urine by the combination preparation of hydrates of potassium citrate and sodium citrate were recognized from 12 weeks after the administration.

Based on the value of the ratio of indoxyl sulfate concentration in urine to indoxyl sulfate concentration in plasma, it was shown that administration of the combination preparation of hydrates of potassium citrate and sodium citrate led to the excretion of indoxyl sulfate from blood into urine, and thereby excretion thereof outside the body was promoted. The effect of excretion of indoxyl sulfate from blood into urine was recognized by administration of the combination preparation of hydrates of potassium citrate and sodium citrate, but not by administration of the sodium bicarbonate preparation (refer to Table 1-3-1 and Table 1-3-2).

**[Table 1-1-1]**

| **Plasma Indoxyl Sulfate** | | | | | | |
|---|---|---|---|---|---|---|
| **Group** | **N** | **0W** | **6W** | **12W** | **24W** | **6-24W (45-47)** |
| **Control** | **15** | **1189 ± 634** | **1229 ± 710** | **1326 ± 793** | **1134 ± 371** | **1230 ± 641** |
| **Citrate** | **16** | **1130 ± 1157** | **1170 ± 1056** | **1105 ± 1213^{b}** | **1050 ± 762** | **1108 ± 770 ^{a,c}** |
| **Bicarbonate** | **16** | **1338 ± 770** | **1365 ± 821** | **1432 ± 1031** | **1481 ± 988** | **1426 ± 930** |

| | | | | | | |
|---|---|---|---|---|---|---|
| **Mean ± SD** **^{a}p=0.0288, ^{b}p=0.0451 va Control and ^{c}p=0.0350 vs Bicarbonate (Mann-Whitney)** | | | | | | |

**[Table 1-1-2]**

| **Plasma Indoxyl Sulfate** (Conversion to 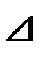 actual measurement value from week 0) | | | | | |
|---|---|---|---|---|---|
| **Group** | **N** | **6W** | **12W** | **24W** | **6-24W (45-47)** |
| **Control** | **15** | **39.5 ± 446.4** | **136.5 ± 468.1** | **-55.8 ± 564.2** | **40.1 ± 490.6** |
| **Citrate** | **16** | **40.0 ± 460.7** | **-36.8 ± 380.8** | **-80.3 ± 583.7** | **-25.5 ± 487.6** |
| **Bicarbonate** | **16** | **27.5 ± 523.0** | **153.1 ± 887.4** | **143.4 ± 682.5** | **107.0 ± 695.8** |

| | | | | | |
|---|---|---|---|---|---|
| **Mean ± SD** | | | | | |

**[Table 1-2-1]**

| **Urine Indoxyl Sulfate** | | | | | | |
|---|---|---|---|---|---|---|
| **Group** | **N** | **0W** | **6W** | **12W** | **24W** | **6-24W (45-48)** |
| **Control** | **15** | **41026 ± 26159** | **34606 ± 23011** | **30959 ± 15288** | **29139 ± 16714^{e}** | **31568 ± 18358** |
| **Citrate** | **16** | **41081 ± 45508^{a}** | **32728 ± 19842** | **58648 ± 96652** | **46808 ± 43377** | **46061 ± 64823** |
| **Bicarbonate** | **16** | **54379 ± 40259** | **52510 ± 39229** | **37860 ± 26401** | **56492 ± 39593** | **48954 ± 35769^{d}** |

| | | | | | | |
|---|---|---|---|---|---|---|
| **Mean ± SD** **^{d}p=0.0073 vs Control and ^{a}p=0.0385 vs Bicarbonate (Mann-Whitney)** **^{e}p=0.0103 vs 0 week (Wilcoxon)** | | | | | | |

**[Table 1-2-2]**

| **Urine Indoxyl Sulfate** (Conversion to 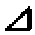 actual measurement value from week 0) | | | | | |
|---|---|---|---|---|---|
| **Group** | **N** | **6W** | **12W** | **24W** | **6-24W (45-48)** |
| **Control** | **15** | **-6419 ± 26152** | **-10067 ± 26839** | **-11887 ± 21994** | **-9458 ± 24617** |
| **Citrate** | **16** | **-8352 ± 41966** | **17566 ± 64586^{c}** | **5727 ± 36794** | **4980 ± 49397 ^{a,d}** |
| **Bicarbonate** | **16** | **-1869 ± 19390** | **-16519 ± 40380** | **2113 ± 49356** | **-5425 ± 38514** |

| | | | | | |
|---|---|---|---|---|---|
| **Mean ± SD** **^{a}p=0.0141 vs Control and ^{c}p=0.0233, ^{d}p=0.0438 vs Bicarbonate (Mann-Whitney)** | | | | | |

**[Table 1-3-1]**

| **Indoxyl Sulfate - Urine/Plasma ratio** | | | | | | |
|---|---|---|---|---|---|---|
| **Group** | **N** | **0W** | **6W** | **12W** | **24W** | **6-24W (45-47)** |
| **Control** | **15** | **39.2 ± 21.1** | **31.8 ± 21.2^{e}** | **27.7 ± 16.1^{f}** | **26.4 ± 13.8^{e}** | **28.6 ± 17.1** |
| **Citrate** | **16** | **37.5 ± 24.7** | **38.6 ± 31.8** | **51.5 ± 50.6** | **52.4 ± 64.4^{a}** | **47.4 ± 50.0** |
| **Bicarbonate** | **16** | **55.5 ± 43.6** | **51.7 ± 36.4** | **35.3 :t 26.4** | **50.7 ± 36.5^{c}** | **46.2 ± 33.7^{d}** |

| | | | | | | |
|---|---|---|---|---|---|---|
| **Mean ± SD** **^{a}p=0.0482, ^{c}p=0.0322 and ^{d}p=0.0141 vs Control (Mann-Whitney)** **^{e}p=0.0479, ^{f}p=0.0413 and ^{g}p=0.0151 vs 0 week (Wilcoxon)** | | | | | | |

**[Table 1-3-2]**

| **Indoxyl Sulfate - Urine/Plasma ratio** (Conversion to 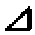 actual measurement value from week 0) | | | | | |
|---|---|---|---|---|---|
| **Group** | **N** | **6W** | **12W** | **24W** | **6-24W (45-47)** |
| **Control** | **15** | **-7.41 ± 24.69** | **-11.52 ± 9.87** | **-12.79 ± 16.00** | **-10.57 ± 20.16** |
| **Citrate** | **16** | **1.10 ± 22.99** | **10.70 ± 39.08^{d}** | **14.91 ± 56.11^{b}** | **8.90 ± 40.99 ^{c,e}** |
| **Bicarbonate** | **16** | **-3.81 ± 37.48** | **-22.41 ± 35.49** | **-4.78 ± 35.33** | **-10.33 ± 36.35** |

| | | | | | |
|---|---|---|---|---|---|
| **Mean ± SD** **^{b}p=0.0239, ^{c}p=0.0006 vs Control and ^{d}p=0.0457, ^{e}p=0.0292 vs Bicarbonate (Mann-Whitney)** | | | | | |

Regarding p-cresyl sulfate (PCS), in the group A (Citrate: the group to which the combination preparation of hydrates of potassium citrate and sodium citrate had been administered), the concentration of p-cresyl sulfate in the early morning urine 12 and 24 weeks after administration was a higher value than that of the group C (Control: the control group). In the group A (the group to which the combination preparation of hydrates of potassium citrate and sodium citrate had been administered), even when compared to the group B (Bicarbonate: the group to which the sodium bicarbonate preparation had been administered), the p-cresyl sulfate concentration in the early morning urine at 6, 12, and 24 weeks after administration was a higher value (refer to Table 2-2-1). An increase in p-cresyl sulfate concentration in early morning urine at 6 to 24 weeks was recognized only in the group A (refer to Table 2-2-2).

In addition, administration of the combination preparation of hydrates of potassium citrate and sodium citrate to patients with chronic kidney diseases increased the concentration of p-cresyl sulfate, which is a uremic substance, in urine compared to before administration (refer to Table 2-2-1 and Table 2-2-2). Even with the same alkalinizing agent, such an effect was not recognized in the case of the sodium bicarbonate preparation, and the combination preparation of hydrates of potassium citrate and sodium citrate exhibited a stronger effect of increasing a concentration of p-cresyl sulfate in urine as compared to the sodium bicarbonate preparation. The effect of increasing the p-cresyl sulfate concentration in urine by the combination preparation of hydrates of potassium citrate and sodium citrate was recognized from 12 weeks after administration.

On the other hand, administration of the sodium bicarbonate preparation decreased the concentration of p-cresyl sulfate, which is a uremic substance, in plasma as compared to before administration (refer to Table 2-1-2). Such an effect of decreasing a concentration of p-cresyl sulfate in plasma was more strongly recognized in the group B as compared to the group A and group C (refer to Table 2-1-2).

Based on the value of the ratio of p-cresyl sulfate concentration in urine to p-cresyl sulfate concentration in plasma, it was shown that administration of the combination preparation of hydrates of potassium citrate and sodium citrate led to the excretion of p-cresyl sulfate from blood into urine, and thereby excretion thereof outside the body was promoted. It was shown that the effect of excretion of p-cresyl sulfate from blood into urine was more strongly recognized by administration of the combination preparation of hydrates of potassium citrate and sodium citrate than by administration of the sodium bicarbonate preparation (refer to Table 2-3-1 and Table 2-3-2).

**[Table 2-1-1]**

| **Plasma *p*-Cresyl Sulfate** | | | | | | |
|---|---|---|---|---|---|---|
| **Group** | **N** | **0W** | **6W** | **12W** | **24W** | **6-24W (44-48)** |
| **Control** | **15** | **4467 ± 3531** | **5289 ± 4389** | **4482 ± 4570** | **3852 ± 3454** | **4524 ± 4106** |
| **Citrate** | **16** | **3491 ± 5159** | **3414 ± 3432** | **3306 ± 4140** | **3673 :t 4853** | **3468 ± 4094** |
| **Bicarbonate** | **16** | **5402 ± 5209** | **4506 ± 5300** | **3771 ± 4139** | **4284 ± 3682** | **4187 ± 4354** |

| | | | | | | |
|---|---|---|---|---|---|---|
| **Mean ± SD** | | | | | | |

**[Table 2-1-2]**

| **Plasma *p*-Cresyl Sulfate** (Conversion to 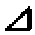 actual measurement value from week 0) | | | | | |
|---|---|---|---|---|---|
| **Group** | **N** | **6W** | **12W** | **24W** | **6-24W (44-47)** |
| **Control** | **15** | **536.1 ± 2801** | **14.3 ± 3291** | **-615.2 ± 2280** | **-34.3 ± 2796** |
| **Citrate** | **16** | **-77.2 ± 2341** | **-6.8 ± 2982** | **181.8 ± 2283** | **33.4 ± 2491 ^{c}** |
| **Bicarbonate** | **16** | **-895.9 ± 2153** | **-1103 ± 2478** | **-1119 ± 3631** | **-1038 ± 2773** |

| | | | | | |
|---|---|---|---|---|---|
| **Mean ± SD** **^{c}p=0.0377 vs Bicarbonate (Mann-Whitney)** | | | | | |

**[Table 2-2-1]**

| **Urine *p*-Cresyl Sulfate** | | | | | | |
|---|---|---|---|---|---|---|
| **Group** | **N** | **0W** | **6W** | **12W** | **24W** | **6-24W (44-48)** |
| **Control** | **15** | **58782 ± 59346** | **60188 ± 72317** | **39257 ± 57116** | **34669 ± 51832** | **44353 ± 60343** |
| **Citrate** | **16** | **39252 ± 53214** | **38343 ± 26834** | **42970 ± 43893** | **55979 ± 68542** | **45764 ± 49000** |
| **Bicarbonate** | **16** | **66164 ± 97704** | **67744 t 105548** | **37730 ± 48411** | **52029 ± 57779** | **52501 ± 73583** |

| | | | | | | |
|---|---|---|---|---|---|---|
| **Mean ± SD** | | | | | | |

**[Table 2-2-2]**

| **Urine *p*-Cresyl Sulfate** (Conversion to 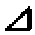 actual measurement value from week 0) | | | | | |
|---|---|---|---|---|---|
| **Group** | **N** | **6W** | **12W** | **24W** | **6-24W (44-48)** |
| **Control** | **15** | **-2403 ± 56332** | **-19525 ± 50834** | **-24113 ± 47778** | **-15641 ± 51296** |
| **Citrate** | **16** | **-909 ± 48618** | **3718 ± 38763** | **16727 ± 42639^{a}** | **6512 ± 43256^{b,c}** |
| **Bicarbonate** | **16** | **1580 ± 51468** | **-28434 ± 72253** | **-14163 ± 95244** | **-13663 ± 74566** |

| | | | | | |
|---|---|---|---|---|---|
| **Mean ± SD** **^{a}p=0.0170, *p=0.0109 va Control and** ^{c}**p=0.0242 vs Bicarbonate (Mann-Whitney)** | | | | | |

**[Table 2-3-1]**

| ***p*-Cresyl Sulfate - Urine/Plasma ratio** | | | | | | |
|---|---|---|---|---|---|---|
| **Group** | **N** | **0W** | **6W** | **12W** | **24W** | **6-24W (44-48)** |
| **Control** | **15** | **11.9 ± 9.4** | **10.9 ± 4.9** | **9.8 ± 5.9** | **9.3 ± 6.2** | **10.0 ± 5.6** |
| **Citrate** | **16** | **14.0 ± 9.2** | **16.1 ± 13.7** | **16.3 ± 12.9** | **20.5 ± 21.5 ^{a}** | **17.7 ± 16.3 ^{b}** |
| **Bicarbonate** | **16** | **15.6 ± 11.1** | **17.0 ± 12.0** | **16.5 ± 17.9** | **17.0 ± 11.8 ^{c}** | **16.8 ± 13.9 ^{d}** |

| | | | | | | |
|---|---|---|---|---|---|---|
| **Mean ± SD** **^{a}p=0.0221, ^{b}p=0.0094, ^{c}p=0.0364 and ^{d}p=0.0055 vs Control (Mann-Whitney)** | | | | | | |

**[Table 2-3-2]**

| ***p*-Cresyl Sulfate - Urine/Plasma ratio** (Conversion to 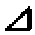 actual measurement value from week 0) | | | | | |
|---|---|---|---|---|---|
| **Group** | **N** | **6W** | **12W** | **24W** | **6-24W (44-48)** |
| **Control** | **15** | **-1.08 ± 10.03** | **-2.14 ± 9.15** | **-2.59 ± 9.68** | **-1.96 ± 9.41** |
| **Citrate** | **16** | **2.08 ± 11.30** | **1.22 ± 11.94** | **6.51 ± 17.93** | **3.21 ± 13.94** |
| **Bicarbonate** | **16** | **1.39 ± 8.25** | **0.91 ± 15.68** | **1.31 ± 9.81** | **1.20 ± 11.45** |

| | | | | | |
|---|---|---|---|---|---|
| **Mean ± SD** | | | | | |

Regarding hippuric acid (HA), in the group A (Citrate: the group to which the combination preparation of hydrates of potassium citrate and sodium citrate had been administered), a hippuric acid concentration in plasma 24 weeks after the administration was a lower value, compared to those of the group B (Bicarbonate: the group to which the sodium bicarbonate preparation had been administered) and the group C (Control: the control group) (refer to Table 3-1-1). Such an effect was not seen in the administration of the sodium bicarbonate preparation. In addition, in the group A, the hippuric acid concentration in the early morning urine 12 and 24 weeks after administration was a higher value than that in the group C (refer to Table 3-2-1).

In addition, by administering the combination preparation of hydrates of potassium citrate and sodium citrate to patients with chronic kidney diseases, the concentration of hippuric acid, which is a uremic substance, in plasma 24 weeks after administration was decreased as compared to that before administration (refer to Tables 3-1-1 and 3-2-2), and the concentration of hippuric acid in urine increased compared to that before administration (refer to Tables 3-2-1 and 3-2-2). An increase in hippuric acid concentration in the early morning urine at 6 to 24 weeks was recognized only in the group A (refer to Table 2-2-2). Even with the same alkalinizing agent, compared with the sodium bicarbonate preparation, the combination preparation of hydrates of potassium citrate and sodium citrate exhibited an excellent effect of increasing a hippuric acid concentration in urine. The effect of increasing the hippuric acid concentration in urine by the combination preparation of hydrates of potassium citrate and sodium citrate was recognized from 6 weeks after administration.

Based on the value of the ratio of hippuric acid concentration in urine to hippuric acid concentration in plasma, it was shown that administration of the combination preparation of hydrates of potassium citrate and sodium citrate led to the excretion of hippuric acid from blood into urine, and thereby excretion thereof outside the body was promoted. In addition, it was shown that the effect of excretion of hippuric acid from blood into urine at 6 to 24 weeks was more strongly recognized by administration of the combination preparation of hydrates of potassium citrate and sodium citrate than by administration of the sodium bicarbonate preparation (refer to Table 3-3-1 and Table 3-3-2).

**[Table 3-1-1]**

| **Plasma Hippuric Acid** | | | | | | |
|---|---|---|---|---|---|---|
| **Group** | **N** | **0W** | **6W** | **12W** | **24W** | **6-24W (45-47)** |
| **Control** | **15** | **1096 ± 828** | **752 ± 192^{b}** | **975 ± 615** | **818 ± 615** | **848 ± 572** |
| **Citrate** | **16** | **868 ± 1015** | **1464 ± 2213** | **985 ± 1376** | **520 ± 437** | **990 ± 1546** |
| **Bicarbonate** | **16** | **742 ± 706** | **609 ± 496** | **994 ± 806** | **1465 ± 1387^{c}** | **1091 ± 991** |

| | | | | | | |
|---|---|---|---|---|---|---|
| **Mean ± SD** **"p=0.0181 and ^{c}p=0.0215vs 0 week (Wilcoxon)** | | | | | | |

**[Table 3-1-2]**

| **Plasma Hippuric Acid** (Conversion to 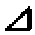 actual measurement value from week 0) | | | | | |
|---|---|---|---|---|---|
| **Group** | **N** | **6W** | **12W** | **24W** | **6-24W (45-47)** |
| **Control** | **15** | **-343.6 ± 620.1** | **-120.6 ± 700.9** | **-227.9 ± 893.8** | **-247.3 ± 736.1** |
| **Citrate** | **16** | **596.5 ± 2370^{a}** | **153.7 ± 1671** | **-347.1 ± 1080^{d}** | **133.7 ± 1794** |
| **Bicarbonate** | **16** | **66.6 ± 681.8** | **230.0 ± 655.6** | **722.3 ± 1023^{c}** | **342.1 ± 838.5^{e}** |

| | | | | | |
|---|---|---|---|---|---|
| **Mean ± SD** **^{a}p=0.0430, ^{c}p=0.0164, ^{e}p=0.0008 vs Control** **and ^{d}p=0.0093 vs Bicarbonate (Mann-Whitney)** | | | | | |

**[Table 3-2-1]**

| **Urine Hippuric Acid** | | | | | | |
|---|---|---|---|---|---|---|
| **Group** | **N** | **0W** | **6W** | **12W** | **24W** | **6-24W (45-48)** |
| **Control** | **15** | **128966 ± 110600** | **169650 ± 81018** | **66202 ± 43032** | **88804 ± 43032** | **94885 ± 64566** |
| **Citrate** | **16** | **94469 ± 57885** | **112946 ± 80846** | **112486 ± 86932** | **99287 ± 70822** | **108240 ± 78356** |
| **Bicarbonate** | **16** | **109249 ± 96133** | **98283 ± 80730** | **96346 ± 70315** | **114453 ± 81562** | **103027 ± 76475** |

| | | | | | | |
|---|---|---|---|---|---|---|
| **Mean ± SD** | | | | | | |

**[Table 3-2-2]**

| **Urine Hippuric Acid** (Conversion to 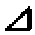 actual measurement value from week 0) | | | | | |
|---|---|---|---|---|---|
| **Group** | **N** | **6W** | **12W** | **24W** | **6-24W (44-48)** |
| **Control** | **15** | **-19316 ± 96398** | **-42764 ± 105143** | **-40162 ± 85472** | **-34080 ± 94400** |
| **Citrate** | **16** | **18478 ± 89920** | **18018 ± 62771** | **4818 ± 81234** | **13771 ± 77363 ^{a}** |
| **Bicarbonate** | **16** | **-10966 ± 75133** | **-12903 ± 61236** | **5204 ± 85546** | **-6221 ± 73493** |

| | | | | | |
|---|---|---|---|---|---|
| **Mean ± SD** **^{a}p=0.0431 vs Control (Mann-Whitney)** | | | | | |

**[Table 3-3-1]**

| **Hippuric Acid - Urine/Plasma ratio** | | | | | | |
|---|---|---|---|---|---|---|
| **Group** | **N** | **0W** | **6W** | **12W** | **24W** | **6-24W (41-47)** |
| **Control** | **15** | **139.0 ± 69.6** | **146.2 ± 70.2** | **119.3 ± 75.1** | **134.4 ± 100.5** | **133.3 ± 61.9** |
| **Citrate** | **16** | **316.8 ± 374.8** | **160.8 ± 179.9** | **275.1 ± 315.5** | **430.1 ± 619.6^{b,c}** | **288.9 ± 61.7^{e}** |
| **Bicarbonate** | **16** | **304.5 ± 361.8** | **148.7 ± 119.2^{f}** | **151.8 ± 115.8** | **523.0 ± 1438** | **265.8 ± 812.9** |

| | | | | | | |
|---|---|---|---|---|---|---|
| **Mean ± SD** **^{b}p=0.0239 vs Control and ^{c}p=0.0355, ^{e}p=0.0478 vs Bicarbonate** **(Mann-Whitney)** **^{f}p=0.0479 va 0 week (Wilcoxon)** | | | | | | |

**[Table 3-3-2]**

| **Hippuric Acid - Urine/Plasma ratio** (Conversion to 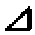 actual measurement value from week 0) | | | | | |
|---|---|---|---|---|---|
| **Group** | **N** | **6W** | **12W** | **24W** | **6-24W (44-48)** |
| **Control** | **15** | **72 ± 73.2** | **-19.1 ± 83.1** | **-4.6 ± 82.5** | **-5.7 ± 78.7** |
| **Citrate** | **16** | **-156.0 ± 362.1** | **-60.2 ± 475.5** | **113.3 ± 588.8** | **-33.7 ± 487.4** |
| **Bicarbonate** | **15** | **-155.8 ± 312.2^{a}** | **-161.2 ± 302.1** | **191.1 ± 1147** | **-50.2 ± 688.3^{b}** |

| | | | | | |
|---|---|---|---|---|---|
| **Mean ± SD** **^{a}p=0.0451 ,^{b}p=0.0170 vs Control (Mann-Whitney)** | | | | | |

Regarding argininosuccinic acid (ASA), in the group A (Citrate: the group to which the combination preparation of hydrates of potassium citrate and sodium citrate had been administered), a concentration of argininosuccinic acid in the early morning urine was a higher value than that of the group C (Control: the control group), whereas the value was lower than that of the group B (Bicarbonate: the group to which the sodium bicarbonate preparation had been administered) (refer to Table 4-2-1).

In addition, administration of the combination preparation of hydrates of potassium citrate and sodium citrate to patients with chronic kidney diseases increased the concentration of argininosuccinic acid, which is a uremic substance, in urine compared to before administration (refer to Table 4-2-1 and Table 4-2-2). The effect of increasing the argininosuccinic acid concentration in urine by the combination preparation of hydrates of potassium citrate and sodium citrate was recognized from 12 weeks after administration. The increase in argininosuccinic acid concentration in early morning urine at 6 to 24 weeks was more increased in the group B than in the group A (refer to Table 4-2-2).

Based on the value of the ratio of an argininosuccinic acid concentration in urine to an argininosuccinic acid concentration in plasma, it was shown that administration of the combination preparation of hydrates of potassium citrate and sodium citrate led to the excretion of argininosuccinic acid from blood into urine, and thereby excretion thereof outside the body was promoted. In addition, it was shown that the effect of excretion of argininosuccinic acid from blood into urine at 6 to 24 weeks was more strongly recognized by administration of the sodium bicarbonate preparation than that by administration of the combination preparation of hydrates of potassium citrate and sodium citrate (refer to Table 4-3-1 and Table 4-3-2).

**[Table 4-1-1]**

| **Plasma Arginino Succinic Acid** | | | | | | |
|---|---|---|---|---|---|---|
| **Group** | **N** | **0W** | **6W** | **12W** | **24W** | **6-24W (45-48)** |
| **Control** | **15** | **1973 ± 3343** | **1493 ± 1193** | **1464 ± 1211** | **1142 ± 692** | **1366 ± 1047** |
| **Citrate** | **16** | **1288 ± 789** | **1241 ± 847** | **1472 ± 915** | **1404 ± 887** | **1370 ± 869** |
| **Bicarbonate** | **16** | **1178 ± 655** | **1245 ± 748** | **1576 ± 2160** | **1160 ± 977** | **1327 ± 1416** |

| | | | | | | |
|---|---|---|---|---|---|---|
| **Mean ± SD** | | | | | | |

**[Table 4-1-2]**

| **Plasma Arginino Succinic Acid** (Conversion to 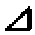 actual measurement value from week 0) | | | | | |
|---|---|---|---|---|---|
| **Group** | **N** | **6W** | **12W** | **24W** | **6-24W (45-48)** |
| **Control** | **15** | **-479.8 ± 3374** | **-508.4 ± 3352** | **-830.6 ± 3349** | **-606.3 ± 3285** |
| **Citrate** | **16** | **-47.0 ± 723.7** | **135.8 ± 710.2** | **116.4 ± 666.6** | **66.9 ± 690.0** |
| **Bicarbonate** | **16** | **66.5 ± 565.5** | **397.7 ± 1733** | **-17.6 ± 776.9** | **148.9 ± 1134** |

| | | | | | |
|---|---|---|---|---|---|
| **Mean ± SD** | | | | | |

**[Table 4-2-1]**

| **Urine Arginino Succinic Acid** | | | | | | |
|---|---|---|---|---|---|---|
| **Group** | **N** | **0W** | **6W** | **12W** | **24W** | **6-24W (45-48)** |
| **Control** | **15** | **2861 ± 1563** | **2726 ± 1757** | **2289 ± 1381** | **1977 ± 1076^{b}** | **2331 ± 1433** |
| **Citrate** | **16** | **2483 ± 1649** | **2399 ± 979** | **2625 ± 1485** | **2796 ± 1526** | **2607 ± 1333** |
| **Bicarbonate** | **16** | **2911 ± 1691** | **3346 ± 2636** | **2647 ± 2123** | **3221 ± 2466** | **3071 ± 2386** |

| | | | | | | |
|---|---|---|---|---|---|---|
| **Mean ± SD** **^{b}p=0.0215 vs 0 week (Wilcoxon)** | | | | | | |

**[Table 4-2-2]**

| **Urine Arginino Succinic Acid** (Conversion to 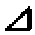 actual measurement value from week 0) | | | | | |
|---|---|---|---|---|---|
| **Group** | **N** | **6W** | **12W** | **24W** | **6-24W (44-48)** |
| **Control** | **15** | **-110.7 ± 1578** | **-571.8 ± 1159** | **-884.0 ± 1363** | **-531.5 ± 1377** |
| **Citrate** | **16** | **-84.7 ± 1617** | **142.0 ± 1785** | **312.1 ± 1591*** | **123.1 ± 1639^{b}** |
| **Bicarbonate** | **16** | **434.7 ± 1299** | **-264.1 ± 1491** | **309.9 ± 2335** | **160.2 ± 1756** |

| | | | | | |
|---|---|---|---|---|---|
| **Mean ± SD** ***p=0.0105 and ^{b}p=0.0175 vs Control (Mann-Whitney)** | | | | | |

**[Table 4-3-1]**

| **Arginino Succinic Acid - Urine/Plasma ratio** | | | | | | |
|---|---|---|---|---|---|---|
| **Group** | **N** | **0W** | **6W** | **12W** | **24W** | **6-24W (45-48)** |
| **Control** | **15** | **3.17 ± 2.57** | **3.67 ± 3.69** | **3.28 ± 3.47** | **2.16 ± 1.35** | **3.04 ± 3.02** |
| **Citrate** | **16** | **2.39 ± 1.60** | **2.92 ± 2.31** | **2.87 ± 3.27** | **3.24 ± 3.17** | **3.01 ± 2.88** |
| **Bicarbonate** | **16** | **3.82 ± 4.93** | **5.75 ± 9.80** | **2.81 ± 1.86** | **6.36 ± 9.60** | **4.97 ± 7.98** |

| | | | | | | |
|---|---|---|---|---|---|---|
| **Mean ± SD** | | | | | | |

**[Table 4-3-2]**

| **ASA** - **Urine/Plasma ratio** (Conversion to 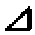 actual measurement value from week 0) | | | | | |
|---|---|---|---|---|---|
| **Group** | **N** | **6W** | **12W** | **24W** | **6-24W (44-48)** |
| **Control** | **15** | **0.49 ± 3.25** | **0.11 ± 3.74** | **-1.01 ± 2.43** | **-0.14 ± 3.18** |
| **Citrate** | **16** | **0.53 ± 2.61** | **0.49 ± 3.55** | **0.84 ± 2.90** | **0.62 ± 2.97** |
| **Bicarbonate** | **16** | **1.93 ± 7.90** | **-1.01 ± 4.47** | **2.54 ± 10.20^{a}** | **1.15 ± 7.87** |

| | | | | | |
|---|---|---|---|---|---|
| **Mean ± SD** ***p=0.0403 vs Control (Mann-Whitney)** | | | | | |

Regarding phenylacetyl-L-glutamine (PAG), in the group A (Citrate: the group to which the combination preparation of hydrates of potassium citrate and sodium citrate had been administered), a phenylacetyl-L-glutamine concentration in plasma was a lower value compared to that of the group C (Control: the control group) (refer to Table 5-1-1). In the group A, a phenylacetyl-L-glutamine concentration in early morning urine 12 and 24 weeks after administration was a higher value than that in the group C (refer to Table 5-2-1).

In addition, administration of the combination preparation of hydrates of potassium citrate and sodium citrate to patients with chronic kidney diseases increased the concentration of phenylacetyl-L-glutamine, which is a uremic substance, in urine 12 and 24 weeks after administration, compared to before administration (refer to Table 5-2-1 and Table 5-2-2). Even with the same alkalinizing agent, such an effect was not recognized in the case of the sodium bicarbonate preparation. The effect of increasing the phenylacetyl-L-glutamine concentration in urine by the combination preparation of hydrates of potassium citrate and sodium citrate was recognized from 12 weeks after administration. Administration of the combination preparation of hydrates of potassium citrate and sodium citrate to patients with chronic kidney diseases decreased the concentration of phenylacetyl-L-glutamine, which is a uremic substance, in plasma compared to before administration (refer to Table 5-1-1 and Table 5-2-2). The effect of decreasing a phenylacetyl-L-glutamine concentration in plasma was strongly recognized by the sodium bicarbonate preparation than that by the combination preparation of hydrates of potassium citrate and sodium citrate (refer to Table 5-1-2).

Based on the value of the ratio of phenylacetyl-L-glutamine concentration in urine to phenylacetyl-L-glutamine concentration in plasma, it was shown that administration of the combination preparation of hydrates of potassium citrate and sodium citrate led to the excretion of phenylacetyl-L-glutamine from blood into urine, and thereby excretion thereof outside the body was promoted. In addition, it was shown that the effect of excretion of phenylacetyl-L-glutamine from blood into urine was more strongly recognized by administration of the combination preparation of hydrates of potassium citrate and sodium citrate than that by administration of the sodium bicarbonate preparation (refer to Table 5-3-1 and Table 5-3-2).

**[Table 5-1-1]**

| **Plasma Phenyl Acetyl L-Glutamine (PAG)** | | | | | | |
|---|---|---|---|---|---|---|
| **Group** | **N** | **0W** | **6W** | **12W** | **24W** | **6-24W (45-48)** |
| **Control** | **15** | **1121 ± 534** | **1144 ± 644** | **1138 ± 623** | **1088 ± 485** | **1123 ± 575** |
| **Citrate** | **16** | **919 ± 962^{a}** | **818 ± 523** | **934 ± 843** | **907 ± 772** | **885 ± 709^{c}** |
| **Bicarbonate** | **16** | **1324 ± 949** | **1240 ± 1062** | **1153 ± 1000** | **1284 ± 945** | **1226 ± 983** |

| | | | | | | |
|---|---|---|---|---|---|---|
| **Mean** ± **SD** **^{a}p=0.0326 and ^{c}p=0.0065 vs Control (Mann-Whitney)** | | | | | | |

**[Table 5-1-2]**

| **Plasma PAG** (Conversion to 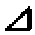 actual measurement value from week 0) | | | | | |
|---|---|---|---|---|---|
| **Group** | **N** | **6W** | **12W** | **24W** | **6-24W (45-48)** |
| **Control** | **15** | **22.9 ± 521.1** | **17.1 ± 445.3** | **-32.7 ± 468.1** | **2.4 ± 468.9** |
| **Citrate** | **16** | **-101.4 ± 666.0** | **45.6 ± 521.2** | **-12.8 ± 578.6** | **-24.4 ± 583.2** |
| **Bicarbonate** | **16** | **-84.0 ± 636.1** | **-171.8 ± 896.9** | **-40.3 ± 514.7** | **-98.6 ± 688.1** |

| | | | | | |
|---|---|---|---|---|---|
| **Mean ± SD** | | | | | |

**[Table 5-2-1]**

| | **Urine Phenyl Acetyl L-Olutamine (PAG)** | | | | | |
|---|---|---|---|---|---|---|
| **Group** | **N** | **0W** | **6W** | **12W** | **24W** | **6-24W (45-48)** |
| **Control** | **15** | **94801 ± 62107** | **86419 ± 91066** | **75897 ± 68238** | **60028 ± 90932^{a}** | **74115 ± 73634** |
| **Citrate** | **16** | **73688 ± 76268** | **73505 ± 49199** | **85591 ±104730** | **91951 ±106696** | **83682 ± 89253** |
| **Bicarbonate** | **16** | **124676 ±149757** | **144753 ±246773** | **82478 ±112943^{b}** | **100902 ± 91424** | **109378 ±163923** |

| | | | | | | |
|---|---|---|---|---|---|---|
| **Mean ± SD** **^{a}p=0.0151, *p=0.0335 vs 0 week (Wilcoxon)** | | | | | | |

**[Table 5-2-2]**

| **Urine PAG** (Conversion to 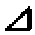 actual measurement value from week 0) | | | | | |
|---|---|---|---|---|---|
| **Group** | **N** | **6W** | **12W** | **24W** | **6-24W (45-48)** |
| **Control** | **15** | **-8382 ± 71407** | **-18904 ± 61477** | **-34773 ± 49511** | **-20687 ± 61034** |
| **Citrate** | **16** | **-183± 55548** | **11902 ± 57092^{a}** | **18263 ± 60893^{b}** | **9994 ± 57168^{c,d}** |
| **Bicarbonate** | **16** | **20077 ± 118811** | **-42198 ± 85069** | **-23774 ±117957** | **-13663 ± 74566** |

| | | | | | |
|---|---|---|---|---|---|
| **Mean ± SD** **^{b}p=0.0105, ^{c}p=0.0062 vs Control and ^{a}p=0.0287, ^{d}p=0.0282 vs Bicarbonate (Mann-Whitney)** | | | | | |

**[Table 5-3-1]**

| **Phenyl Acetyl L-Glutamine (PAG) - Urine/Plasma ratio** | | | | | | |
|---|---|---|---|---|---|---|
| **Group** | **N** | **0W** | **6W** | **12W** | **24W** | **6-24W (45-48)** |
| **Control** | **15** | **97.4 ± 88.9** | **71.1 ± 43.6** | **62.3 ± 30.4** | **53.0 ± 33.5^{c}** | **62.2 ± 36.2** |
| **Citrate** | **16** | **95.0 ± 52.9** | **105.4 ± 74.6** | **99.1 ± 88.6** | **108.5 ± 94.5^{a}** | **104.4 ± 84.4^{b}** |
| **Bicarbonate** | **16** | **102.2 ± 96.5** | **93.7 ± 84.0** | **70.4 ± 46.8** | **97.9 t 116.4** | **87.3 ± 86.2** |

| | | | | | | |
|---|---|---|---|---|---|---|
| **Mean ± SD** **^{a}p=0.0358 and ^{b}p=0.0107 vs Control (Mann-Whitney)** **^{c}p=0.0103 vs 0 week (Wilcoxon)** | | | | | | |

**[Table 5-3-2]**

| **PAG - Urine/Plasma ratio** (Conversion to 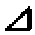 actual measurement value from week 0) | | | | | |
|---|---|---|---|---|---|
| **Group** | **N** | **6W** | **12W** | **24W** | **6-24W (45-48)** |
| **Control** | **15** | **-26.3 ± 82.0** | **-35.1 ± 76.6** | **-44.4 ± 74.7** | **-35.2 ± 76.4** |
| **Citrate** | **16** | **10.4 ± 58.2** | **1.4 ± 81.4** | **13.6 ± 77.5** | **8.6 ± 71.5 ^{b}** |
| **Bicarbonate** | **16** | **-8.5 ± 57.8** | **-31.8 ± 81.8** | **-4.3 ± 133.9** | **-14.9 ± 95.3** |

| | | | | | |
|---|---|---|---|---|---|
| **Mean ± SD** **^{b}p=0.0194 vs Control (Mann-Whitney)** | | | | | |

Effects of potassium citrate/sodium citrate hydrate (Citrate) and sodium bicarbonate (Bicarbonate) on a concentration of each uremic substance in plasma, a concentration of each uremic substance in early morning urine, and a ratio of a concentration of uremic substance in early morning urine to a concentration of uremic substance in plasma are summarized in the following tables. In the tables below, the sodium bicarbonate preparation (Bicarbonate) is a reference drug for the case of the combination preparation of hydrates of potassium citrate and sodium citrate (Citrate), and the combination preparation of hydrates of potassium citrate and sodium citrate (Citrate) is a reference drug for the case of the sodium bicarbonate preparation (Bicarbonate). When the effect is significantly superior to the control group or the reference drug group, O is described, when the effect is significantly inferior to the control group or the reference drug group, X is described, and when there is no significant difference, - is described. The effect of the group to which the sodium bicarbonate preparation (Bicarbonate) had been administered with respect to the control group for the indoxyl sulfate concentration in the early morning urine is indicated as (O) in the tables below. This is because in the group to which the sodium bicarbonate preparation (Bicarbonate) had been administered, the indoxyl sulfate concentration in the early morning urine significantly increased compared to that of the control group, but the indoxyl sulfate concentration in the early morning urine after administration of the sodium bicarbonate preparation (Bicarbonate) decreased compared to before the start of the administration, and thus whether or not there is an effect of promoting excretion of indoxyl sulfate into the urine cannot be determined.

Based on the tables below, the effect of decreasing the concentration of uremic substance in blood (plasma) by the alkalinizing agent was clearly recognized in the cases of indoxyl sulfate (IS) and phenylacetyl-L-glutamine (PAG). Among them, for the case of indoxyl sulfate (IS), administration of the combination preparation of hydrates of potassium citrate and sodium citrate (Citrate) significantly decreased the concentration of uremic substance in blood (plasma) compared to administration of the sodium bicarbonate preparation (Bicarbonate).

In addition, an effect of increasing a uremic substance concentration in urine by the alkalinizing agent (an effect of excreting uremic toxin substances into urine) is clearly recognized by indoxyl sulfate (IS), p-cresyl sulfate (PCS), hippuric acid (HA), argininosuccinic acid (ASA), and phenylacetyl-L-glutamine (PAG). Among them, in the cases of indoxyl sulfate (IS), p-cresyl sulfate (PCS), and phenylacetyl-L-glutamine (PAG), administration of the combination preparation of hydrates of potassium citrate and sodium citrate (Citrate) significantly increased the concentration of uremic substance in urine (excretion into urine) than that by administration of the sodium bicarbonate preparation (Bicarbonate).

The excretion of uremic substance from the blood into the urine by the alkalinizing agent (an effect of excretion outside of the body) is clearly recognized in the cases of indoxyl sulfate (IS), p-cresyl sulfate (PCS), and phenylacetyl-L-glutamine (PAG). Among them, for the case of indoxyl sulfate (IS), administration of the combination preparation of hydrates of potassium citrate and sodium citrate (Citrate) significantly increased excretion of uremic substance from the blood into the urine (excretion outside of the body) compared to administration of the sodium bicarbonate preparation (Bicarbonate).

**[Table 6]**

| Influence of alkalinizing agent on concentration of uremic substance in plasma and in urine - Comparison between Cont and reference drug - | | | | | | | | | | | |
|---|---|---|---|---|---|---|---|---|---|---|---|
| Drug | Sample | is | | PCS | | HA | | ASA | | PAG | |
| | | Cont | Reference drug | Cont | Reference drug | Cont | Reference drug | Cont | Reference drug | Cont | Reference drug |
| Citrate | Plasma | O | O | - | X | - | - | - | - | O | - |
| | Urine | O | O | O | O | O | - | O | - | O | O |
| | U/P ratio | O | O | O | - | - | - | - | - | O | - |
| Bicarbonate | Plasma | - | X | - | O | X | - | - | - | - | - |
| | Urine | (O) | X | - | X | | - | - | - | - | X |
| | U/P ratio | O | X | O | - | X | - | - | - | - | - |

| | | | | | | | | | | | |
|---|---|---|---|---|---|---|---|---|---|---|---|
| Preferable results: Plamsa ↓, Urine ↑, U/P ratio ↑ Excellent effects: O, Poor effects: X, No significant difference: - | | | | | | | | | | | |

Based on the above table, it can be generally understood that the combination preparation of hydrates of potassium citrate and sodium citrate exhibits a greater effect of excreting the uremic substance outside the body compared to the sodium bicarbonate preparation. In addition, administration of the alkalinizing agent to patients with stage G2 chronic kidney disease as well as stage G3b can suppress progression of chronic kidney disease, and it was suggested that the combination preparation of hydrates of potassium citrate and sodium citrate further suppresses the progression of chronic kidney disease than the sodium bicarbonate preparation.

The results of measuring the amount of urinary β2-microglobulin and the amount of cystatin C in serum are shown below.

**[Table 7-1]**

| **Urine *β₂*-microglobulin (µg/L)** | | | | | | |
|---|---|---|---|---|---|---|
| **Group** | **N** | **0W** | **6W** | **12W** | **24W** | **6-24W (45-48)** |
| **Control** | **15** | **100.8 ± 85.2** | **145.8 ± 163.2** | **159.9 ± 167.3** | **187.2± 366.8** | **164.3± 245.9** |
| **Citrate** | **16** | **100.7± 81.2** | **93.1± 46.9** | **88.7± 65.3^{d}** | **154.4 ± 181.8** | **112.6± 117.1^{b}** |
| **Bicarbonate** | **16** | **99.0± 62.8** | **216.3± 228.2^{e}** | **264.5± 301.9** | **261.0± 301.5^{c,f}** | **208.2± 255.8^{a}** |

| | | | | | | |
|---|---|---|---|---|---|---|
| **Mean +/- SD** **^{a}p=0.0338, ^{c}p=0.0220 vs Control and ^{b}p=0.0077, ^{d}p=0.0156 vs Bicarbonate (Mann-Whitney)** **^{e}p=0.0110 and ^{f}p=0.0063 vs 0 week (Wilcoxon)** (No significant difference between groups at week 0) | | | | | | |

**[Table 7-2]**

| **Urine *β*₂-microglobulin** (Conversion to 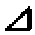 ug/L from week 0) | | | | | |
|---|---|---|---|---|---|
| **Group** | **N** | **6W** | **12W** | **24W** | **6-24W (45-48)** |
| **Control** | **15** | **45.0± 153.3** | **59.1± 161.7** | **86.4± 361.8** | **63.5± 240.3** |
| **Citrate** | **16** | **-14.6± 59.3^{e}** | **-22.2± 98.7^{d}** | **46.7± 189.9** | **3.9± 129.8^{a}** |
| **Bicarbonate** | **16** | **117.3 ± 220.7** | **165.5 ± 282.0** | **162.0 ± 281.8^{c}** | **148.3± 258.4^{b}** |

| | | | | | |
|---|---|---|---|---|---|
| **Mean +/- SD** **^{a}p=0.0095, ^{d}p=0.0415, ^{e}p=0.0295 vs Bicarbonate and ^{b}p=0.0002, ^{c}p=0.0437 vs Control (Mann-Whitney)** (No significant difference between groups at week 0) | | | | | |

**[Table 8]**

| **Plasma Cystatine C (mg/L)** | | | | | | |
|---|---|---|---|---|---|---|
| **Group** | **N** | **0W** | **6W** | **12W** | **24W** | **6·24W (45·48)** |
| **Control** | **16** | **1.209± 0.397** | **1.191± 0.423** | **1.155± 0.372^{a}** | **1.173± 0.339** | **1.173± 0.371** |
| **Citrate** | **16** | **1.040± 0.262** | **1.069± 0.228** | **1.065± 0.254** | **1.089± 0.290** | **1.075± 0.253** |
| **Bicarbonate** | **16** | **1.165± 0.296** | **1.171± 0.281** | **1.151± 0.295** | **1.141± 0.275** | **1.154 ± 0.278** |

| | | | | | | |
|---|---|---|---|---|---|---|
| **Mean +/- SD** **Not Significant between Groups (Mann -Whitney)** **^{a}p=0.0303 vs 0 week (Wilcoxon)** (No significant difference between groups at week 0) | | | | | | |

The concentration of cystatin C in the plasma was not different between the group A (Citrate: the group to which the combination preparation of hydrates of potassium citrate and sodium citrate had been administered), the group B (Bicarbonate: the group to which the sodium bicarbonate preparation had been administered), and the group C (Control: the control group), and there was no influence on the function of glomeruli by the group to which the combination preparation of hydrates of potassium citrate and sodium citrate had been administered, and the group to which the sodium bicarbonate preparation had been administered (Table 8).

On the other hand, regarding a urinary β2-microglobulin concentration, in the group A (Citrate: the group to which the combination preparation of hydrates of potassium citrate and sodium citrate had been administered), a urinary β2-microglobulin concentration was a lower value compared to that of the group C (Control: the control group), and even in comparison with the group B (Bicarbonate: the group to which the sodium bicarbonate preparation had been administered), a urinary β2-microglobulin concentration of the group A (the group to which the combination preparation of hydrates of potassium citrate and sodium citrate had been administered) was a lower value. The group B (Bicarbonate: the group to which the sodium bicarbonate preparation had been administered) had a higher urinary β2-microglobulin concentration than that of the group C (Control: the control group). Administration of the combination preparation of hydrates of potassium citrate and sodium citrate suppresses an increase in urinary β2-microglobulin concentration associated with progression of the stage, and it was recognized that there is no change in the urinary β2-microglobulin concentration compared to before administration.

Based on these results, it was shown that administration of the combination preparation of hydrates of potassium citrate and sodium citrate suppresses kidney tubular damage (kidney proximal tubular damage) associated with progression of the stage. In addition, it was shown that administration of the sodium bicarbonate preparation did not suppress kidney tubular damage (kidney proximal tubular damage) associated with progression of the stage, and it rather worsened the progression. These effects were recognized from 6 weeks after administration.

In addition, an effect of increasing a concentration of uremic substance in urine and an effect of decreasing a concentration of uremic substance in blood by administration of the combination preparation of hydrates of potassium citrate and sodium citrate was not recognized to have a correlation with an effect of suppressing an increase in urinary β2-microglobulin concentration by administration of the combination preparation of hydrates of potassium citrate and sodium citrate. It was suggested that an effect of promoting excretion of uremic substance into urine by administration of the combination preparation of hydrates of potassium citrate and sodium citrate was not caused only by suppression of glomerular and kidney proximal tubular damage.

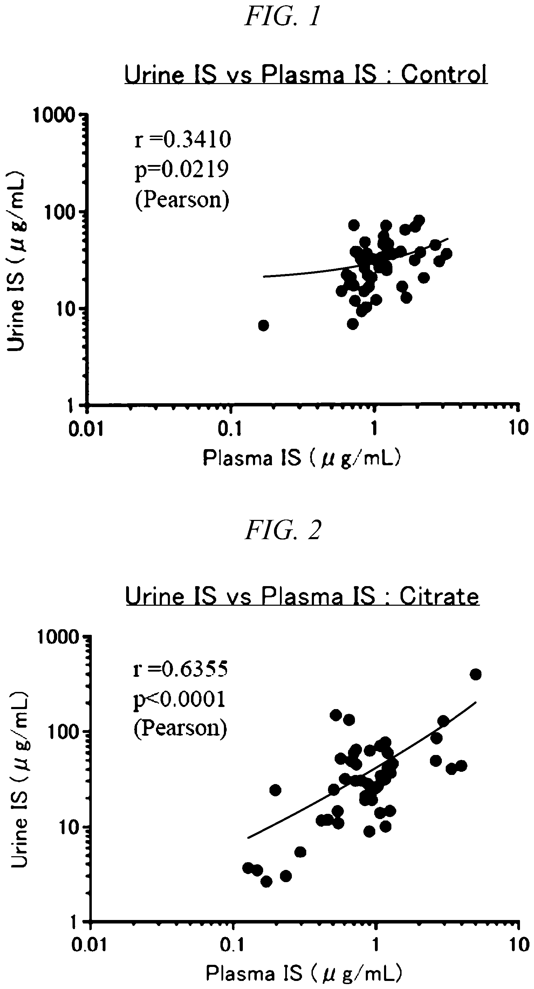

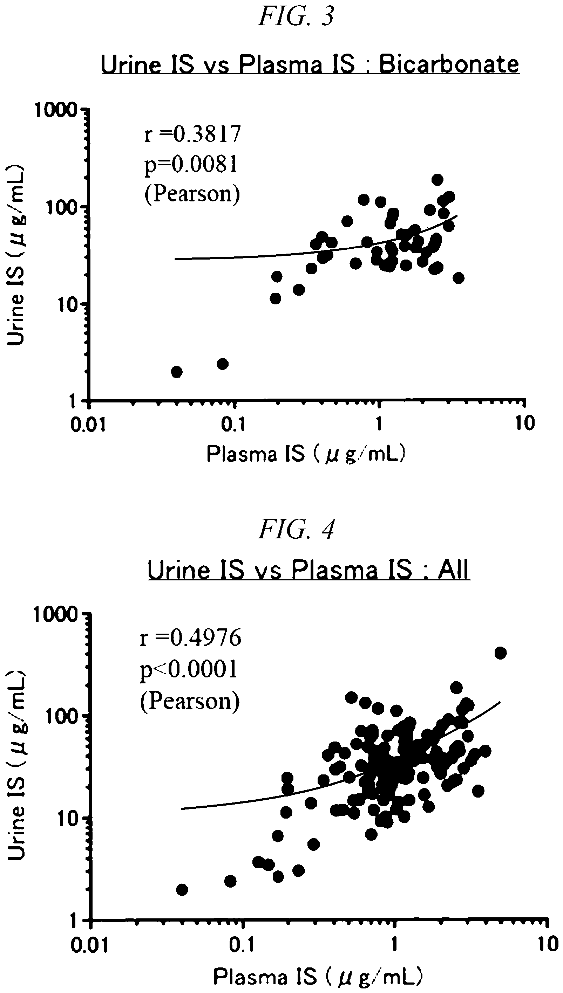

Relevance of each of a concentrations of indoxyl sulfate (IS) in early morning urine and a concentration of indoxyl sulfate (IS) in plasma at 6, 12, and 24 weeks after the start of the test (6W, 12W, and 24W) was analyzed using a Pearson test for each of the group C (Control: the control group), the group A (Citrate: the group to which the combination preparation of hydrates of potassium citrate and sodium citrate had been administered), the group B (Bicarbonate: the group to which the sodium bicarbonate preparation had been administered), and all the patients (all the patients in the groups A, B, and C). The results are shown in FIGS. 1 to 4.

For indoxyl sulfate, a higher correlation between plasma concentration and urine concentration was recognized in the group to which the combination preparation of hydrates of potassium citrate and sodium citrate had been administered as compared to the control group, and a high correlation was recognized even when compared to the group to which the sodium bicarbonate preparation had been administered (refer to r values in FIGS. 1 to 3). Based on FIGS. 1 to 4, it was suggested that administration of the combination preparation of hydrates of potassium citrate and sodium citrate causes excretion of indoxyl sulfate into urine depending on the indoxyl sulfate concentration in blood. It was suggested that excretion of indoxyl sulfate into the urine depending on the indoxyl sulfate concentration in blood suppresses an increase in the concentration of indoxyl sulfate in blood, and a ratio of the indoxyl sulfate concentration in blood to the indoxyl sulfate concentration in urine was within a certain range.

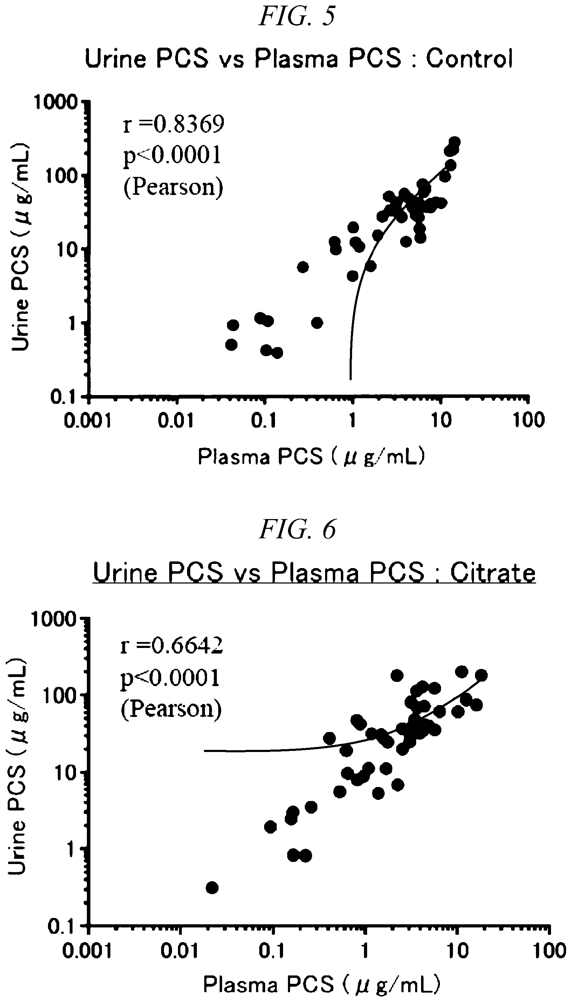

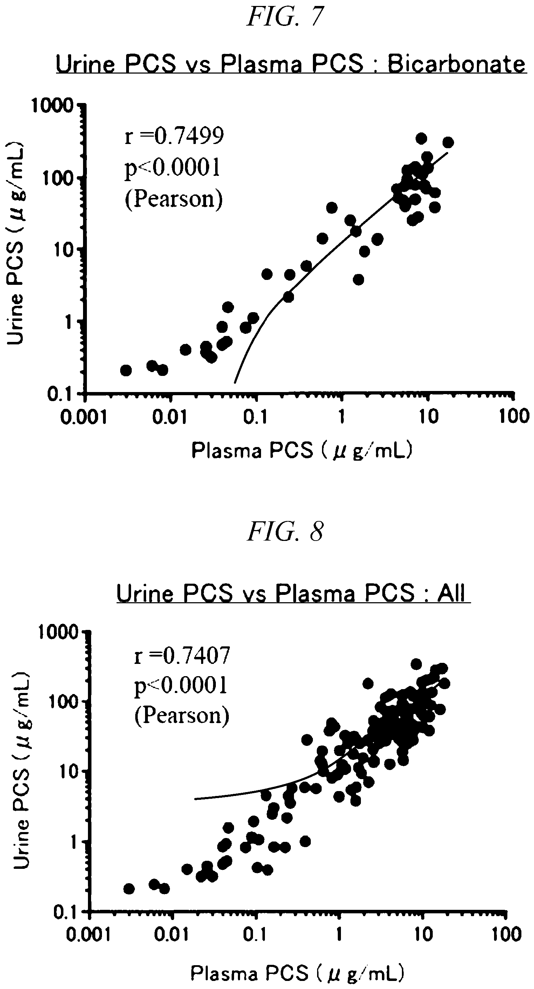

Relevance of each of a concentrations of p-cresyl sulfate (PCS) in early morning urine and a concentration of p-cresyl sulfate (PCS) in plasma at 6, 12, and 24 weeks after the start of the test (6W, 12W, and 24W) was analyzed using a Pearson test for each of the group C (Control: the control group), the group A (Citrate: the group to which the combination preparation of hydrates of potassium citrate and sodium citrate had been administered), the group B (Bicarbonate: the group to which the sodium bicarbonate preparation had been administered), and all the patients (all the patients in the groups A, B, and C). The results are shown in FIGS. 5 to 8.

Regarding p-cresyl sulfate, a correlation between plasma concentration and urine concentration was recognized between the control group, the group to which the combination preparation of hydrates of potassium citrate and sodium citrate had been administered, and the group to which the sodium bicarbonate preparation had been administered, and a higher correlation was recognized in the group to which the sodium bicarbonate preparation had been administered as compared to the group to which the combination preparation of hydrates of potassium citrate and sodium citrate had been administered (refer to r values in FIGS. 5 to 7). Based on FIGS. 5 to 8, it was suggested that administration of the sodium bicarbonate preparation or the combination preparation of hydrates of potassium citrate and sodium citrate causes excretion of p-cresyl sulfate into urine depending on the p-cresyl sulfate concentration in blood. It was suggested that excretion of p-cresyl sulfate into the urine depending on the p-cresyl sulfate concentration in blood suppresses an increase in the concentration of p-cresyl sulfate in blood, and a ratio of the p-cresyl sulfate concentration in blood to the p-cresyl sulfate concentration in urine was within a certain range.

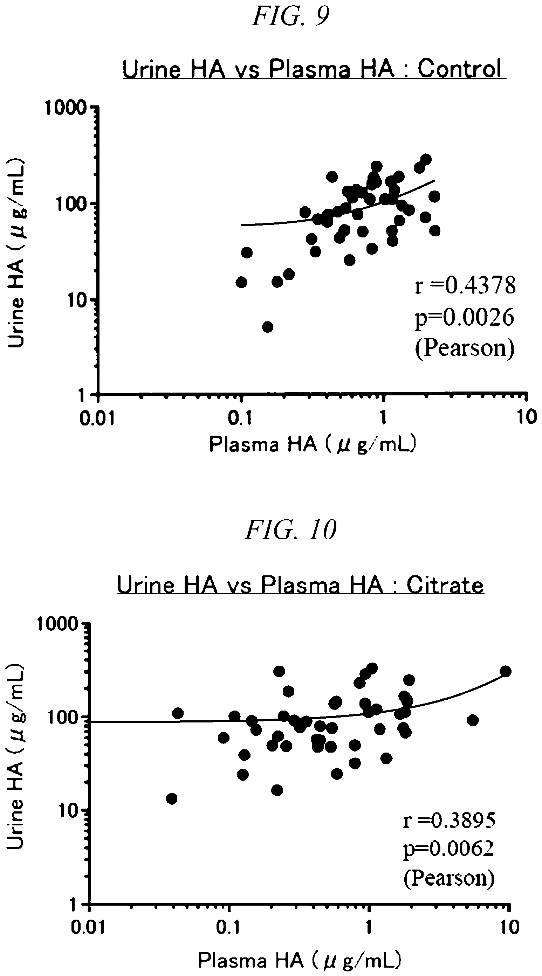

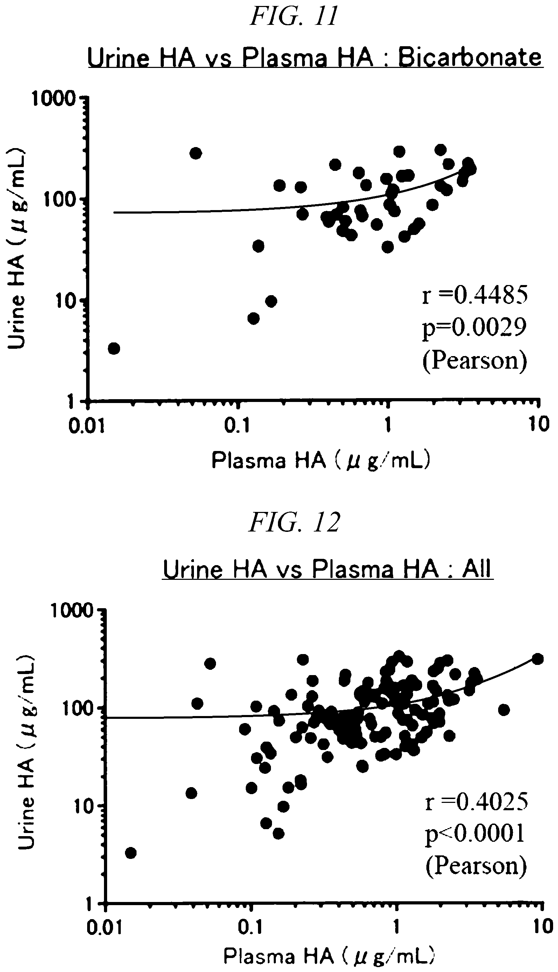

Relevance of each of a concentrations of hippuric acid (HA) in early morning urine and a concentration of hippuric acid (HA) in plasma at 6, 12, and 24 weeks after the start of the test (6W, 12W, and 24W) was analyzed using a Pearson test for each of the group C (Control: the control group), the group A (Citrate: the group to which the combination preparation of hydrates of potassium citrate and sodium citrate had been administered), the group B (Bicarbonate: the group to which the sodium bicarbonate preparation had been administered), and all the patients (all the patients in the groups A, B, and C). The results are shown in FIGS. 9 to 12.

Regarding hippuric acid, no high correlation between plasma concentration and urinary concentration in the control group, the group to which the sodium bicarbonate preparation had been administered, and the group to which the combination preparation of hydrates of potassium citrate and sodium citrate had been administered was recognized.

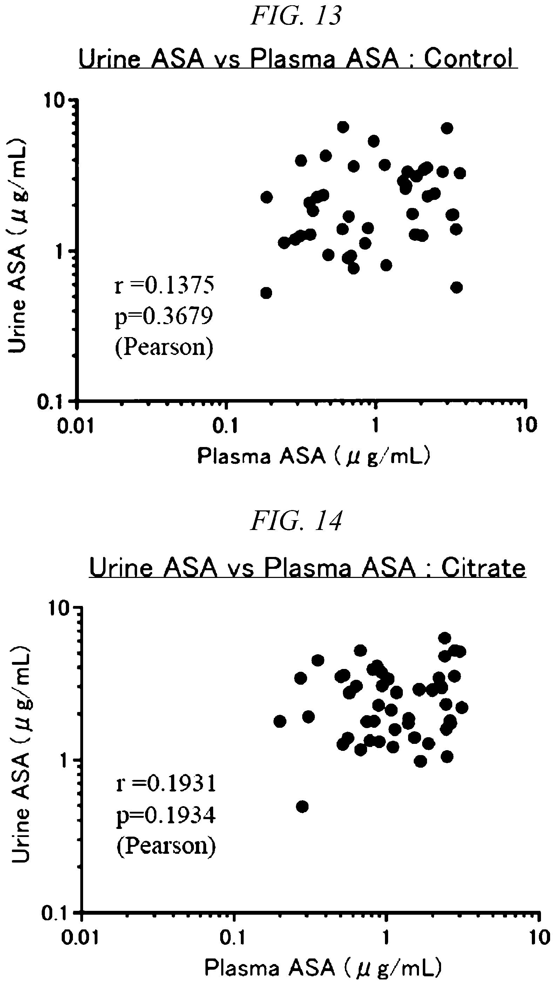

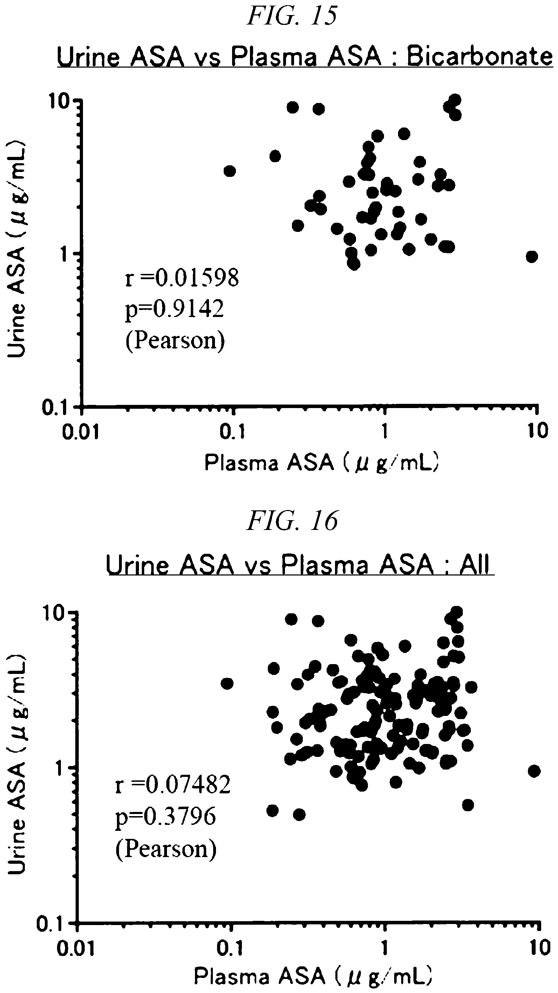

Relevance of each of a concentrations of argininosuccinic acid (ASA) in early morning urine and a concentration of argininosuccinic acid (ASA) in plasma at 6, 12, and 24 weeks after the start of the test (6W, 12W, and 24W) was analyzed using a Pearson test for each of the group C (Control: the control group), the group A (Citrate: the group to which the combination preparation of hydrates of potassium citrate and sodium citrate had been administered), the group B (Bicarbonate: the group to which the sodium bicarbonate preparation had been administered), and all the patients (all the patients in the groups A, B, and C). The results are shown in FIGS. 13 to 16.

Regarding argininosuccinic acid, no high correlation between plasma concentration and urine concentration in the control group, the group to which the sodium bicarbonate preparation had been administered, and the group to which the combination preparation of hydrates of potassium citrate and sodium citrate had been administered was recognized.

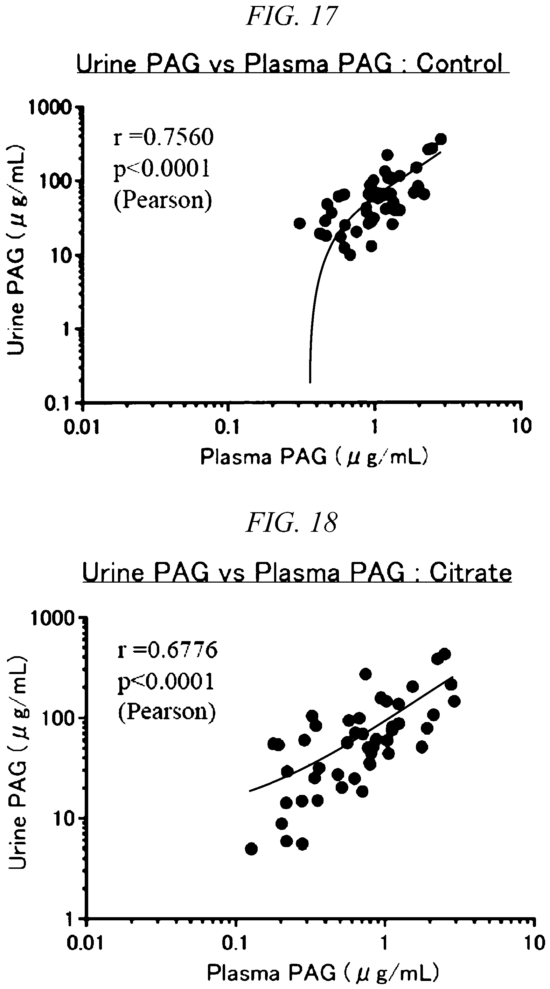

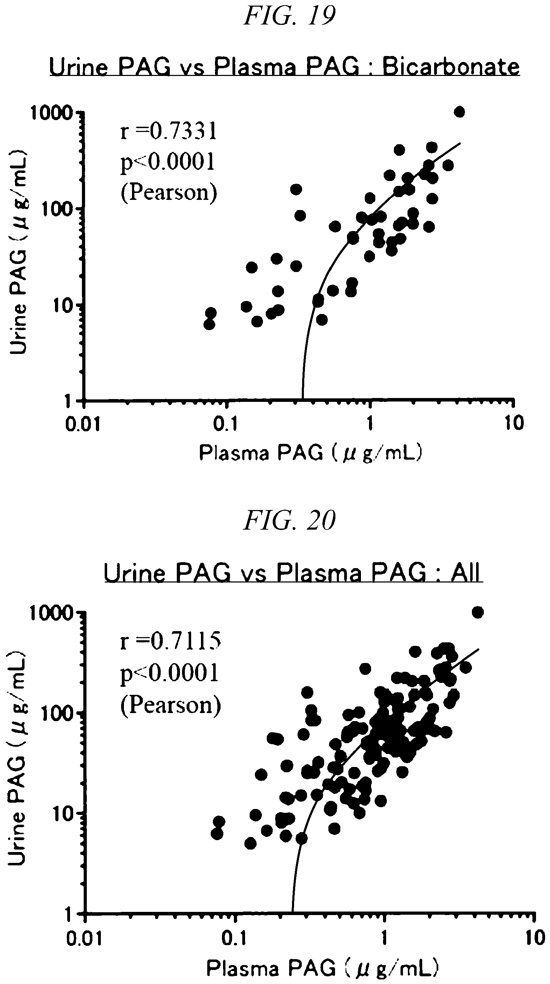

Relevance of each of a concentrations of phenylacetyl-L-glutamine (PAG) in early morning urine and a concentration of phenylacetyl-L-glutamine (PAG) in plasma at 6, 12, and 24 weeks after the start of the test (6W, 12W, and 24W) was analyzed using a Pearson test for each of the group C (Control: the control group), the group A (Citrate: the group to which the combination preparation of hydrates of potassium citrate and sodium citrate had been administered), the group B (Bicarbonate: the group to which the sodium bicarbonate preparation had been administered), and all the patients (all the patients in the groups A, B, and C). The results are shown in FIGS. 17 to 20.

Regarding phenylacetyl-L-glutamine, a correlation between plasma concentration and urine concentration was recognized between the control group, the group to which the combination preparation of hydrates of potassium citrate and sodium citrate had been administered, and the group to which the sodium bicarbonate preparation had been administered, and a higher correlation was recognized in the group to which the sodium bicarbonate preparation had been administered as compared to the group to which the combination preparation of hydrates of potassium citrate and sodium citrate had been administered (refer to r values in FIGS. 17 to 19). Based on FIGS. 17 to 20, it was suggested that administration of the sodium bicarbonate preparation or the combination preparation of hydrates of potassium citrate and sodium citrate causes excretion of phenylacetyl-L-glutamine into the urine depending on the concentration of phenylacetyl-L-glutamine in the blood. It was suggested that excretion of phenylacetyl-L-glutamine into the urine depending on the phenylacetyl-L-glutamine concentration in blood suppresses an increase in the concentration of phenylacetyl-L-glutamine in blood, and a ratio of the phenylacetyl-L-glutamine concentration in blood to the phenylacetyl-L-glutamine concentration in urine falls within a certain range.

Relevance of respective concentrations of indoxyl sulfate (IS), p-cresyl sulfate (PCS), hippuric acid (HA), argininosuccinic acid (ASA), and phenylacetyl-L-glutamine (PAG) in early morning urine at 6, 12, and 24 weeks after the start of the test (6W, 12W, and 24W) was analyzed using a Pearson test for each of the group C (Control: the control group), the group A (Citrate: the group to which the combination preparation of hydrates of potassium citrate and sodium citrate had been administered), and the group B (Bicarbonate: the group to which the sodium bicarbonate preparation had been administered). The results are shown in Table 9. In the table, "Contro" indicates the control group, "Citrate" indicates the group to which the combination preparation of hydrates of potassium citrate and sodium citrate had been administered, and "Bicarb" indicates the group to which the sodium bicarbonate preparation had been administered.

As a result, in the group to which the combination preparation of hydrates of potassium citrate and sodium citrate had been administered high correlation was observed between indoxyl sulfate and phenylacetyl-L-glutamine, p-cresyl sulfate and phenylacetyl-L-glutamine, and argininosuccinic acid and phenylacetyl-L-glutamine. In the group to which the sodium bicarbonate preparation had been administered, high correlation was observed between indoxyl sulfate and argininosuccinic acid, indoxyl sulfate and phenylacetyl-L-glutamine, p-cresyl sulfate and argininosuccinic acid, p-cresyl sulfate and phenylacetyl-L-glutamine, and argininosuccinic acid and phenylacetyl-L-glutamine. It was suggested that the combination preparation of hydrates of potassium citrate and sodium citrate may increase concentrations of indoxyl sulfate, phenylacetyl-L-glutamine, p-cresyl sulfate, and argininosuccinic acid in urine by the same mechanism. In addition, it was suggested that the sodium bicarbonate preparation may increase concentrations of indoxyl sulfate, phenylacetyl-L-glutamine, p-cresyl sulfate, and argininosuccinic acid in urine by the same mechanism.

**[Table 9]**

| Relevance and influence of five uremic substances on concentration in urine | | | | | | | | | | | | |
|---|---|---|---|---|---|---|---|---|---|---|---|---|
| | IS | | | PCS | | | HA | | | ASA | | |
| | Contro | Citrate | Bicarb | Contro | Citrate | Bicarb | Contro | Citrate | Bicarb | Contro | Citrate | Bicarb |
| PCS | 0.5385 | 0.5789 | 0.6500 | - | - | - | - | - | - | - | - | - |
| | 0.0001 | <0.0001 | <0.0001 | | | | | | | | | |
| HA | 0.5439 | 0.4181 | 0.6281 | 0.4106 | 0.5114 | 0.4176 | - | - | - | - | - | - |
| | 0.0001 | 0.0031 | <0.0001 | 0.0051 | 0.0002 | 0.0031 | | | | | | |
| ASA | 0.6935 | 0.6207 | 0.7537 | 0.7531 | 0.6964 | 0.8596 | 0.5791 | 0.2946 | 0.4946 | - | - | - |
| | <0.0001 | <0.0001 | <0.0001 | <0.0001 | <0.0001 | <0.0001 | <0.0001 | 0.0421 | 0.0004 | | | |
| PAG | 0.6184 | 0.8370 | 0.7638 | 0.8811 | 0.8613 | 0.8185 | 0.3582 | 0.3813 | 0.5954 | 0.6510 | 0.7108 | 0.7385 |
| | <0.0001 | <0.0001 | <0.0001 | <0.0001 | <0.0001 | <0.0001 | 0.0157 | 0.0070 | <0.0001 | <0.0001 | <0.0001 | <0.0001 |

| | | | | | | | | | | | | |
|---|---|---|---|---|---|---|---|---|---|---|---|---|
| Upper: correlation coefficient (r), lower: significance level (p), Pearson. r > 0.7 is underlined. 3 group comparison (6 to 24 weeks, n = 45 - 48) | | | | | | | | | | | | |

Relevance of respective concentrations of indoxyl sulfate (IS), p-cresyl sulfate (PCS), hippuric acid (HA), argininosuccinic acid (ASA), and phenylacetyl-L-glutamine (PAG) in plasma at 6, 12, and 24 weeks after the start of the test (6W, 12W, and 24W) was analyzed using a Pearson test for each of the group C (Control: the control group), the group A (Citrate: the group to which the combination preparation of hydrates of potassium citrate and sodium citrate had been administered), and the group B (Bicarbonate: the group to which the sodium bicarbonate preparation had been administered). The results are shown in Table 10. In the table, "Contro" indicates the control group, "Citrate" indicates the group to which the combination preparation of hydrates of potassium citrate and sodium citrate had been administered, and "Bicarb" indicates the group to which the sodium bicarbonate preparation had been administered.

As a result, in the group to which the combination preparation of hydrates of potassium citrate and sodium citrate had been administered high correlation was observed between indoxyl sulfate and p-cresyl sulfate, indoxyl sulfate and phenylacetyl-L-glutamine, and p-cresyl sulfate and phenylacetyl-L-glutamine. In the group to which the sodium bicarbonate preparation had been administered, a high correlation was recognized between indoxyl sulfate and p-cresyl sulfate. It was suggested that the combination preparation of hydrates of potassium citrate and sodium citrate may reduce concentrations of indoxyl sulfate, phenylacetyl-L-glutamine, and p-cresyl sulfate in blood by the same mechanism. In addition, it was suggested that the sodium bicarbonate preparation may reduce concentrations of p-cresyl sulfate and phenylacetyl-L-glutamine in blood by the same mechanism. It was suggested that the combination preparation of hydrates of potassium citrate and sodium citrate and the sodium bicarbonate preparation have different mechanisms for reducing the concentration of uremic substance in blood.

**[Table 10]**

| Relevance and influence of five uremic substances on concentration in plasma | | | | | | | | | | | | |
|---|---|---|---|---|---|---|---|---|---|---|---|---|
| | IS | | | PCS | | | HA | | | ASA | | |
| | Contro | Citrate | Bicarb | Contro | Citrate | Bicarb | Contro | Citrate | Bicarb | Contro | Citrate | Bicarb |
| PCS | 0.1488 | 0.7128 | 0.5973 | - | - | - | - | - | - | - | - | - |
| | 0.3351 | <0.0001 | <0.0001 | | | | | | | | | |
| HA | -0.0656 | 0.2504 | 0.0056 | 0.0424 | 0.2841 | 0.2949 | - | - | - | - | - | - |
| | 0.6686 | 0.0896 | 0.0002 | 0.7849 | 0.0504 | 0.0580 | | | | | | |
| ASA | 0.4001 | 0.3069 | 0.3690 | 0.3629 | 0.1660 | -0.0486 | -0.0538 | 0.0068 | 0.2653 | - | - | - |
| | 0.0065 | 0.0359 | 0.0107 | 0.0155 | 0.2649 | 0.7428 | 0.7255 | 0.9640 | 0.0896 | | | |
| PAG | 0.0333 | 0.7664 | 0.6513 | 0.7552 | 0.9037 | 0.8486 | 0.1058 | 0.2464 | 0.3978 | 0.0792 | 0.1880 | 0.1434 |
| | 0.8280 | <0.0001 | <0.0001 | <0.0001 | <0.0001 | <0.0001 | 0.4890 | 0.0950 | 0.0091 | 0.6050 | 0.2057 | 0.3307 |

| | | | | | | | | | | | | |
|---|---|---|---|---|---|---|---|---|---|---|---|---|
| Upper: correlation coefficient (r), lower: significance level (p), Pearson. r> 0.7 is underlined. 3 group comparison (6 to 24 weeks, n = 45 - 48) | | | | | | | | | | | | |

A urinary specific gravity of early morning urine before the start of the test (0 W) and at 6, 12, and 24 weeks after the start of the test (6 W, 12 W, and 24 W) was analyzed for each of the group C (Control: the control group), the group A (Citrate: the group to which the combination preparation of hydrates of potassium citrate and sodium citrate had been administered), and the group B (Bicarbonate: the group to which the sodium bicarbonate preparation had been administered). The results are shown in Table 11-0-1. In addition, amounts of change in each urinary specific gravity after 6, 12, and 24 weeks after the start of the test are respectively shown as a % relative value with respect to each urinary specific gravity before the start of the test, and a difference from urinary specific gravity before the start of the teste in Tables 11-0-2 and 11-0-3. The urinary specific gravity was measured using a urine hydrometer (PAL-09S, Atago Co., Ltd., Tokyo, Japan).

**[Table 11-0-1]**

| Urine Specific Gravity | | | | | | |
|---|---|---|---|---|---|---|
| Group | N | 0W | 6W | 12W | 24W | 6-24W (45-48) |
| Control | 15 | 1.014 ± 0.005 | 1.013 ± 0.005 | 1.011 ± 0.004^{a} | 1.012 ± 0.005 | 1.012 ± 0.005 |
| Citrate | 16 | 1.012 ± 0.006 | 1.013 ± 0.005 | 1.014 ± 0.006 | 1.014 ± 0.006 | 1.014 ± 0.006 |
| Bicarbonate | 16 | 1.014 ± 0.007 | 1.014 ± 0.006 | 1.013 ± 0.005 | 1.016 ± 0.007 | 1.014 ± 0.006 |

| | | | | | | |
|---|---|---|---|---|---|---|
| Mean +/- SD Not Significant between Groups (Mann-Whitney) ^{a}p=0.0464 vs 0 week (Wilcoxon) Not Significant between Groups (Kruskal-Wallis & Dunn) (No significant difference between groups at week 0) | | | | | | |

**[Table 11-0-2]**

| Urine Specific Gravity (% Relative value vs week 0) | | | | | |
|---|---|---|---|---|---|
| Group | N | 6W | 12W^{b} | 24W | 6-24W^{a} (45-48) |
| Control | 15 | 99.96 ± 0.52 | 99.77 ± 0.40^{d,d} | 99.80 ± 0.51^{c} | 99.84 ± 0.48^{a,b,c} |
| Citrate | 16 | 100.11 ± 0.65 | 100.22 ± 0.54 | 100.23 ± 0.60 | 100.20 ± 0.59 |
| Bicarbonate | 16 | 100.02 ± 0.53 | 99.89 ± 0.58' | 100.17 ± 0.61 | 100.00 ± 0.57 |

| | | | | | |
|---|---|---|---|---|---|
| Mean +/- SD ^{a}p=0.0023, ^{b}p=0.0577,^{c}p=0.0734, ^{d}p=0.0289 and ^{e}p=0.0715 vs Citrate (Mann-Whitney) ^{a}p=0.0100, ^{b}p=0.0652 (Kruskal-Wallis) and ^{c}p=0.0084, ^{d}p=0.0941 vs Citrate (Dunn) | | | | | |

**[Table 11-0-3]**

| Urine Specific Gravity ( 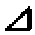 Actual measurement value vs week 0) | | | | | |
|---|---|---|---|---|---|
| Group | N | 6W | 12W^{b} | 24W | 6-24W^{a} (45-48) |
| Control | 15 | -0.0004 ±0.0053 | -0.0023 ±0.0041^{d,d} | -0.0017 ±0.0043^{c} | -0.0015 ±0.0046^{a,c} |
| Citrate | 16 | 0.0011 ±0.0066 | 0.0022 ±0.0055 | 0.0023 ±0.0061 | 0.0019 ±0.0060 |
| Bicarbonate | 16 | 0.0002 ±0.0054 | -0.0012 ±0.0058^{e} | 0.0017 ±0.0062 | 0.0002 ±0.0058^{b} |

| | | | | | |
|---|---|---|---|---|---|
| Mean +/- SD ^{a}p=0.0023,^{b}p=0.0592,^{c}p=0.0704,^{d}p=0.0289 and ^{e}p=0.0746 vs Citrate (Mann-Whitney) ^{a}p=0.0142, ^{b}p=0.0668 (Kruskal-Wallis) and ^{c}p=0.0085, ^{d}p=0.0956 vs Citrate (Dunn) | | | | | |

As a result of the above measurement, compared with the group C (Control: the control group), in the group A (Citrate: the group to which the combination preparation of hydrates of potassium citrate and sodium citrate had been administered) and the group B (Bicarbonate: the group to which the sodium bicarbonate preparation had been administered), the urinary specific gravity value was maintained or increased over 6, 12, and 24 weeks after the start of the test. In addition, compared to the group B (Bicarbonate: the group to which the sodium bicarbonate preparation had been administered), in the group A (Citrate: the group to which the combination preparation of hydrates of potassium citrate and sodium citrate had been administered), the urinary specific gravity showed tendency to be more favorably maintained or to increase.

It could be understood that maintaining or increasing the urinary specific gravity is based on maintenance or improvement of kidney function. Accordingly, based on the above results, administration of the alkalinizing agent to patients with stage G2 chronic kidney disease as well as stage G3b can suppress progression of chronic kidney disease, and it was suggested that the combination preparation of hydrates of potassium citrate and sodium citrate further suppresses the progression of chronic kidney disease than the sodium bicarbonate preparation.

Values obtained by correcting respective concentrations of uremic substance in early morning urine, that is, indoxyl sulfate (IS), p-cresyl sulfate (PCS), phenylacetyl-L-glutamine (PAG), hippuric acid (HA), and argininosuccinic acid (ASA) before the start of the test (0 W), and 6, 12, and 24 weeks after the start of the test (6 W, 12 W, and 24 W) by the following equation using the urinary specific gravity before the start of the test (0 W), and at 6, 12, and 24 weeks after the start of the test (6 W, 12 W, and 24 W) were analyzed for each of the group C (Control: the control group), the group A (Citrate: the group to which the combination preparation of hydrates of potassium citrate and sodium citrate had been administered), and the group B (Bicarbonate: the group to which the sodium bicarbonate preparation had been administered). Medical Examination, Vol. 44, No. 1, 1995, pages 79-83 by Tetsuo Aoki et al. was referred to. A urinary specific gravity 1.022 was converted into a reference value. Specific gravity correction value (unit/1.022·UG) = actual measurement value × (1.022 - 1.000)/(specific gravity value - 1.000)

The results are shown in Table 11-1-1, Table 11-2-1, Table 11-3-1, Table 11-4-1, and Table 11-5-1. In addition, amounts of change in the values obtained by correcting, with the urinary specific gravity, the concentration of the uremic substance in the early morning urine 6, 12, and 24 weeks after the start of the test are respectively shown as a % relative value with respect to the correction value before the start of the test, and a difference from the correction value before the start of the test in Table 11-1-2, Table 11-1-3, Table 11-2-2, Table 11-2-3, Table 11-3-2, Table 11-3-3, Table 11-4-2, Table 11-4-3, Table 11-5-2, and Table 11-5-3.

**[Table 11-1-1]**

| Urine IS (µg·mL⁻¹/ 1.022·UG) | | | | | | |
|---|---|---|---|---|---|---|
| Group | N | 0W^{a} | 6W | 12W | 24W | 6·24W^{b}(45·48) |
| Control | 15 | 65.0 ± 27.5^{a} | 58.7 ± 33.0^{f} | 60.9 ± 24.2 | 53.5 ± 19.6^{e,b} | 57.7 ± 25.8^{c,d} |
| Citrate | 16 | 66.1 ± 51.1^{b} | 58.6 ± 34.6 | 74.9 ± 81.5 | 71.1 ± 54.6 | 68.2 ± 59.2^{d} |
| Bicarbonate | 16 | 92.6 ± 43.5^{c} | 83.3 ± 41.9 | 65.5 ± 29.0^{a} | 81.1 ± 41.6 | 76.7 ± 38.0 |

| | | | | | | |
|---|---|---|---|---|---|---|
| Mean +/- SD ^{a}p=0.0849,^{b}p=0.0559,^{c}p=0.0065,^{d}p=0.0729,^{e}p=0.0266 and ^{f}p=0.0849 vs Bicarbonate (Mann-Whitney) ^{a}p=0.0052 and ^{b}p=0.0730 vs 0 week (Wilcoxon) ^{a}p=0.0844,^{b}p=0.0301 (Kruskal-Wallis) and ^{c}p=0.0909,^{d}p=0.0385 vs Citrate (Dunn) (There is a difference between groups at week 0) | | | | | | |

**[Table 11-1-2]**

| Urine IS (µg ·mL⁻¹ / 1.022-UG, % Relative value vs week 0 ) | | | | | |
|---|---|---|---|---|---|
| Group | N | 6W | 12W | 24W | 6·24W^{a}(45·48) |
| Control | 15 | 88 ± 26 | 118 ± 101 | 96 ± 55 | 101 ± 67 |
| Citrate | 16 | 103 ± 67 | 110 ± 62 | 117 ± 70 | 110 ± 65 |
| Bicarbonate | 16 | 95 ± 30 | 77 ± 42 | 95 ± 59 | 89 ± 45 |

| | | | | | |
|---|---|---|---|---|---|
| Mean +/- SD Not Significant between Groups (Mann-Whitney) Not Significant between Groups (Kruskal-Wallis & Dunn) | | | | | |

**[Table 11-1-3]**

| Urine IS (µg ·mL⁻¹ / 1.022 -UG, 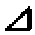 Actual measurement value vs week 0) | | | | | |
|---|---|---|---|---|---|
| Group | N | 6W | 12W^{b} | 24W | 6·24W^{a}(45·48) |
| Control | 15 | - 6.3 ± 17.4 | - 4.2 ± 27.0^{c} | - 13.8 ± 20.9 | - 8.1 ± 22.0 |
| Citrate | 16 | - 7.5 ± 36.7 | 8.8 ± 44.6^{b,d} | 5.0 ± 44.4 | 2.1 ± 41.8^{a,c} |
| Bicarbonate | 16 | - 9.3 ± 27.7 | - 27.0 ± 32.9 | - 11.4 ± 40.8 | - 15.9 ± 34.4 |

| | | | | | |
|---|---|---|---|---|---|
| Mean +/- SD ^{a}p=0.0251,^{b}p=0.0135 and ^{c}p=0.0651 vs Bicarbonate (Mann-Whitney) ^{a}p=0.0648,^{b}p=0.0368 (Kruskal-Wallis) and ^{c}p=0.0581,^{d}p=0.0429 vs Citrate (Dunn) | | | | | |

**[Table 11-2-1]**

| Urine PCS (µg ·mL⁻¹/ 1.022·UG) | | | | | | |
|---|---|---|---|---|---|---|
| Group | N | 0W | 6W | 12W | 24W | 6-24W(45-48) |
| Control | 14-15 | 86.7 ± 80.6 | 91.8 ± 72.3 | 73.8 ± 89.6 | 59.8 ± 69.8 | 74.8 ± 77.2 |
| Citrate | 16 | 61.1 ± 64.3 | 74.6 ± 59.6 | 68.7 ± 60.2 | 91.2 ± 101.0 | 78.2 ± 75.1 |
| Bicarbonate | 16 | 113.0 ±119.5 | 98.1 ± 118.3 | 66.7 ± 73.0 | 84.8 ± 86.0 | 83.2 ± 93.2 |

| | | | | | | |
|---|---|---|---|---|---|---|
| Mean +/- SD Not Significant between Groups (Mann-Whitney) ^{a}p=0.0507 vs 0 week (Wilcoxon) Not Significant between Groups (Krurkal-Wallis & Dunn) (No significant difference between groups at week 0) | | | | | | |

**[Table 11-2-2]**

| Urine PCS (µg ·mL⁻¹ / 1.022. UG, % Relative value vs week 0 ) | | | | | |
|---|---|---|---|---|---|
| Group | N | 6W^{c} | 12W | 24W^{b} | 6-24W^{a}(45-48) |
| Control | 14-15 | 152 ± 170 | 117 ± 169 | 85 ± 97^{c,f} | 121 ± 149^{a,d} |
| Citrate | 16 | 193 ± 160 | 343 ± 829 | 209 ± 216 | 248 ± 497 |
| Bicarbonate | 16 | 83 ± 34^{d,g} | 105 ± 112 | 265 t 540 | 151 ± 323^{b,e} |

| | | | | | |
|---|---|---|---|---|---|
| Mean +/- SD ^{a}p=0.0095, ^{b}p=0.0125, ^{c}p=0.0149, ^{d}p=0.0240 vs Citrate (Mann-Whitney) ^{a}p=0.0129,^{b}p=D.0449,^{c}p=0.0869 (Kruskal-Wallis) and ^{d}p=0.0250,^{e}p=0.0439,^{f}p=0.0393, ^{g}p=0.0849 vs Citrate (Dunn) | | | | | |

**[Table 11-2-3]**

| Urine PCS (µg ·mL⁻¹ / 1.022-UG, 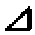 Actual measurement value vs week 0 ) | | | | | |
|---|---|---|---|---|---|
| Group | N | 6W | 12W^{c} | 24W^{b} | 6-24W^{a}(45-48) |
| Control | 14-15 | - 1.0 ± 41.7 | - 12.9 ± 58.7 | - 28.7 ± 50.2^{c,f} | - 14.3 ± 51.4^{a,e} |
| Citrate | 16 | 13.5 ± 39.7 | 7.6 ± 53.3 | 30.1 ± 64.2 | 17.1 ± 53.1 |
| Bicarbonate | 16 | -14.9 ± 36.6^{e} | - 46.3 ± 68.4^{d,g} | - 28.3 ± 84.4 | - 29.8 ± 66.1^{b,d} |

| | | | | | |
|---|---|---|---|---|---|
| Mean +/- SD ^{a}p=0.0226, ^{b}p=0.0007, ^{c}p=0.0214, ^{d}p=0.0287 and ^{e}p=0.0465 vs Citrate (Mann-Whitney) ^{e}p=0.0025,^{b}p=0.0661,^{c}p=0.0795 (Kruskal-Wallis) and ^{d}p=0.0020,^{e}p=0.0736,^{f}p=0.0863, ^{g}p=0.0849 vs Citrate (Dunn) | | | | | |

**[Table 11-3-1]**

| Urine PAG (µg ·mL⁻¹/ 1.022·UG) | | | | | | |
|---|---|---|---|---|---|---|
| Group | N | 0W | 6W | 12W | 24W | 6-24W(45-48) |
| Control | 15 | 146.4 ± 88.2 | 143.8 ±110.4 | 144.8 ±105.4 | 108.9 ± 82.4^{a} | 132.5 ± 99.3 |
| Citrate | 16 | 124.2 ± 94.3 | 143.8 ± 145.6 | 126.4 ±108.7 | 145.3 ±147.4 | 138.5 ±132.5 |
| Bicarbonate | 16 | 203.6 ±169.1 | 202.9 ±260.9 | 136.0 ±141.4^{b} | 147.1 ±116.4 | 162.0 ±182.5 |

| | | | | | | |
|---|---|---|---|---|---|---|
| Mean +/- SD Not Significant between Groups (Mann-Whitney) ^{a}p=0.0302 and ^{b}p=0.0110 vs 0 week (Wilcoxon) Not Significant between Groups (Kruskal-Wallis & Dunn) (No significant difference between groups at week 0) | | | | | | |

**[Table 11-3-2]**

| Urine PAG (µg ·mL⁻¹ / 1.022-UG, % Relative value vs week 0 ) | | | | | |
|---|---|---|---|---|---|
| Group | N | 6W | 12W | 24W | 6-24W^{a}(45-48) |
| Control | 15 | 108 ± 70 | 113 ± 67^{b} | 85 ± 48 | 102 ± 62 |
| Citrate | 16 | 132 ± 104 | 113 ± 93 | 122 ± 108 | 123 ± 100^{a} |
| Bicarbonate | 16 | 97 ± 71 | 76 ± 60 | 88 ± 49 | 87 ± 60 |

| | | | | | |
|---|---|---|---|---|---|
| Mean +/- SD ^{a}p=0.0828 and ^{b}p=0.0799 vs Bicarbonate (Mann-Whitney) Not Significant between Groups (Kruskal-Wallis & Dunn) | | | | | |

**[Table 11-3-3]**

| Urine PAG (µg ·mL⁻¹ / 1.022-UG, 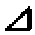 Actual measurement value vs week 0 ) | | | | | |
|---|---|---|---|---|---|
| Group | N | 6W | 12W^{b} | 24W | 6-24W^{a}(45-48) |
| Control | 15 | - 2.6 ± 86.0 | - 1.6 ± 84.1^{d} | - 36.0 ± 70.7 | - 13.4 ± 80.4 |
| Citrate | 16 | 19.7 ± 90.3 | 2.3 ± 61.7^{c} | 18.0 ± 59.8^{b} | 14.4 ± 79.9^{a,c} |
| Bicarbonate | 16 | -0.7 ±162.0 | - 67.6 ± 91.9 | - 56.5 ± 113.7 | - 41.6 ±126.8 |

| | | | | | |
|---|---|---|---|---|---|
| Mean +/- SD ^{a}p=0.0078, ^{c}p=0.0385, ^{d}p=0.0491 vs Bicarbonate and ^{b}p=0.0712 vs Control (Mann-Whitney) ^{a}p=0.0248, ^{b}p=0.0613 (Kruskal-Wallis) and ^{c}p=0.0197 vs Bicarbonate (Dunn) | | | | | |

**[Table 11-4-1]**

| Urine HA (µg ·mL⁻¹/ 1.022-UG) | | | | | | |
|---|---|---|---|---|---|---|
| Group | N | 0W | 6W | 12W | 24W | 6-24W(45-48) |
| Control | 15 | 219.0 ±157.4 | 188.8 ±124.5 | 169.2 ±64.3 | 164.3 +90.9 | 174.1 ±94.9 |
| Citrate | 16 | 201.2 ±109.8 | 209.3 ±160.3 | 176.9 ± 97.7 | 176.1 ±129.8 | 187.7 ±120.5 |
| Bicarbonate | 16 | 202.3 ±185.6 | 161.8 ±111.1 | 171.1 ±87.0 | 177.7 ±111.0 | 170.2 ±101.6 |

| | | | | | | |
|---|---|---|---|---|---|---|
| Mean +/- SD Not Significant between Groups (Mann-Whitney) Not Significant vs 0 week (Wilcoxon) Not Significant between Groups (Kruskal-Wallis & Dunn) (No significant difference between groups at week 0) | | | | | | |

**[Table 11-4-2]**

| Urine HA (µg ·m^{L-1} I 1.022·UG, % Relative value vs week 0 ) | | | | | |
|---|---|---|---|---|---|
| Group | N | 6W | 12W | 24W | 6-24W(45-48) |
| Control | 15 | 97 ± 52 | 110 ± 87 | 119 ± 113 | 109 ± 86 |
| Citrate | 16 | 146 ± 157 | 119 ± 104 | 125 ± 121 | 130 ± 127 |
| Bicarbonate | 16 | 104 ± 89 | 133 ± 134 | 118 ± 96 | 118 ± 106 |

| | | | | | |
|---|---|---|---|---|---|
| Mean +/- SD Not Significant between Groups (Mann-Whitney) Not Significant between Groups (Kruskal-Wallis & Dunn) | | | | | |

**[Table 11-4-3]**

| Urine HA (µg ·mL⁻¹ / 1.022 -UG, 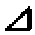 Actual measurement value vs week 0 ) | | | | | |
|---|---|---|---|---|---|
| Group | N | 6W | 12W^{b} | 24W | 6-24W(45-48) |
| Control | 15 | - 30.2 ±113.2 | - 49.8 ±125.1 | - 52.6 ±121.9 | - 44.2±117.8 |
| Citrate | 16 | 8.0 ±139.4 | - 24.3 ± 91.6 | - 25.1 ±162.6 | - 13.8±132.5 |
| Bicarbonate | 16 | - 40.5 ±171.7 | - 31.2 ± 163.7 | - 24.6 ±155.3 | - 32.1 ± 160.3 |

| | | | | | |
|---|---|---|---|---|---|
| Mean +/- SD Not Significant between Groups (Mann-Whitney) Not Significant between Groups (Kruskal-Wallis & Dunn) | | | | | |

**[Table 11-5-1]**

| | Urine ASA (µg ·mL⁻¹/ 1.022-UG) | | | | | |
|---|---|---|---|---|---|---|
| Group | N | 0W | 6W | 12W | 24W | 6-24W(45-48) |
| Control | 15 | 4.60 ± 1.77 | 4.43 ± 1.78 | 4.31 ± 2.07 | 3.66 ± 1.15^{a} | 4.13 ± 1.71 |
| Citrate | 16 | 4.77 ± 1.15 | 4.36 ± 1.90 | 4.37 ± 1.80 | 4.64 ± 2.19 | 4.46 ± 1.93 |
| Bicarbonate | 16 | 5.87 ± 5.13 | 5.47 ± 3.87 | 4.66 ± 2.78 | 4.93 ± 3.53 | 5.02 ± 3.37 |

| | | | | | | |
|---|---|---|---|---|---|---|
| Mean +/- SD Not Significant between Groups (Mann-Whitney) ^{a}p=0.0413 vs 0 week (Wilcoxon) Not Significant between Groups (Kruskal-Wallis & Dunn) (No significant difference between groups at week 0) | | | | | | |

**[Table 11-5-2]**

| Urine ASA (µg ·mL⁻¹ / 1.022·UG, % Relative value vs week 0 ) | | | | | |
|---|---|---|---|---|---|
| Group | N | 6W | 12W | 24W | 6-24W(45-48) |
| Control | 15 | 101 ± 39 | 97 ± 40 | 88 ± 44 | 96 ± 41 |
| Citrate | 16 | 101 ± 60 | 103 ± 66 | 106 ± 52 | 103 ± 59 |
| Bicarbonate | 16 | 101 ± 32 | 93 ± 29 | 97 ± 33 | 97 ± 31 |

| | | | | | |
|---|---|---|---|---|---|
| Mean +/- SD Not Significant between Groups (Mann-Whitney) Not Significant between Groups (Kruskal-Wallis & Dunn) | | | | | |

**[Table 11-5-3]**

| Urine ASA (µg ·mL⁻¹ / 1.022·UG, 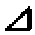 Actual measurement value vs week 0 ) | | | | | |
|---|---|---|---|---|---|
| Group | N | 6W | 12W | 24W | 6-24W (45-48) |
| Control | 15 | - 0.17 ± 1.65 | - 0.29 ± 1.54 | - 1.14 ± 1.60 | - 0.53 ± 1.62 |
| Citrate | 16 | - 0.41 ± 1.95 | 0.14 ± 1.74 | - 0.13 ± 1.55 | 0.32 ± 2.05 |
| Bicarbonate | 16 | - 0.40 ± 2.25 | . 0.21 ± 3.04 | - 0.94 ± 3.02 | - 0.85 ± 2.76 |

| | | | | | |
|---|---|---|---|---|---|
| Mean +/· SD Not Significant between Groups (Mann-Whitney) Not Significant between Groups (Kruskal-Wallis & Dunn) | | | | | |

As a result, in the cases of indoxyl sulfate (IS), p-cresyl sulfate (PCS), and phenylacetyl-L-glutamine (PAG), the concentration of uremic substance in urine (excretion into urine) significantly increased in the group to which the combination preparation of hydrates of potassium citrate and sodium citrate (Citrate) had been administered than that group to which the sodium bicarbonate preparation (Bicarbonate) had been administered and the control group. Also regarding the cases of hippuric acid (HA) and argininosuccinic acid (ASA), the concentration of uremic substance in urine (excretion into urine) increased in the group to which the combination preparation of hydrates of potassium citrate and sodium citrate (Citrate) had been administered than that group to which the sodium bicarbonate preparation (Bicarbonate) had been administered and the control group. In addition, administration of the combination preparation of hydrates of potassium citrate and sodium citrate (Citrate) increased concentrations of indoxyl sulfate (IS), p-cresyl sulfate (PCS), and phenylacetyl-L-glutamine (PAG) in the urine (excretion into urine) compared to before the start of the test (0 W).

The osmotic pressure of early morning urine before the start of the test (0 W) and at 6, 12, and 24 weeks after the start of the test (6 W, 12 W, and 24 W) was analyzed for each of the group C (Control: the control group), the group A (Citrate: the group to which the combination preparation of hydrates of potassium citrate and sodium citrate had been administered), and the group B (Bicarbonate: the group to which the sodium bicarbonate preparation had been administered). The results are shown in Table 12-0-1. In addition, amounts of change in the osmotic pressure of each early morning urine after 6, 12, and 24 weeks after the start of the test are respectively shown as a % relative value with respect to the osmotic pressure of the early morning urine before the start of the test, and a difference from osmotic pressure of early morning urine before the start of the teste in Tables 12-0-2 and 12-0-3. The osmotic pressure was measured using the freezing point depression method.

**[Table 12-0-1]**

| | Urine Osmotic Pressure (mOsm/kg) | | | | | |
|---|---|---|---|---|---|---|
| Group | N | 0W | 6W | 12W | 24W | 6-24W(45-48) |
| Control | 15 | 489 ± 162 | 487 ± 167 | 413 ± 137^{a} | 428 ± 169 | 443 ± 158 |
| Citrate | 16 | 418 ± 197 | 440 ± 168 | 477 ± 209 | 482 ± 177 | 467 ± 182 |
| Bicarbonate | 16 | 492 ± 206 | 488 ± 154 | 456 ± 143 | 515 ± 195 | 486 ± 164 |

| | | | | | | |
|---|---|---|---|---|---|---|
| Mean +/- SD Not Significant between Groups (Mann-Whitney) ^{a}p=0.0616 vs 0 week (Wilcoxon) Not Significant between Groups (Kruskal-Wallis & Dunn) (No significant difference between groups at week 0) | | | | | | |

**[Table 12-0-2]**

| Urine Osmotic Pressure (% Relative value vs week 0) | | | | | |
|---|---|---|---|---|---|
| Group | N | 6W | 12W | 24W | 6-24W^{a}(45-48) |
| Control | 15 | 108 ± 45 | 89 ± 26^{c} | 95 ± 38 | 97 ± 37^{a,b} |
| Citrate | 16 | 121 ± 52 | 123 ± 48 | 142 ± 101 | 129 ± 70 |
| Bicarbonate | 16 | 108 ± 34 | 105 ± 57 | 114 ± 45 | 109 ± 45^{b} |

| | | | | | |
|---|---|---|---|---|---|
| Mean +/- SD ^{a}p=0.0123, ^{b}p=0.0997 and ^{c}p=0.0436 vs Citrate (Mann-Whitney) ^{a}p=0.0382 (Kruskal-Wallis) and ^{b}p=0.0358 vs Citrate (Dunn) | | | | | |

**[Table 12-0-3]**

| Urine Osmotic Pressure ( 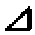 mOsm/kg vs week 0) | | | | | |
|---|---|---|---|---|---|
| Group | N | 6W | 12W | 24W | 6-24W^{a}(45-48) |
| Control | 15 | -2 ± 170 | -76 ± 132^{b} | -47 ± 147 | -41 ± 150^{a,b} |
| Citrate | 16 | 22 ± 206 | 59 ± 184 | 64 ± 230 | 48 ± 204 |
| Bicarbonate | 16 | -4 ± 165 | -36 ± 176 | 23 ± 169 | -6 ± 168 |

| | | | | | |
|---|---|---|---|---|---|
| Mean +/- SD ^{a}p=0.0179 and ^{b}p=0.0375 vs Citrate (Mann-Whitney) ^{a}p=0.0597 (Kruskal-Wallis) and ^{b}p=0.0618 vs Citrate (Dunn) | | | | | |

As a result of the above measurement, compared with the group C (Control: the control group), in the group A (Citrate: the group to which the combination preparation of hydrates of potassium citrate and sodium citrate had been administered) and the group B (Bicarbonate: the group to which the sodium bicarbonate preparation had been administered), the osmotic pressure value of early morning urine was maintained or increased over 6, 12, and 24 weeks after the start of the test. In addition, compared to the group B (Bicarbonate: the group to which the sodium bicarbonate preparation had been administered), in the group A (Citrate: the group to which the combination preparation of hydrates of potassium citrate and sodium citrate had been administered), an osmotic pressure in the early morning urine showed tendency to be more favorably maintained or to increase.

It could be understood that maintaining or increasing the osmotic pressure of early morning urine is based on maintenance or improvement of kidney function. Accordingly, based on the above results, administration of the alkalinizing agent to patients with stage G2 chronic kidney disease as well as stage G3b can suppress progression of chronic kidney disease, and it was suggested that the combination preparation of hydrates of potassium citrate and sodium citrate further suppresses the progression of chronic kidney disease than the sodium bicarbonate preparation.

Values obtained by correcting respective concentrations of uremic substance in early morning urine, that is, indoxyl sulfate (IS), p-cresyl sulfate (PCS), phenylacetyl-L-glutamine (PAG), hippuric acid (HA), and argininosuccinic acid (ASA) before the start of the test (0 W), and 6, 12, and 24 weeks after the start of the test (6 W, 12 W, and 24 W) by the following equation using the osmotic pressure of early morning urine before the start of the test (0 W), and at 6, 12, and 24 weeks after the start of the test (6 W, 12 W, and 24 W) were analyzed for each of the group C (Control: the control group), the group A (Citrate: the group to which the combination preparation of hydrates of potassium citrate and sodium citrate had been administered), and the group B (Bicarbonate: the group to which the sodium bicarbonate preparation had been administered). Medical Examination, Vol. 44, No. 1, 1995, pages 79-83 by Tetsuo Aoki et al. was referred to. In addition, a urine osmotic pressure 770 mOsm/kg was converted into a reference value.

Osmotic pressure correction value (unit/500 mOsm·P) = actual measurement value x 500/osmotic pressure value

The results are shown in Table 12-1-1, Table 12-2-1, Table 12-3-1, Table 12-4-1, and Table 12-5-1. In addition, amounts of change in the values obtained by correcting, with the osmotic pressure of the early morning urine, the concentration of the uremic substance in the early morning urine 6, 12, and 24 weeks after the start of the test are respectively shown as a % relative value with respect to the correction value before the start of the test, and a difference from the correction value before the start of the test in Table 12-1-2, Table 12-1-3, Table 12-2-2, Table 12-2-3, Table 12-3-2, Table 12-3-3, Table 12-4-2, Table 12-4-3, Table 12-5-2, and Table 12-5-3.

**[Table 12-1-1]**

| Urine IS ·mL⁻¹/ 770 mOsm·P) | | | | | | |
|---|---|---|---|---|---|---|
| Group | N | 0W^{a} | 6W | 12W | 24W | 6-24W^{b}(45-48) |
| Control | 15 | 65.4 ± 32.0 | 56.5 ± 32.1^{a} | 59.7 ± 26.0 | 51.0 ± 19.7^{d,b} | 55.7 ± 26.1^{b,d} |
| Citrate | 16 | 67.1 ± 59.5^{a,c} | 59.4 ± 36.8 | 77.7 ± 91.9 | 70.8 ± 58.1 | 69.3 ± 65.3^{c} |
| Bicarbonate | 16 | 87.9 ± 40.0 | 82.9 ± 44.6 | 63.9 ± 32.4^{a} | 84.4 ± 48.4 | 77.0 ± 42.5 |

| | | | | | | |
|---|---|---|---|---|---|---|
| Mean +/- SD ^{a}p=0.0424,^{b}p=0.0060,^{c}p=0.0939,^{d}p=0.0214 and ^{e}p=0.0650 vs Bicarbonate (Mann⁻Whiₜney) ^{a}p=0.0092 and ^{b}p=0.0413 vs 0 week (Wilcoxon) ^{a}p=0.0830,^{b}p=0.0266 (Kruskal-Wallis) and ^{c}p=0.0880,^{d}p=0.0416 vs Bicarbonate (Dunn) (There is a significant difference between groups at week 0) | | | | | | |

**[Table 12-1-2]**

| Urine IS (µg ·mL⁻¹ 1 770 mOsm·P, % Relative value vs week 0 ) | | | | | |
|---|---|---|---|---|---|
| Group | N | 6W | 12W | 24W | 6-24W^{a}(45-48) |
| Control | 15 | 87 ± 29 | 119 ± 107 | 93 ± 54 | 100 ± 71 |
| Citrate | 16 | 107 ± 68 | 114 ± 64 | 119 ± 68 | 114 ± 66 |
| Bicarbonate | 16 | 98 ± 30 | 77 ± 39 | 104 ± 68 | 93 ± 49 |

| | | | | | |
|---|---|---|---|---|---|
| Mean +/- SD Not Significant between Groups (Mann-Whitney) Not Significant between Groups (Kruskal-Wallis & Dunn) | | | | | |

**[Table 12-1-3]**

| Urine IS (µg ·mL⁻¹ / 770 mOsm·P, 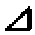 Actual measurement value vs week 0) | | | | | |
|---|---|---|---|---|---|
| Group | IV | 6W | 12W^{a} | 24W | 6-24W(45-48) |
| Control | 15 | - 8.9 ± 20.4 | . 5.7 ± 29.5 | - 16.7 ± 25.0 | - 10.4 ± 25.1 |
| Citrate | 16 | - 7.6 ± 41.0 | 10.6 ± 45.6^{a,b} | 3.7 ± 46.5 | 2.2 ± 44.1 |
| Bicarbonate | 16 | - 5.1 ± 28.3 | - 24.1 ± 33.2 | - 3.6 ± 49.0 | - 10.9 ± 38.2 |

| | | | | | |
|---|---|---|---|---|---|
| Mean +/- SD ^{a}p=0.0169 vs Bicarbonate (Mann-Whitney) ^{a}p=0.0492 (Kruskal-Wallis) and ^{b}p=0.0445 vs Bicarbonate (Dunn) | | | | | |

**[Table 12-2-1]**

| Urine PCS (µg·mL⁻¹ / 770 mOsm·P) | | | | | | |
|---|---|---|---|---|---|---|
| Group | N | 0W^{a} | 6W | 12W | 24W | 6-24W(45-48) |
| Control | 15 | 88.1 ± 82.8 | 90.4 ± 75.6 | 72.0 ± 87.1 | 56.4 ± 64.6^{a} | 72.5 ± 75.9 |
| Citrate | 16 | 62.4 ± 72.2 | 75.3 ± 61.9 | 70.6 ± 62.1^{b} | 90.2 ± 105.0 | 78.7 ± 77.8 |
| Bicarbonate | 16 | 108.8 ±118.2 | 100.7 ± 126.6 | 66.9 ± 74.4 | 88.5 ± 93.1 | 85.4 ± 99.2 |

| | | | | | | |
|---|---|---|---|---|---|---|
| Mean +/- SD Not Significant between Groups (Mann-Whitney) ^{a}p=0.0637 and ^{b}p=0.0507 vs 0 week (Wilcoxon) Not Significant between Groups (Kruskal-Wallis & Dunn) (No significant difference between groups at week 0) | | | | | | |

**[Table 12-2-2]**

| Urine PCS (µg·mL⁻¹ / 770 mOsm·P, % Relative value vs week 0 ) | | | | | |
|---|---|---|---|---|---|
| Group | N | 6W | 12W | 24W^{b} | 6-24W^{a}(44-48) |
| Control | 14-15 | 159 ± 162 | 121 ± 186 | 85 ± 103^{c,e} | 121 ± 153^{a,c} |
| Citrate | 16 | 206 ± 173 | 375 ± 917 | 203 ± 200 | 262 ± 545 |
| Bicarbonate | 16 | 86 ± 35^{d} | 102 ± 100 | 294 ± 590^{e} | 161 ± 352^{b,d} |

| | | | | | |
|---|---|---|---|---|---|
| Mean +/- SD ^{a}p=0.0054, ^{b}p=0.0127, ^{c}p=0.0085, ^{d}p=0.0259 vs Citrate and ^{e}p=0.0704 vs Control (Mann-Whitney) ^{a}p=0.0085,^{b}p=0.0266 (Kruskal-Wallis) and^{c}p=0.0120,^{d}p=0.0524,^{e}p=0.0241 vs Citrate (Dunn) | | | | | |

**[Table 12-2-3]**

| Urine PCS (µg·mL⁻¹ / 770 mOsm·P, 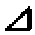 Actual measurement value vs week 0 ) | | | | | |
|---|---|---|---|---|---|
| Group | N | 6W | 12W | 24W^{b} | 6-24W^{a}(45-48) |
| Control | 15 | - 3.5 ± 49.0 | - 16.1 ± 63.1 | . 33.5 ± 56.5^{c,e} | - 18.0 ± 56.7^{a,c} |
| Citrate | 16 | 12.9 ± 43.9 | 8.2 ± 62.3 | 27.8 ± 62.0 | 16.3 ± 56.2 |
| Bicarbonate | 16 | - 8.1 ± 32.0 | - 41.9 ± 67.7^{d} | . 20.4 ± 95.1 | . 29.5 ± 69.8^{b,d} |

| | | | | | |
|---|---|---|---|---|---|
| Mean +/- SD ^{a}p=0.0117, ^{b}p=0.0047, ^{c}p=0.0093, ^{d}p=0.0465 vs Citrate (Mann-Whitney) ^{a}p=0.0080,^{b}p=0.0436 (Kruskal-Wallis) and ^{c}p=0.0407,^{d}p=0.0130,^{e}p=0.0370 vs Citrate (Dunn) | | | | | |

**[Table 12-3-1]**

| Urine PAG (µg-mL⁻¹/ 770 mOsm·P) | | | | | | |
|---|---|---|---|---|---|---|
| Group | N | 0W | 6W | 12W | 24W | 6-24W(45-48) |
| Control | 15 | 146.2 ± 87.4 | 139.0 ±109.5 | 139.9 ±100.3 | 103.3 ± 76.5^{a} | 127.4 ± 95.8 |
| Citrate | 16 | 125.0 ±107.6 | 147.3 ±157.9 | 129.5 ±115.2 | 145.9 ±158.5 | 140.9 ±142.4 |
| Bicarbonate | 16 | 197.7 ±170.9 | 209.0 ±279.2 | 135.4 ±145.4^{b} | 153.8 ±128.0 | 166.1 ±194.6 |

| | | | | | | |
|---|---|---|---|---|---|---|
| Mean +/- SD Not Significant between Groups (Mann-Whitney) ^{a}p=0.0256 and ^{b}p=0.0092 vs 0 week (Wilcoxon) Not Significant between Groups (Kruskal-Wallis & Dunn) (No significant difference between groups at week 0) | | | | | | |

**[Table 12-3-2]**

| Urine PAG (µg·mL⁻¹ / 770 mOsm·P, % Relative value vs week 0 ) | | | | | |
|---|---|---|---|---|---|
| Group | N | 6W | 12W | 24W | 6-24W^{a}(45-48) |
| Control | 15 | 105 ± 68 | 114 ± 72^{b} | 82 ± 46 | 100 ± 63 |
| Citrate | 16 | 137 ± 107 | 119 ± 99 | 123 ± 107 | 127 ± 103^{a} |
| Bicarbonate | 16 | 99 ± 68 | 75 ± 55 | 97 ± 58 | 90 ± 60 |

| | | | | | |
|---|---|---|---|---|---|
| Mean +/- SD ^{a}p=0.0764 and ^{b}p=0.0912 vs Bicarbonate (Mann-Whitney) Not Significant between Groups (Kruskal Wallis & Dunn) | | | | | |

**[Table 12-3-3]**

| Urine PAG (µg-mL⁻¹ / 770 mOsm·P, 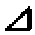 Actual measurement value vs week 0 ) | | | | | |
|---|---|---|---|---|---|
| Group | N | 6W | 12W^{b} | 24W | 6-24W^{a}(45-48) |
| Control | 15 | - 7.2 ± 84.9 | - 6.3 ± 82.9 | - 42.3± 70.5^{c} | - 18.6 ± 79.7^{a} |
| Citrate | 16 | 22.3 ± 87.1 | 4.6 ± 65.8 | 20.9 ± 85.4 | 15.9 ± 78.7 |
| Bicarbonate | 16 | 11.3 ±167.3 | - 62.4 ± 83.7^{d,e} | - 44.0 ± 132.2 | - 31.7±134.5^{b} |

| | | | | | |
|---|---|---|---|---|---|
| Mean +/- SD ^{a}p=0.0912, ^{b}p=0.0386, ^{c}p=0.0712, ^{d}p=0.0510 vs Bicarbonate and ^{e}p=0.0491 vs Control (Mann-Whitney) ^{a}p=0.0794,^{b}p=0.0727 (Kruskal-Wallis) and ^{c}p=0.0903 vs Bicarbonate (Dunn) | | | | | |

**[Table 12-4-1]**

| | Urine HA (µg·mL⁻¹ / 770 mOsm·P) | | | | | |
|---|---|---|---|---|---|---|
| Group | N | 0W | 6W | 12W | 24W | 6-24W(45-48) |
| Control | 15 | 225.6 ± 180.2 | 182.5 ± 123.7 | 166.0 ± 68.6 | 157.1 ± 90.4 | 168.5 ± 95.3 |
| Citrate | 16 | 198.4 ± 109.5 | 210.0 ± 156.9 | 181.2 ± 103.3 | 171.7 ± 131.4 | 187.6 ± 130.5 |
| Bicarbonate | 16 | 188.9 ± 161.7 | 162.2 ± 114.0 | 166.3 ± 89.9 | 182.0 ± 114.5 | 170.1 ± 104.8 |

| | | | | | | |
|---|---|---|---|---|---|---|
| Mean +/- SD Not Significant between Groups (Mann-Whitney) Not Significant vs 0 week (Wilcoxon) Not Significant between Groups (Kruskal-Wallis & Dunn) (No significant difference between groups at week 0) | | | | | | |

**[Table 12-4-2]**

| Urine HA (µg·mL⁻¹ / 770 mOsm·P, % Relative value vs week 0 ) | | | | | |
|---|---|---|---|---|---|
| Group | N | 6W | 12W | 24W | 6-24W (45-48) |
| Control | 15 | 97 ± 56 | 112 ± 97 | 116 ± 109 | 108 ± 89 |
| Citrate | 16 | 150 ± 161 | 124 ± 109 | 129 ± 128 | 134 ± 132 |
| Bicarbonate | 16 | 106 ± 91 | 130 ± 122 | 127 ± 106 | 121 ± 105 |

| | | | | | |
|---|---|---|---|---|---|
| Mean +/- SD Not Significant between Groups (Mann-Whitney) Not Significant between Groups (Kruskal-Wallis & Dunn) | | | | | |

**[Table 12-4-3]**

| Urine HA (µg·mL⁻¹ / 770 mOsm·P, 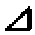 Actual measurement value vs week 0) | | | | | |
|---|---|---|---|---|---|
| Group | N | 6W | 12W | 24W | 6-24W (45-48) |
| Control | 15 | - 43.1 ± 131.0 | - 59.6 ± 146.6 | - 67.4 ± 137.5 | - 56.7 ± 135.7 |
| Citrate | 16 | 11.6 ± 144.1 | - 17.1 ± 96.5 | - 26.7 ± 168.7 | - 10.8 ± 137.7 |
| Bicarbonate | 16 | - 26.8 ± 151.8 | - 22.7 ± 135.9 | - 7.0 ± 136.0 | - 18.8 ± 138.7 |

| | | | | | |
|---|---|---|---|---|---|
| Mean +/- SD Not Significant between Groups (Mann-Whitney) Not Significant between Groups (Kruskal-Wallis & Dunn) | | | | | |

**[Table 12-5-1]**

| | Urine ASA (µg·mL⁻¹/770 mOsm·P) | | | | | |
|---|---|---|---|---|---|---|
| Group | N | 0W | 6W | 12W | 24W | 6-24W(45-48) |
| Control | 15 | 4.62 ± 2.07 | 4.27 ± 1.81 | 4.27 ± 2.28 | 3.48 ± 1.13^{a} | 4.01 ± 1.80 |
| Citrate | 16 | 4.72 ± 1.96 | 4.49 ± 2.06 | 4.47 ± 1.96 | 4.70 ± 2.36 | 4.55 ± 2.09 |
| Bicarbonate | 16 | 5.48 ± 4.33 | 5.50 ± 4.08 | 4.63 ± 3.04 | 5.14 ± 3.95 | 5.09 ± 3.66 |

| | | | | | | |
|---|---|---|---|---|---|---|
| Mean +/- SD Not Significant between Groups (Mann-Whitney) ^{a}p=0.0302 vs 0 week (Wilcoxon) Not Significant between Groups (Kruskal-Wallis & Dunn) (No significant difference between groups at week 0) | | | | | | |

**[Table 12-5-2]**

| Urine ASA (µg·mL⁻¹ / 770 mOsm·P, % Relative value vs week 0 ) | | | | | |
|---|---|---|---|---|---|
| Group | N | 6W | 12W | 24W | 6-24W (45-48) |
| Control | 15 | 99 ± 40 | 98 ± 45 | 86 ± 44 | 94 ± 42 |
| Citrate | 16 | 124 ± 61 | 109 ± 70 | 111 ± 57 | 115 ± 62^{a} |
| Bicarbonate | 16 | 104 ± 33 | 93 ± 30 | 107 ± 42 | 101 ± 35 |

| | | | | | |
|---|---|---|---|---|---|
| Mean +/- SD ^{a}p=0.0716 vs Control (Mann-Whitney) Not Significant between Groups (Kruskal-Wallis & Dunn) | | | | | |

**[Table 12-5-3]**

| Urine ASA (µg·mL⁻¹ / 770 mOsm·P, 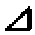 Actual measurement value vs week 0 ) | | | | | |
|---|---|---|---|---|---|
| Group | N | 6W | 12W | 24W^{a} | 6-24W (45-48) |
| Control | 15 | - 0.36 ± 1.78 | - 0.35 ± 1.71 | - 1.35 ± 1.84 | - 0.69 ± 1.80 |
| Citrate | 16 | - 0.24 ± 2.02 | - 0.25 ± 2.31 | - 0.03 ± 2.53^{a,b} | - 0.17 ± 2.25 |
| Bicarbonate | 16 | 0.02 ± 1.67 | - 0.85 ± 2.02 | - 0.34 ± 2.72 | - 0.39 ± 2.17 |

| | | | | | |
|---|---|---|---|---|---|
| **Mean** +/- SD ^{a}p=0.0364 vs Control (Mann-Whitney) ^{a}p=0.0935 (Kruskal-Wallis) and ^{b}p=0.0962 vs Control (Dunn) | | | | | |

As a result, in the cases of p-cresyl sulfate (PCS) and phenylacetyl-L-glutamine (PAG), the concentration of uremic substance in urine (excretion into urine) significantly increased in the group to which the combination preparation of hydrates of potassium citrate and sodium citrate (Citrate) had been administered than that group to which the sodium bicarbonate preparation (Bicarbonate) had been administered and the control group. Also regarding the cases of indoxyl sulfate (IS), hippuric acid (HA), and argininosuccinic acid (ASA), the concentration of uremic substance in urine (excretion into urine) increased in the group to which the combination preparation of hydrates of potassium citrate and sodium citrate (Citrate) had been administered than that group to which the sodium bicarbonate preparation (Bicarbonate) had been administered and the control group. In addition, administration of the combination preparation of hydrates of potassium citrate and sodium citrate (Citrate) increased concentrations of indoxyl sulfate (IS), p-cresyl sulfate (PCS), and phenylacetyl-L-glutamine (PAG) in the urine (excretion into urine) compared to before the start of the test (0 W).

### INDUSTRIAL APPLICABILITY

By the pharmaceutical composition provided by the present invention, uremic substance are excreted outside the body in mammals. By the method described herein, it is possible to preliminarily determine as to whether or not uremic substances are excreted outside the body and/or whether or not progression of chronic kidney disease can be suppressed.

## Claims

1. A pharmaceutical composition comprising an alkalinizing agent for use in treating or preventing kidney tubular damage,
wherein the alkalinizing agent is a mixture of sodium citrate or a hydrate thereof, and potassium citrate or a hydrate thereof,
0.1 to 5 g/day of each of sodium citrate or the hydrate thereof, and potassium citrate or the hydrate thereof are orally administered at a total of 0.2 to 10 g/day, 1 to 5 times a day, and
the alkalinizing agent is administered to a patient with stage G2 to stage G3b chronic kidney disease.

2. A pharmaceutical composition comprising an alkalinizing agent for use in suppressing progression of chronic kidney disease,
wherein the alkalinizing agent is a mixture of sodium citrate or a hydrate thereof, and potassium citrate or a hydrate thereof,
0.1 to 5 g/day of each of sodium citrate or the hydrate thereof, and potassium citrate or the hydrate thereof are orally administered at a total of 0.2 to 10 g/day, 1 to 5 times a day, and
the alkalinizing agent is administered to a patient with stage G2 to stage G3b chronic kidney disease.

3. The pharmaceutical composition for use according to any one of claims 1 or 2, wherein 0.5 to 1.5 g/day of each of sodium citrate dihydrate (C₆H₅Na₃O₇· 2H₂O), and potassium citrate hydrate (C₆H₅K₃O₇·H₂O) are orally administered at a total of 1 to 3 g/day, 1 to 5 times a day.

4. The pharmaceutical composition for use according to any one of claims 1 to 3, wherein a decrease in concentration of the uremic substance in blood compared to before the start of administration of the alkalinizing agent is detected 12 weeks after the administration of the alkalinizing agent.

5. The pharmaceutical composition for use according to any one of claims 1 to 4, wherein an increase in concentration of the uremic substance in urine compared to before the start of administration of the alkalinizing agent is detected 12 weeks after the administration of the alkalinizing agent.

6. The pharmaceutical composition for use according to claim 4 or 5, wherein the uremic substance is at least one selected from the group consisting of indoxyl sulfate, p-cresyl sulfate, phenylacetyl-L-glutamine, hippuric acid and argininosuccinic acid.

7. The pharmaceutical composition for use according to claim 4 or 5, wherein the uremic substance is indoxyl sulfate.

8. The pharmaceutical composition for use according to any one of claims 1 to 7, wherein the pharmaceutical composition is a tablet.

## Patentansprüche

1. Pharmazeutische Zusammensetzung, die ein Alkalisierungsmittel enthält, zur Verwendung bei der Behandlung oder Vorbeugung von Nierentubulusschäden,
wobei das Alkalisierungsmittel eine Mischung aus Natriumcitrat oder einem Hydrat davon und Kaliumcitrat oder einem Hydrat davon ist,
0,1 bis 5 g/Tag Natriumcitrat oder dessen Hydrat und Kaliumcitrat oder dessen Hydrat in einer Gesamtmenge von 0,2 bis 10 g/Tag 1 - bis 5-mal täglich oral verabreicht werden und
das Alkalisierungsmittel einem Patienten mit einer chronischen Nierenerkrankung im Stadium G2 bis G3b verabreicht wird.

2. Pharmazeutische Zusammensetzung, die ein Alkalisierungsmittel enthält, zur Verwendung bei der Unterdrückung des Fortschreitens einer chronischen Nierenerkrankung,
wobei das Alkalisierungsmittel eine Mischung aus Natriumcitrat oder einem Hydrat davon und Kaliumcitrat oder einem Hydrat davon ist,
0,1 bis 5 g/Tag Natriumcitrat oder dessen Hydrat und Kaliumcitrat oder dessen Hydrat in einer Gesamtmenge von 0,2 bis 10 g/Tag 1 - bis 5-mal täglich oral verabreicht werden und
das Alkalisierungsmittel einem Patienten mit einer chronischen Nierenerkrankung im Stadium G2 bis G3b verabreicht wird.

3. Pharmazeutische Zusammensetzung zur Verwendung nach einem der Ansprüche 1 oder 2, wobei 0,5 bis 1,5 g/Tag von jedem vom Natriumcitrat-Dihydrat (C₆H₅Na₃O₇·2H₂O) und Kaliumcitrat-Hydrat (C₆H₅K₃O₇·H₂O) in einer Gesamtmenge von 1 bis 3 g/Tag, 1 - bis 5-mal täglich, oral verabreicht werden.

4. Pharmazeutische Zusammensetzung zur Verwendung nach einem der Ansprüche 1 bis 3, wobei 12 Wochen nach der Verabreichung des Alkalisierungsmittels eine Abnahme der Konzentration der urämischen Substanz im Blut im Vergleich zu der Zeit vor Beginn der Verabreichung des Alkalisierungsmittels festgestellt wird.

5. Pharmazeutische Zusammensetzung zur Verwendung nach einem der Ansprüche 1 bis 4, wobei 12 Wochen nach der Verabreichung des Alkalisierungsmittels ein Anstieg der Konzentration der urämischen Substanz im Urin im Vergleich zu der Zeit vor Beginn der Verabreichung des alkalisierenden Mittels festgestellt wird.

6. Pharmazeutische Zusammensetzung zur Verwendung gemäß Anspruch 4 oder 5, wobei die urämische Substanz mindestens eine ist, die aus der Gruppe ausgewählt ist, die aus Indoxylsulfat, p-Kresylsulfat, Phenylacetyl-L-Glutamin, Hippursäure und Argininobernsteinsäure besteht.

7. Pharmazeutische Zusammensetzung zur Verwendung nach Anspruch 4 oder 5, wobei die urämische Substanz Indoxylsulfat ist.

8. Pharmazeutische Zusammensetzung zur Verwendung nach einem der Ansprüche 1 bis 7, wobei die Pharmazeutische Zusammensetzung eine Tablette ist.

## Revendications

1. Composition pharmaceutique comprenant un agent alcalinisant pour l'utilisation dans le traitement ou la prévention des lésions des tubules rénaux,
où l'agent alcalinisant est un mélange de citrate de sodium ou d'un de ses hydrates, et de citrate de potassium ou d'un de ses hydrates,
0,1 à 5 g/jour de citrate de sodium ou de son hydrate, et de citrate de potassium ou de son hydrate sont administrés par voie orale à raison d'un total de 0,2 à 10 g/jour, 1 à 5 fois par jour, et
l'agent alcalinisant est administré à un patient atteint d'une maladie rénale chronique de stade G2 à G3b.

2. Composition pharmaceutique comprenant un agent alcalinisant pour l'utilisation dans la suppression de la progression de la maladie rénale chronique,
où l'agent alcalinisant est un mélange de citrate de sodium ou d'un de ses hydrates, et de citrate de potassium ou d'un de ses hydrates,
0,1 à 5 g/jour de citrate de sodium ou de son hydrate, et de citrate de potassium ou de son hydrate sont administrés par voie orale à raison d'un total de 0,2 à 10 g/jour, 1 à 5 fois par jour, et
l'agent alcalinisant est administré à un patient atteint d'une maladie rénale chronique de stade G2 à G3b.

3. Composition pharmaceutique à utiliser selon l'une des revendications 1 ou 2, dans laquelle 0,5 à 1,5 g/jour de citrate de sodium dihydraté (C₆H₅Na₃O₇·2H₂O) et de citrate de potassium hydraté (C₆H₅K₃O₇·H₂O) sont administrés par voie orale à raison d'un total de 1 à 3 g/jour, 1 à 5 fois par jour.

4. Composition pharmaceutique à utiliser selon l'une des revendications 1 à 3, dans laquelle une diminution de la concentration de la substance urémique dans le sang par rapport au début de l'administration de l'agent alcalinisant est détectée 12 semaines après l'administration de l'agent alcalinisant.

5. Composition pharmaceutique à utiliser selon l'une des revendications 1 à 4, dans laquelle une augmentation de la concentration de la substance urémique dans l'urine par rapport au début de l'administration de l'agent alcalinisant est détectée 12 semaines après l'administration de l'agent alcalinisant.

6. Composition pharmaceutique à utiliser selon la revendication 4 ou 5, dans laquelle la substance urémique est au moins une substance choisie dans le groupe constitué par le sulfate d'indoxyle, le sulfate de p-crésyle, la phénylacétyl-L-glutamine, l'acide hippurique et l'acide argininosuccinique.

7. Composition pharmaceutique à utiliser selon la revendication 4 ou 5, dans laquelle la substance urémique est le sulfate d'indoxyle.

8. Composition pharmaceutique à utiliser selon l'une des revendications 1 à 7, dans laquelle la composition pharmaceutique est un comprimé.
